# EUROPEAN PATENT APPLICATION

(11) **EP 2 017 284 A1**
(43) Date of publication of application: **21.01.2009**
(21) Application number: 07301239.5
(22) Date of filing: 16.07.2007
(51) Int. Cl.: C07K 14/025, C12N 15/37, A61K 38/16

(54) **New polypeptides inducing apoptosis and uses thereof**

(71) Applicant: INSTITUT PASTEUR, 75015 Paris (FR); Anaconda Pharma, 75015 Paris (FR)
(72) Inventor: Demeret, Caroline, 91410 Dourdan (FR); Thierry, Françoise, 138680 Singapore (SG); Blumenfeld, Marta, 75013 Paris (FR); Gauthier, Jean-Michel, 78700 Conflans-Sainte-Honorine (FR)
(74) Representative: Warcoin, Jacques

(57) **Abstract**

The present invention concerns a polypeptide comprising the sequence X₁WX₂X₃X₄RX₅X₆X₇ X₈ X₉, and derivatives thereof, wherein X₁ is an amino acid selected in the group consisting in histidinc (H), tyrosinc (Y), aspartic acid (D), glutamine (Q), and leucine (L), preferably histidine (H) or tyrosine (Y); X₂ is a polar amino acid, charged or not, and selected in the group consisting in serine (S) threonine (T), lysine (K), arginine (R), asparagine (N), aspartic acid (D), cysteine (C), glycine (G), glutamine (Q), histidine (H), and glutamic acid (E); X₃ is a polar amino acid, charged or not, and selected in the group consisting in serine (S), threonine (T), asparagine (N), cysteine (C), histidine (H), leucine (L), tyrosine (Y), alanine (A), and isoleucine (1); X₄ is an amino acid selected in the group consisting in threonine (T), arginine (R), asparagine (N), alanine (A), isoleucine (I), leucine (L), methionine (M), phenylalanine (F), tryptophane (W), valine (V) and tyrosine; X₅ is an amino acid selected in the group consisting in leucine (L), methionine (M), isoleucine (I), tryptophane (W), tyrosine (Y), histidine (H), glutamine (Q), lysine (K), and arginine (R); X₆ is an amino acid selected in the group consisting in leucine (L), and glutamic acid (E); X₇ is an amino acid selected in the group consisting in cysteine (C), asparagine (N), alanine (A), tyrosine (Y), serine (S), glutamine (Q), and proline (P); X₈ is a hydrophobic amino acid selected in the group consisting in alanine (A), isoleucine (I), leucine (L), methionine (M), phenylalanine (F), tryptophane (W), valine (V) and tyrosine; and X₉ is a threonine or an hydrophobic amino acid selected in the group consisting in alanine (A), isoleucine (I), leucine (L), methionine (M), phenylalanine (F), tryptophane (W), valine (V) and tyrosine.

SEQUENCE LISTING

<110>  ANACONDA
       INSTITUT PATSEUR
       DEMERET, caroline
       THIERRY, francoise
       BLUMENFELD, marta
       GAUTHIER, jean-michel

<120>  NEW POLYPEPTIDES INDUCING APOPTOSIS AND USES THEREOF

<130>  66962
<160>  70

<170>  PatentIn version 3.3

<210>  1
<211>  11
<212>  PRT
<213>  artificial

<220>
<223>  consensus sequence


<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H, or E

<220>
<221>  MISC_FEATURE
<222>  (4)..(4)
<223>  X= S, T, N, C, H, L, Y, A, or I

<220>
<221>  MISC_FEATURE
<222>  (5)..(5)
<223>  X=T, R, N, A, I, L, M, F, W, V or Y

<220>
<221>  MISC_FEATURE
<222>  (7)..(7)
<223>  X=L, M, I, W, Y, H, Q, K, or R

<220>
<221>  MISC_FEATURE
<222>  (8)..(8)
<223>  X=L or E

<220>
<221>  MISC_FEATURE
<222>  (9)..(9)
<223>  X= NC, N, A, Y, S, Q or P

<220>
<221>  MISC_FEATURE
<222>  (10)..(10)
<223>  X=A, I, L, M, F, W, V or Y

<220>
<221>  MISC_FEATURE
<222>  (11)..(11)
<223>  X= A, I, L, M, F, W, V or Y

<400>  1

Xaa Trp Xaa Xaa Xaa Arg Xaa Xaa Xaa Xaa Xaa
1               5                   10


<210>  2
<211>  201
<212>  PRT
<213>  Human papillomavirus

<400>  2

Met Glu Thr Leu Cys Gln Arg Leu Asn Val Cys Gln Asp Lys Ile Leu
1               5                   10                  15

Thr His Tyr Glu Asn Asp Ser Thr Asp Leu Arg Asp His Ile Asp Tyr
            20                  25                  30

Trp Lys His Met Arg Leu Glu Cys Ala Ile Tyr Tyr Lys Ala Arg Glu
        35                  40                  45

Met Gly Phe Lys His Ile Asn His Gln Val Val Pro Thr Leu Ala Val
    50                  55                  60

Ser Lys Asn Lys Ala Leu Gln Ala Ile Glu Leu Gln Leu Thr Leu Glu
65                  70                  75                  80

Thr Ile Tyr Asn Ser Gln Tyr Ser Asn Glu Lys Trp Thr Leu Gln Asp
                85                  90                  95

Val Ser Leu Glu Val Tyr Leu Thr Ala Pro Thr Gly Cys Ile Lys Lys
            100                 105                 110

His Gly Tyr Thr Val Glu Val Gln Phe Asp Gly Asp Ile Cys Asn Thr
        115                 120                 125

Met His Tyr Thr Asn Trp Thr His Ile Tyr Ile Cys Glu Glu Ala Ser
    130                 135                 140

Val Thr Val Val Glu Gly Gln Val Asp Tyr Tyr Gly Leu Tyr Tyr Val
145                 150                 155                 160

His Glu Gly Ile Arg Thr Tyr Phe Val Gln Phe Lys Asp Asp Ala Glu
                165                 170                 175

Lys Tyr Ser Lys Asn Lys Val Trp Glu Val His Ala Gly Gly Gln Val
            180                 185                 190

Ile Leu Cys Pro Thr Ser Val Phe Ser
        195                 200


<210>  3
<211>  201
<212>  PRT

<213>  Human papillomavirus

<400>  3

Met Glu Thr Leu Ser Gln Arg Leu Asn Val Cys Gln Asp Lys Ile Leu
1               5                   10                  15

Glu His Tyr Glu Asn Asp Ser Lys Arg Leu Cys Asp His Ile Asp Tyr
            20                  25                  30

Trp Lys His Ile Arg Leu Glu Cys Val Leu Met Tyr Lys Ala Arg Glu
        35                  40                  45

Met Gly Ile His Ser Ile Asn His Gln Val Val Pro Ala Leu Ser Val
    50                  55                  60

Ser Lys Ala Lys Ala Leu Gln Ala Ile Glu Leu Gln Met Met Leu Glu
65                  70                  75                  80

Thr Leu Asn Asn Thr Glu Tyr Lys Asn Glu Asp Trp Thr Met Gln Gln
                85                  90                  95

Thr Ser Leu Glu Leu Tyr Leu Thr Ala Pro Thr Gly Cys Leu Lys Lys
            100                 105                 110

His Gly Tyr Thr Val Glu Val Gln Phe Asp Gly Asp Val His Asn Thr
        115                 120                 125

Met His Tyr Thr Asn Trp Lys Phe Ile Tyr Leu Cys Ile Asp Gly Gln
    130                 135                 140

Cys Thr Val Val Glu Gly Gln Val Asn Cys Lys Gly Ile Tyr Tyr Val
145                 150                 155                 160

His Glu Gly His Ile Thr Tyr Phe Val Asn Phe Thr Glu Glu Ala Lys
                165                 170                 175

Lys Tyr Gly Thr Gly Lys Lys Trp Glu Val His Ala Gly Gly Gln Val
            180                 185                 190

Ile Val Phe Pro Glu Ser Val Phe Ser
        195                 200

<210>  4
<211>  201
<212>  PRT
<213>  Human papillomavirus

<400>  4

Met Glu Thr Leu Ser Gln Arg Leu Ser Val Cys Gln Asp Lys Ile Leu
1               5                   10                  15

Glu His Tyr Glu Thr Asp Ser Thr Cys Leu Ser Asp His Ile Gln Tyr
            20                  25                  30

Trp Lys Leu Ile Arg Leu Glu Cys Ala Val Phe Tyr Lys Ala Arg Glu
        35                  40                  45

Met Gly Ile Lys Thr Leu Asn His Gln Val Val Pro Thr Gln Ala Ile
    50                  55                  60

Ser Lys Ala Lys Ala Met Gln Ala Ile Glu Leu Gln Leu Met Leu Glu
65                  70                  75                  80

Thr Leu Asn Thr Thr Glu Tyr Ser Thr Glu Thr Trp Thr Leu Gln Glu
                85                  90                  95

Thr Ser Ile Glu Leu Tyr Thr Thr Val Pro Gln Gly Cys Phe Lys Lys
            100                 105                 110

His Gly Val Thr Val Glu Val Gln Phe Asp Gly Asp Lys Gln Asn Thr
        115                 120                 125

Met His Tyr Thr Asn Trp Thr His Ile Tyr Ile Leu Glu Asp Ser Ile
    130                 135                 140

Cys Thr Val Val Lys Gly Leu Val Asn Tyr Lys Gly Ile Tyr Tyr Val
145                 150                 155                 160

His Gln Gly Val Glu Thr Tyr Tyr Val Thr Phe Arg Glu Glu Ala Lys
                165                 170                 175

Lys Tyr Gly Lys Lys Asn Ile Trp Glu Val His Val Gly Gly Gln Val
            180                 185                 190

Ile Val Cys Pro Glu Ser Val Phe Ser
        195                 200

<210>  5
<211>  201
<212>  PRT
<213>  Human papillomavirus

<400>  5

Met Glu Ser Ile Pro Ala Arg Leu Asn Ala Val Gln Glu Lys Ile Leu
1               5                   10                  15

Asp Leu Tyr Glu Ala Asp Ser Asn Asp Leu Asn Ala Gln Ile Glu His
            20                  25                  30

Trp Lys Leu Thr Arg Met Glu Cys Val Leu Phe Tyr Lys Ala Lys Glu
        35                  40                  45

Leu Gly Ile Thr His Ile Gly His Gln Val Val Pro Pro Met Ala Val
    50                  55                  60

Ser Lys Ala Lys Ala Cys Gln Ala Ile Glu Leu Gln Leu Ala Leu Glu
65                  70                  75                  80

Ala Leu Asn Lys Thr Gln Tyr Ser Thr Asp Gly Trp Thr Leu Gln Gln
                85                  90                  95

Thr Ser Leu Glu Met Trp Arg Ala Glu Pro Gln Lys Tyr Phe Lys Lys
            100                 105                 110

His Gly Tyr Thr Ile Thr Val Gln Tyr Asp Asn Asp Lys Asn Asn Thr
        115                 120                 125

Met Asp Tyr Thr Asn Trp Lys Glu Ile Tyr Leu Leu Gly Glu Cys Glu
    130                 135                 140

Cys Thr Ile Val Glu Gly Gln Val Asp Tyr Tyr Gly Leu Tyr Tyr Trp
145                 150                 155                 160

Cys Asp Gly Glu Lys Ile Tyr Phe Val Lys Phe Ser Asn Asp Ala Lys
                165                 170                 175

Gln Tyr Cys Val Thr Gly Val Trp Glu Val His Val Gly Gly Gln Val
            180                 185                 190

Ile Val Cys Pro Ala Ser Val Ser Ser
        195                 200

<210>  6
<211>  201
<212>  PRT
<213>  Human papillomavirus

<400>  6

Met Glu Glu Ile Ser Ala Arg Leu Ser Ala Val Gln Asp Lys Ile Leu
1               5                   10                  15

Asp Ile Tyr Glu Ala Asp Lys Asn Asp Leu Thr Ser Gln Ile Glu His
            20                  25                  30

Trp Lys Leu Ile Arg Met Glu Cys Ala Ile Met Tyr Thr Ala Arg Gln
        35                  40                  45

Met Gly Ile Ser His Leu Cys His Gln Val Val Pro Ser Leu Val Ala
    50                  55                  60

Ser Lys Thr Lys Ala Phe Gln Val Ile Glu Leu Gln Met Ala Leu Glu
65                  70                  75                  80

Thr Leu Asn Ala Ser Pro Tyr Lys Thr Asp Glu Trp Thr Leu Gln Gln
                85                  90                  95

Thr Ser Leu Glu Val Trp Leu Ser Glu Pro Gln Lys Cys Phe Lys Lys
            100                 105                 110

Lys Gly Ile Thr Val Thr Val Gln Tyr Asp Asn Asp Lys Ala Asn Thr
        115                 120                 125

Met Asp Tyr Thr Asn Trp Ser Glu Ile Tyr Ile Ile Glu Glu Thr Thr
    130                 135                 140

Cys Thr Leu Val Ala Gly Glu Val Asp Tyr Val Gly Leu Tyr Tyr Ile
145                 150                 155                 160

His Gly Asn Glu Lys Thr Tyr Phe Lys Tyr Phe Lys Glu Asp Ala Lys
                165                 170                 175

Lys Tyr Ser Lys Thr Gln Leu Trp Glu Val His Val Gly Ser Arg Val
            180                 185                 190

Ile Val Cys Pro Thr Ser Ile Pro Ser
        195                 200


<210>  7
<211>  201
<212>  PRT
<213>  Human papillomavirus

<400>  7

Met Glu Glu Ile Ser Ala Arg Leu Asn Ala Val Gln Glu Lys Ile Leu
1               5                   10                  15

Asp Leu Tyr Glu Ala Asp Lys Thr Asp Leu Pro Ser Gln Ile Glu His
            20                  25                  30

Trp Lys Leu Ile Arg Met Glu Cys Ala Leu Leu Tyr Thr Ala Lys Gln
        35                  40                  45

Met Gly Phe Ser His Leu Cys His Gln Val Val Pro Ser Leu Leu Ala
    50                  55                  60

Ser Lys Thr Lys Ala Phe Gln Val Ile Glu Leu Gln Met Ala Leu Glu
65                  70                  75                  80

Thr Leu Ser Lys Ser Gln Tyr Ser Thr Ser Gln Trp Thr Leu Gln Gln
                85                  90                  95

Thr Ser Leu Glu Val Trp Leu Cys Glu Pro Pro Lys Cys Phe Lys Lys
            100                 105                 110

Gln Gly Glu Thr Val Thr Val Gln Tyr Asp Asn Asp Lys Lys Asn Thr
        115                 120                 125

Met Asp Tyr Thr Asn Trp Gly Glu Ile Tyr Ile Ile Glu Glu Asp Thr
    130                 135                 140

Cys Thr Met Val Thr Gly Lys Val Asp Tyr Ile Gly Met Tyr Tyr Ile
145                 150                 155                 160

His Asn Cys Glu Lys Val Tyr Phe Lys Tyr Phe Lys Glu Asp Ala Ala
                165                 170                 175

Lys Tyr Ser Lys Thr Gln Met Trp Glu Val His Val Gly Gly Gln Val
            180                 185                 190

Ile Val Cys Pro Thr Ser Ile Ser Ser
        195                 200

<210>  8
<211>  201
<212>  PRT
<213>  Human papillomavirus

<400>  8

Met Glu Glu Ile Ala Thr Arg Leu Asn Ala Val Gln Glu Lys Ile Leu
1               5                   10                  15

Asp Val Tyr Glu Ala Asn Lys Lys Asp Leu Cys Thr Gln Ile Glu His
            20                  25                  30

Trp Arg Leu Arg Arg Ile Glu Cys Ala Leu Tyr Tyr Lys Ala Lys Glu
        35                  40                  45

Leu Gly Leu Ala Arg Ile Gly His Gln Val Val Pro Pro Met Ser Val
    50                  55                  60

Thr Lys Ala Lys Ala Cys Gln Ile Ile Glu Leu Gln Met Ala Leu Gln
65                  70                  75                  80

Thr Leu Asn Glu Thr Gln Tyr Ser Asn Asp Glu Trp Thr Leu Gln Ser
                85                  90                  95

Thr Ser Leu Glu Leu Trp Leu Cys Glu Pro Lys Lys Cys Leu Lys Lys
            100                 105                 110

Lys Gly Glu Thr Val Thr Val Gln Tyr Asp Asn Asp Lys Glu Asn Thr
        115                 120                 125

Met Glu Tyr Thr Ser Trp Asn Glu Ile Tyr Ile Val Lys Asp Ser Val
    130                 135                 140

Trp Thr Val Val Val Gly Lys Val Asp Tyr Met Gly Leu Tyr Tyr Ile
145                 150                 155                 160

Tyr Glu Thr Glu Lys Thr Tyr Tyr Val Lys Phe Glu Thr Asp Ala Lys
                165                 170                 175

Lys Tyr Ser Lys Gln Asn Ile Trp Glu Val His Val Gly Ala Gln Val
            180                 185                 190

Ile Val Cys Pro Thr Ser Met Phe Ser
        195                 200

<210>  9
<211>  202
<212>  PRT
<213>  Human papillomavirus

<400>  9

Met Glu Thr Leu Ser Gln Arg Leu Asn Val Leu Gln Glu Lys Ile Leu
1               5                   10                  15

Glu His Tyr Glu Gln Asp Ser Lys Cys Ile Gln Asp His Ile Asn Tyr
            20                  25                  30

Trp Asn Cys Val Arg Leu Glu Asn Ala Ile Tyr Tyr Ala Ala Arg Glu
        35                  40                  45

Arg Gly Met His Asn Ile Asp His Gln Val Val Pro Pro Val Asn Ile
    50                  55                  60

Ser Lys Thr Lys Ala Tyr Gln Ala Ile Glu Leu Gln Met Ala Leu Glu
65                  70                  75                  80

Ser Leu Ala Lys Thr Ala Tyr Ser Ala Glu Glu Trp Thr Leu Arg Asp
                85                  90                  95

Thr Ser Asn Glu Leu Trp His Thr Lys Pro Lys Gln Cys Phe Lys Lys
            100                 105                 110

His Gly Val Thr Val Glu Val Trp Tyr Asp Gly Asp Lys Ser Asn Ser
        115                 120                 125

Met His Tyr Val Val Trp Gly Thr Ile Tyr Phe Lys Asn Ser Thr Asp
    130                 135                 140

Thr Trp Cys Lys Thr Gln Gly Arg Val Asp Tyr Trp Gly Val Tyr Tyr
145                 150                 155                 160

Met Tyr Glu Gln Gln Lys Thr Tyr Tyr Glu Arg Phe Met Gln Asp Ala
                165                 170                 175

Gln Leu Tyr Gly Thr Ser Gly Lys Trp Asp Val His Tyr Asn Gly Asn
            180                 185                 190

Ile Ile His Cys Pro Asp Ser Met Cys Ser
        195                 200

<210>  10
<211>  202
<212>  PRT
<213>  Human papillomavirus

<400>  10

Met Glu Thr Leu Ser Gln Arg Leu Asn Ala Leu Gln Glu Lys Ile Leu
1               5                   10                  15

Glu His Tyr Glu Gln Asp Ser Lys Leu Ile Tyr Asp Gln Ile Asn Tyr
            20                  25                  30

Trp Lys Tyr Val Arg Leu Glu Asn Ala Ile Phe Tyr Ala Ala Arg Glu
        35                  40                  45

Arg Gly Met His Thr Ile Asp His Gln Val Val Pro Pro Gly Thr Thr
    50                  55                  60

Ser Lys Ala Lys Ala Tyr Gln Ala Ile Glu Leu Gln Met Ala Leu Glu
65                  70                  75                  80

Ser Leu Ala Gln Thr Asp Phe Asn Lys Glu Glu Trp Thr Leu Lys Asp
                85                  90                  95

Thr Ser Asn Glu Met Trp Gln Thr Lys Pro Lys Gln Cys Phe Lys Lys
            100                 105                 110

Lys Gly Val Thr Val Glu Val Trp Tyr Asp Gly Asn Lys Asp Asn Ser
        115                 120                 125

Met His Tyr Val Val Trp Gly Ala Ile Tyr Tyr Lys Thr His Thr Asp
    130                 135                 140

Thr Trp Cys Lys Thr Glu Gly Tyr Val Asp Tyr Trp Gly Ile Tyr Tyr
145                 150                 155                 160

Val His Glu Gln His Lys Thr Tyr Tyr Glu Val Phe Lys Gln Asp Ala
                165                 170                 175

Gln Met Tyr Gly Thr Ser Gly Lys Trp Glu Val His Cys Asn Gly Asn
            180                 185                 190

Ile Ile His Cys Pro Asp Ser Met Tyr Ser
        195                 200

<210>  11
<211>  202
<212>  PRT
<213>  Human papillomavirus

<400>  11

Met Lys Thr Leu Ser Gln Arg Leu Asn Val Leu Gln Asp Lys Ile Leu
1               5                   10                  15

Glu Tyr Tyr Glu Gln Asp Ser Lys Ser Ile Tyr Asp Gln Ile Asn Tyr
            20                  25                  30

Trp Lys Cys Val Arg Met Glu Asn Ala Ile Phe Tyr Ala Ala Arg Glu
            35                  40                  45

Arg Gly Met His Thr Ile Asp His Gln Val Val Pro Thr Ile Asn Ile
    50                  55                  60

Ser Lys Cys Lys Ala Tyr Gln Ala Ile Glu Leu Gln Met Ala Leu Glu
65                  70                  75                  80

Ser Val Ala Gln Thr Glu Tyr Asn Thr Glu Glu Trp Thr Leu Lys Asp
                85                  90                  95

Thr Ser Asn Glu Leu Trp His Thr Gln Pro Lys Gln Cys Phe Lys Lys
            100                 105                 110

Gln Gly Thr Thr Val Glu Val Trp Tyr Asp Gly Asp Lys Cys Asn Ala
        115                 120                 125

Met Asn Tyr Val Leu Trp Gly Ala Ile Tyr Tyr Lys Asn Asn Ile Asp
    130                 135                 140

Ile Trp Cys Lys Thr Glu Gly Cys Val Asp Tyr Trp Gly Ile Tyr Tyr
145                 150                 155                 160

Met Asn Glu His Leu Lys Val Tyr Tyr Glu Val Phe Ile Gln Asp Ala
                165                 170                 175

Glu Arg Tyr Gly Thr Ser Gly Lys Trp Glu Val His Tyr Asn Gly Asn
            180                 185                 190

Ile Ile His Cys Pro Asp Ser Met Cys Ser
        195                 200

<210>  12
<211>  202
<212>  PRT
<213>  Human papillomavirus

<400>  12

Met Asp Thr Leu Ser Gln Arg Leu Ser Val Leu Gln Asp Gln Ile Leu
1               5                   10                  15

Glu His Tyr Glu Asn Asp Ser Lys Asp Ile Asn Glu His Ile Asn Tyr
            20                  25                  30

Trp Lys Leu Val Arg Met Glu Asn Val Ile Leu Phe Ala Ala Arg Glu
        35                  40                  45

Asn Asn Ile His Thr Leu Asn His Gln Val Val Pro Thr Phe Leu Val
    50                  55                  60

Ser Lys Asn Lys Ala Cys Glu Ala Ile Glu Leu Gln Ser Asn Arg Thr

65                  70                  75                  80

Ser Thr Val Met Pro Cys Phe Leu Lys His Phe Leu Gly Ala Val Cys
                85                  90                  95

His Ser Ser Trp His Val Ser Cys Ile Val His Cys Ser Phe Leu Asn
            100                 105                 110

Ser Val Cys Ala Lys Leu Ser Asn Ala Ile Cys Ser Lys Glu Asn Thr
        115                 120                 125

Met His Tyr Thr Ser Trp Thr Phe Ile Tyr Tyr Val Asn Asp Val Gly
    130                 135                 140

Gln Trp Cys Lys Thr Thr Gly Asn Val Asp Phe Trp Gly Leu Tyr Tyr
145                 150                 155                 160

Lys Val Glu Glu Glu Gln Val Tyr Tyr Val Lys Phe Ile His Asp Ala
                165                 170                 175

Lys Lys Tyr Gly Thr Thr Asp Lys Trp Glu Val His Tyr Asn Gly Lys
            180                 185                 190

Val Ile Asp Cys Tyr Asp Ser Met Cys Ser
        195                 200

<210>  13
<211>  201
<212>  PRT
<213>  Human papillomavirus

<400>  13

Glu Thr Leu Ser Glu Arg Leu Ser Ala Leu Gln Asp Lys Ile Ile Asp
1               5                   10                  15

His Tyr Glu Asn Asp Ser Lys Asp Ile Asp Ser Gln Ile Gln Tyr Trp
            20                  25                  30

Gln Leu Ile Arg Trp Glu Asn Ala Ile Phe Phe Ala Ala Arg Glu His
        35                  40                  45

Gly Ile Gln Thr Leu Asn His Gln Val Val Pro Ala Tyr Asn Ile Ser
    50                  55                  60

Lys Ser Lys Ala His Lys Ala Ile Glu Leu Gln Met Ala Leu Gln Gly
65                  70                  75                  80

Leu Ala Gln Ser Ala Tyr Lys Thr Glu Asp Trp Thr Leu Gln Asp Thr
                85                  90                  95

Cys Glu Glu Leu Trp Asn Thr Glu Pro Thr His Cys Phe Lys Lys Gly
            100                 105                 110

Gly Gln Thr Val Gln Val Tyr Phe Asp Gly Asn Lys Asp Asn Cys Met
        115                 120                 125

Thr Tyr Val Ala Trp Asp Ser Val Tyr Tyr Met Thr Asp Ala Gly Thr
    130                 135                 140

Trp Asp Lys Thr Ala Thr Cys Val Ser His Arg Gly Leu Tyr Tyr Val
145                 150                 155                 160

Lys Glu Gly Tyr Asn Thr Phe Tyr Ile Glu Phe Lys Ser Glu Cys Glu
                165                 170                 175

Lys Tyr Gly Asn Thr Gly Thr Trp Glu Val His Phe Gly Asn Asn Val
            180                 185                 190

Ile Asp Cys Asn Asp Ser Met Cys Ser
        195                 200

<210>  14
<211>  201
<212>  PRT
<213>  Human papillomavirus

<400>  14

Glu Ser Leu Ser Glu Arg Leu Ser Ala Leu Gln Asp Lys Ile Leu Asp
1               5                   10                  15

His Tyr Glu Asn Asp Ser Lys Asp Ile Asn Ser Gln Ile Ser Tyr Trp
            20                  25                  30

Gln Leu Ile Arg Leu Glu Asn Ala Ile Leu Phe Thr Ala Arg Glu His
        35                  40                  45

Gly Ile Thr Lys Leu Asn His Gln Val Val Pro Pro Ile Asn Ile Ser
    50                  55                  60

Lys Ser Lys Ala His Lys Ala Ile Glu Leu Gln Met Ala Leu Lys Gly
65                  70                  75                  80

Leu Ala Gln Ser Lys Tyr Asn Asn Glu Glu Trp Thr Leu Gln Asp Thr
                85                  90                  95

Cys Glu Glu Leu Trp Asn Thr Glu Pro Ser Gln Cys Phe Lys Lys Gly
            100                 105                 110

Gly Lys Thr Val His Val Tyr Phe Asp Gly Asn Lys Asp Asn Cys Met
        115                 120                 125

Asn Tyr Val Val Trp Asp Ser Ile Tyr Tyr Ile Thr Glu Thr Gly Ile
    130                 135                 140

Trp Asp Lys Thr Ala Ala Cys Val Ser Tyr Trp Gly Val Tyr Tyr Ile

145                 150                 155                 160

Lys Asp Gly Asp Thr Thr Tyr Tyr Val Gln Phe Lys Ser Glu Cys Glu
                165                 170                 175

Lys Tyr Gly Asn Ser Asn Thr Trp Glu Val Gln Tyr Gly Gly Asn Val
            180                 185                 190

Ile Asp Cys Asn Asp Ser Met Cys Ser
        195                 200

<210>  15
<211>  201
<212>  PRT
<213>  Human papillomavirus

<400>  15

Met Glu Asn Leu Cys Gln Arg Leu Asn Ala Cys Gln Glu Lys Ile Leu
1               5                   10                  15

Asp Tyr Tyr Glu Leu Asp Ser Asp Lys Leu Ser Asp Gln Ile Asp Tyr
            20                  25                  30

Trp Lys Leu Val Arg Tyr Glu Cys Ala Ile Phe Tyr Lys Ala Arg Glu
        35                  40                  45

Gly Asn Met Gln Cys Ile Asn His Gln Val Val Pro Ser Thr Thr Val
    50                  55                  60

Cys Lys Glu Lys Ala Trp Gln Ala Ile Glu Leu His Ile Ala Leu Gln
65                  70                  75                  80

Ser Leu Met Asn Ser Glu Tyr Gly Lys Glu Lys Trp Thr Met Arg Asp
                85                  90                  95

Thr Cys Tyr Glu Leu Tyr Val Thr Glu Pro Lys His Cys Phe Lys Lys
            100                 105                 110

Glu Gly Thr Thr Val Thr Val Val Phe Asp Cys Asn Lys Glu Asn Thr
        115                 120                 125

Met Asp Tyr Ile Arg Trp Thr Ser Val Tyr Tyr Lys Thr Asp Met Gly
    130                 135                 140

Trp Cys Lys Thr Asn Gly Glu Val Asp Tyr Lys Gly Ile Tyr Tyr Met
145                 150                 155                 160

His Gly Cys Cys Lys Gln Tyr Tyr Val Asp Phe Lys Gln Glu Ala Leu
                165                 170                 175

Lys Tyr Gly Thr Gly Leu Gln Trp Asp Val His Val Cys Gly Gln Val
            180                 185                 190

Ile Cys Cys Pro Glu Phe Val Ser Ser
        195                 200

<210>  16
<211>  201
<212>  PRT
<213>  Human papillomavirus

<400>  16

Met Glu Asn Leu Cys Gln Arg Leu Asn Ala Cys Gln Glu Lys Ile Leu
1               5                   10                  15

Asp Tyr Tyr Glu Leu Asp Ser Asn Lys Leu Thr Asp Gln Ile Asp Tyr
            20                  25                  30

Trp Lys Leu Val Arg Tyr Glu Cys Ala Ile Phe Tyr Lys Ala Arg Glu
        35                  40                  45

Gly Asn Met Gln Cys Ile Asn His Gln Val Val Pro Ser Thr Val Val
    50                  55                  60

Cys Lys Gln Lys Ala Trp Gln Ala Ile Glu Ile His Ile Ala Leu Gln
65                  70                  75                  80

Ser Leu Ile Asn Thr Asp Tyr Asn Thr Glu Ala Trp Thr Met Arg Asp
                85                  90                  95

Thr Ser Tyr Glu Met Tyr Met Thr Glu Pro Lys His Cys Phe Lys Lys
            100                 105                 110

Glu Gly Thr Thr Val Thr Val Val Phe Asp Cys Asn Lys Glu Asn Thr
        115                 120                 125

Met Asp Tyr Ile Arg Trp Lys Tyr Val Tyr Tyr Lys Thr Asp Ile Gly
    130                 135                 140

Trp Cys Lys Gly Thr Gly Asp Val Asp Ala Lys Gly Ile Tyr Tyr Thr
145                 150                 155                 160

Gln Gly Ala Tyr Lys Gln Tyr Tyr Val Asp Phe Lys Gln Glu Ala Glu
                165                 170                 175

Lys Tyr Gly Thr Gly Val Gln Trp Ala Val His Val Cys Gly Gln Val
            180                 185                 190

Ile Cys Cys Pro Glu Phe Val Ser Ser
        195                 200

<210>  17
<211>  200
<212>  PRT
<213>  Human papillomavirus

<400>  17

Met Glu Thr Leu Cys His Arg Leu Asn Val Cys Gln Glu Lys Ile Leu
1               5                   10                  15

Asp Cys Tyr Glu Leu Asp Ser Asp Lys Leu Val Asp Gln Ile Asn Tyr
            20                  25                  30

Trp Thr Leu Val Arg Tyr Glu Cys Ala Met Phe Tyr Thr Ala Arg Glu
        35                  40                  45

Arg Asn Met Gln Thr Leu Asn His Gln Val Val Pro Ala Ser Ala Val
    50                  55                  60

Ser Lys Gln Lys Ala Cys Gln Ala Ile Glu Met His Met Ala Leu Glu
65                  70                  75                  80

Ser Leu Asn Lys Ser Glu Tyr Asn Met Glu Pro Trp Thr Met Arg Asp
                85                  90                  95

Thr Cys Tyr Glu Leu Trp Gly Glu Ala Pro Lys His Cys Phe Lys Lys
            100                 105                 110

Gly Gly Lys Thr Val Thr Val Met Phe Asp Gly Asn Lys Asp Asn Thr
        115                 120                 125

Met Asp Tyr Thr Cys Trp Thr Tyr Val Tyr Ile Tyr Lys Glu Asp Arg
    130                 135                 140

Trp Val Lys Thr Gln Gly Asn Val Asp Tyr Thr Gly Ile Tyr Tyr Lys
145                 150                 155                 160

Pro Asp Val Asn Lys Glu Tyr Tyr Val Thr Phe Lys Asp Glu Ala Lys
                165                 170                 175

Lys Tyr Gly Ala Gln Gln Trp Glu Val Tyr Met Cys Gly Asn Val Ile
            180                 185                 190

Thr Cys Pro Glu Tyr Val Ser Ser
        195                 200

<210>  18
<211>  200
<212>  PRT
<213>  Human papillomavirus

<400>  18

Met Glu Thr Leu Cys His Arg Leu Asn Val Cys Gln Glu Lys Ile Leu
1               5                   10                  15

Asp Cys Tyr Glu Leu Asp Ser Asp Lys Leu Val Asp Gln Ile Asn Tyr
            20                  25                  30

Trp Thr Leu Leu Arg Tyr Glu Ala Ala Met Phe Tyr Ala Ala Arg Glu
        35                  40                  45

Arg Asn Leu Arg Thr Ile Asn His Gln Val Val Pro Ala Thr Thr Val
    50                  55                  60

Ser Lys Gln Lys Ala Cys Gln Ala Ile Glu Met His Met Ala Leu Gln
65                  70                  75                  80

Ser Leu Asn Lys Ser Asp Tyr Asn Met Glu Pro Trp Thr Met Arg Glu
                85                  90                  95

Thr Cys Tyr Glu Leu Trp Cys Val Ala Pro Lys Gln Cys Phe Lys Lys
            100                 105                 110

Gly Gly Ile Thr Val Thr Val Ile Phe Asp Gly Asn Lys Asp Asn Ala
        115                 120                 125

Met Asp Tyr Thr Ser Trp Lys Phe Ile Tyr Ile Tyr Asp Asn Asp Lys
    130                 135                 140

Trp Val Lys Thr Asn Gly Asn Val Asp Tyr Thr Gly Ile Tyr Tyr Thr
145                 150                 155                 160

Val Asn Ser Lys Lys Glu Tyr Tyr Val Gln Phe Lys Asp Glu Ala Lys
                165                 170                 175

Ile Tyr Gly Ala Gln Gln Trp Glu Val Tyr Met Tyr Gly Thr Val Ile
            180                 185                 190

Thr Cys Pro Glu Tyr Val Ser Ser
        195                 200

<210> 19
<211> 201
<212> PRT
<213> Human papillomavirus

<400> 19

Met Glu Thr Leu Ser Gln Arg Leu Asp Ala Cys Gln Asn Lys Ile Leu
1               5                   10                  15

Asp Cys Tyr Glu Lys Asp Ser Lys Cys Ile Ile Asp His Ile Asp Tyr
            20                  25                  30

Trp Lys Ala Val Arg His Glu Tyr Val Leu Tyr Tyr Lys Ala Arg Glu
        35                  40                  45

Asn Asp Ile Asn Val Leu Asn His Gln Met Val Pro Ser Leu Gln Val
    50                  55                  60

Cys Lys Ala Lys Ala Cys Ser Ala Ile Glu Leu Gln Ile Ala Leu Glu
65                  70                  75                  80

Ala Ile Ser Asn Thr Ile Tyr Lys Asn Glu Glu Trp Thr Leu Arg Asp
                85                  90                  95

Thr Cys Asp Glu Leu Trp Arg Thr Glu Pro Lys Asn Cys Phe Lys Lys
            100                 105                 110

Glu Gly Gln His Ile Glu Val Trp Phe Asp Gly Asn Lys Asn Asn Cys
        115                 120                 125

Met Glu Tyr Val Val Trp Lys Phe Ile Tyr Tyr Asn Gly Glu Cys Gly
    130                 135                 140

Trp Cys Lys Val Ser Ser Gly Val Asp Tyr Arg Gly Ile Tyr Tyr Met
145                 150                 155                 160

His Asp Gly His Lys Thr Tyr Tyr Thr Asp Phe Glu Gln Glu Ala Lys
                165                 170                 175

Lys Tyr Gly Cys Thr Asn Ile Trp Glu Val His Met Glu Thr Glu Ser
            180                 185                 190

Ile Tyr Cys Pro Asp Ser Val Ser Ser
        195                 200

<210>  20
<211>  201
<212>  PRT
<213>  Human papillomavirus

<400>  20

Met Glu Thr Leu Cys Gln Arg Leu Asp Ala Cys Gln Glu Lys Ile Leu
1               5                   10                  15

Asp Cys Phe Glu Arg Asp Ser Lys Asn Ile Thr Asp His Ile Asp Tyr
            20                  25                  30

Trp Lys Ala Val Arg Gln Glu Asn Val Ile Tyr Tyr Lys Ala Arg Glu
        35                  40                  45

Asn Asn Met Thr Lys Leu Gly His Gln Val Val Pro Cys Leu Gln Val
    50                  55                  60

Cys Lys Ala Lys Ala Cys Val Ala Ile Glu Leu Gln Ile Ala Leu Glu
65                  70                  75                  80

Ser Leu Cys Lys Thr Glu Tyr Asn Met Glu Glu Trp Thr Leu Arg Asp
                85                  90                  95

Val Cys Glu Ser Met Trp Tyr Thr Glu Pro Lys Gln Cys Phe Lys Lys
            100                 105                 110

Gln Gly Gln His Ile Glu Val Trp Phe Asp Gly Ser Lys Asp Asn Arg
        115                 120                 125

Ala Glu Tyr Val Val Trp Lys Trp Val Tyr Tyr Cys Gly Glu Asp Gly
    130                 135                 140

Trp Cys Lys Val Ser Ser Ala Val Ser Tyr Glu Gly Ile Tyr Tyr Ile
145                 150                 155                 160

His Asp Gly His Lys Thr Tyr Tyr Thr Asn Phe Lys Asp Glu Ala Thr
                165                 170                 175

Lys Tyr Gly Cys Lys Gly Thr Trp Glu Val His Met Gly Lys Gln Ser
            180                 185                 190

Ile Tyr Cys Pro Asp Ser Val Ser Ser
        195                 200


<210>  21
<211>  201
<212>  PRT
<213>  Human papillomavirus

<400>  21

Met Glu Thr Leu Cys Gln Arg Leu Asp Ala Cys Gln Glu Lys Ile Leu
1               5                   10                  15

Asp Cys Phe Glu Asn Asp Ser Lys Lys Ile Glu Asp His Ile Val Tyr
            20                  25                  30

Trp Lys Ala Val Arg His Glu Asn Val Val Leu Tyr Lys Ala Arg Gln
        35                  40                  45

Asn Asn Ile Thr Lys Leu Arg His Gln Val Val Pro Cys Leu Gln Val
    50                  55                  60

Cys Lys Ala Lys Ala Cys Val Ala Ile Glu Ile Gln Met Ala Leu Glu
65                  70                  75                  80

Ser Leu Tyr Lys Thr Glu Tyr Lys Val Glu Glu Trp Thr Leu Lys Asp
                85                  90                  95

Val Cys Glu Asn Met Trp His Thr Ala Pro Lys Gln Cys Phe Lys Lys
            100                 105                 110

Ser Gly Lys Arg Ile Glu Val Trp Phe Asp Gly Lys Lys Asp Asn Arg
        115                 120                 125

Thr Glu Tyr Val Val Trp Gln Trp Val Tyr Tyr Cys Gly Asp Asn Gly
    130                 135                 140

Trp Thr Lys Val Pro Ser Val Val Asp Tyr Lys Gly Ile Tyr Tyr Val
145                 150                 155                 160

His Asp Gly Asn Lys Val Tyr Tyr Thr Asp Phe Asn Asp Glu Ala Val
                165                 170                 175

Lys Tyr Gly Tyr Lys Gly Thr Trp Glu Val His Met Gly Asn Glu Ser
            180                 185                 190

Ile Tyr Cys Pro Asp Ser Val Ser Ser
        195                 200

<210>  22
<211>  203
<212>  PRT
<213>  Human papillomavirus

<400>  22

Met Glu Ser Leu Ala Asp Arg Leu Asp Ala Cys Gln Glu Lys Leu Leu
1               5                   10                  15

Asp Leu Tyr Glu Lys Asp Ser Asn Lys Leu Glu Asp Gln Ile Leu His
            20                  25                  30

Trp His Tyr Val Arg Leu Glu Asn Ala Met Leu Phe Lys Ala Arg Gln
        35                  40                  45

Ala Gly Leu Thr Arg Val Gly His Gln Met Val Pro Thr Leu Ser Val
    50                  55                  60

Thr Lys Gly Lys Ala His Lys Ala Ile Glu Val His Leu Ser Leu Gln
65                  70                  75                  80

Gly Leu Gln Thr Ser Ala Tyr Ala His Glu Pro Trp Thr Leu Gln Thr
                85                  90                  95

Thr Ser Leu Glu Met Trp Asn Thr Gln Pro Gln Arg Cys Trp Lys Lys
            100                 105                 110

Lys Gly Arg Arg Leu Thr Val Lys Phe Asp Gly Glu Asp His Lys Ala
        115                 120                 125

Val Glu Tyr Val Ser Trp Gly Tyr Ile Tyr Val Gln Ser Thr Glu Thr
    130                 135                 140

Asp Leu Trp Tyr Lys Val Pro Gly Lys Val Ser Tyr Lys Gly Leu Tyr
145                 150                 155                 160

Tyr Glu Met Glu Gly Gln Glu His Tyr Tyr Val Thr Phe Ala Gln Glu
                165                 170                 175

Ala Gln Lys Tyr Gly Glu Thr Gly Lys Trp Glu Val His Met Gly Asn
            180                 185                 190

Thr Val Ile Tyr Glu Pro Cys Ala Ser Val Ser
        195                 200

<210>  23
<211>  201
<212>  PRT
<213>  Human papillomavirus

<400>  23

Met Glu Thr Ile Ala Lys His Leu Asp Val Cys Gln Glu Gln Leu Leu
1               5                   10                  15

Glu Leu Tyr Glu Glu Asn Ser Asn Asn Leu Thr Lys His Ile Gln His
            20                  25                  30

Trp Lys Cys Ile Arg Tyr Glu Cys Val Leu Leu His Lys Ala Lys Gln
        35                  40                  45

Met Gly Leu Asn His Ile Gly Met Gln Val Val Pro Ala Leu Thr Val
    50                  55                  60

Ser Gln Thr Lys Gly His Gln Ala Ile Glu Met Gln Met Thr Leu Glu
65                  70                  75                  80

Thr Leu Leu Asn Ser Asp Tyr Gly Met Glu Pro Trp Thr Leu Gln Asp
                85                  90                  95

Thr Ser Arg Glu Met Trp Leu Thr Ala Pro Lys Tyr Cys Phe Lys Lys
            100                 105                 110

Gln Gly Gln Thr Val Glu Val Lys Tyr Asp Cys Asn Ala Asp Asn Ile
        115                 120                 125

Met Glu Tyr Val Ser Trp Lys Tyr Ile Tyr Val His Asp Thr Asp Lys
    130                 135                 140

Trp Val Lys Val Thr Gly His Ile Asp Tyr Lys Gly Leu Tyr Tyr Val
145                 150                 155                 160

His Gly Gly His Lys Thr Tyr Tyr Thr Asn Phe Glu Lys Glu Ala Lys
                165                 170                 175

Lys Tyr Gly Asn Ser Leu Gln Trp Glu Val Cys Ile Gly Ser Ser Val
            180                 185                 190

Ile Cys Ser Pro Ala Ser Ile Ser Ser
        195                 200

<210>  24
<211>  201
<212>  PRT
<213>  Human papillomavirus

<400>  24

Met Glu Thr Ile Ala Lys His Leu Asp Val Cys Gln Glu Gln Leu Leu
1               5                   10                  15

Glu Leu Tyr Glu Glu Asn Ser Asn Lys Leu Thr Lys His Ile Gln His
            20                  25                  30

Trp Lys Cys Ile Arg Tyr Glu Cys Val Leu Leu His Lys Ala Lys Gln
        35                  40                  45

Met Gly Leu Asn His Ile Gly Met Gln Val Val Pro Ala Leu Ala Val
    50                  55                  60

Ser Gln Thr Lys Gly His Gln Ala Ile Glu Met Gln Met Thr Leu Glu
65                  70                  75                  80

Thr Leu Leu Asn Ser Asp Tyr Gly Thr Glu Pro Trp Thr Leu Gln Glu
                85                  90                  95

Thr Ser Arg Glu Met Trp Leu Thr Pro Pro Lys Tyr Cys Phe Lys Lys
            100                 105                 110

Gln Gly Gln Thr Val Glu Val Lys Phe Asp Cys Asn Ala Asp Asn Ala
        115                 120                 125

Met Glu Tyr Val Trp Trp Lys Val Ile Tyr Val Phe Asp Thr Asp Lys
    130                 135                 140

Trp Val Lys Val Thr Gly His Ile Asp Tyr Lys Gly Leu Tyr Tyr Val
145                 150                 155                 160

His Gly Gly His Lys Thr Tyr Tyr Thr Asn Phe Glu Lys Glu Ala Glu
                165                 170                 175

Lys Tyr Gly Asn Ser Leu Gln Trp Glu Val Cys Ile Gly Ser Ser Ile
            180                 185                 190

Ile Cys Ser Pro Ala Ser Ile Ser Ser
        195                 200

<210>  25
<211>  204
<212>  PRT
<213>  Human papillomavirus

<400>  25

Met Glu Thr Ile Ala Lys His Leu Asp Ala Cys Gln Glu Gln Gly Arg
1               5                   10                  15

Ile Arg Leu Asn Met Tyr Glu Glu Asn Ser Asn Glu Leu Lys Lys His
            20                  25                  30

Ile Gln His Trp Lys Cys Leu Arg Tyr Glu Ser Val Leu Leu His Lys

35                  40                  45

Ala Arg Gln Met Gly Leu Ser His Ile Gly Leu Gln Val Val Pro Pro
    50                  55                  60

Leu Thr Val Ser Gln Ala Lys Gly His Glu Ala Ile Glu Met Gln Met
65                  70                  75                  80

Thr Leu Glu Thr Leu Leu Glu Ser Glu Phe Gly Met Glu Pro Trp Thr
                85                  90                  95

Leu Gln Asp Thr Ser Arg Glu Met Trp Leu Thr Pro Pro Lys Arg Cys
            100                 105                 110

Phe Lys Lys Gln Gly Gln Thr Val Glu Val Lys Tyr Asp Cys Asn Thr
        115                 120                 125

Asp Asn Arg Met Asp Tyr Val Ser Trp Thr Tyr Ile Tyr Val Phe Asp
    130                 135                 140

Thr Asp Lys Trp Thr Lys Val Lys Gly Met Val Asp Tyr Lys Gly Leu
145                 150                 155                 160

Tyr Tyr Ile His Gly Asn Leu Lys Thr Tyr Tyr Ile Glu Phe Glu Lys
                165                 170                 175

Glu Ala Lys Lys Tyr Gly Glu Thr Leu Gln Trp Glu Val Cys Ile Gly
            180                 185                 190

Ser Thr Val Ile Cys Ser Pro Ala Ser Val Ser Ser
        195                 200

<210>  26
<211>  201
<212>  PRT
<213>  Human papillomavirus

<400>  26

Met Glu Ala Ile Ala Lys Arg Leu Asp Ala Cys Gln Glu Gln Leu Leu
1               5                   10                  15

Glu Leu Tyr Glu Glu Asn Ser Thr Asp Leu Asn Lys His Val Leu His
            20                  25                  30

Trp Lys Cys Met Arg His Glu Ser Val Leu Leu Tyr Lys Ala Lys Gln
        35                  40                  45

Met Gly Leu Ser His Ile Gly Met Gln Val Val Pro Pro Leu Lys Val
    50                  55                  60

Ser Glu Ala Lys Gly His Asn Ala Ile Glu Met Gln Met His Leu Glu
65                  70                  75                  80

Ser Leu Leu Lys Thr Glu Tyr Ser Met Glu Pro Trp Thr Leu Gln Glu
                85                  90                  95

Thr Ser Tyr Glu Met Trp Gln Thr Pro Pro Lys Arg Cys Phe Lys Lys
            100                 105                 110

Arg Gly Lys Thr Val Glu Val Lys Phe Asp Gly Cys Ala Asn Asn Thr
        115                 120                 125

Met Asp Tyr Val Val Trp Thr Asp Val Tyr Val Gln Asp Thr Asp Ser
    130                 135                 140

Trp Val Lys Val His Ser Met Val Asp Ala Lys Gly Ile Tyr Tyr Thr
145                 150                 155                 160

Cys Gly Gln Phe Lys Thr Tyr Tyr Val Asn Phe Val Lys Glu Ala Glu
                165                 170                 175

Lys Tyr Gly Ser Thr Lys Gln Trp Glu Val Cys Tyr Gly Ser Thr Val
            180                 185                 190

Ile Cys Ser Pro Ala Ser Val Ser Ser
        195                 200

<210>  27
<211>  201
<212>  PRT
<213>  Human papillomavirus

<400>  27

Met Glu Ala Ile Ala Lys Arg Leu Asp Ala Cys Gln Glu Gln Leu Leu
1               5                   10                  15

Glu Leu Tyr Glu Glu Asn Ser Thr Asp Leu His Lys His Val Leu His
            20                  25                  30

Trp Lys Cys Met Arg His Glu Ser Val Leu Leu Tyr Lys Ala Lys Gln
        35                  40                  45

Met Gly Leu Ser His Ile Gly Met Gln Val Val Pro Pro Leu Lys Val
    50                  55                  60

Ser Glu Ala Lys Gly His Asn Ala Ile Glu Met Gln Met His Leu Glu
65                  70                  75                  80

Ser Leu Leu Arg Thr Glu Tyr Ser Met Glu Pro Trp Thr Leu Gln Glu
                85                  90                  95

Thr Ser Tyr Glu Met Trp Gln Thr Pro Pro Lys Arg Cys Phe Lys Lys
            100                 105                 110

Arg Gly Lys Thr Val Glu Val Lys Phe Asp Gly Cys Ala Asn Asn Thr

115                 120                 125

Met Asp Tyr Val Val Trp Thr Asp Val Tyr Val Gln Asp Asn Asp Thr
    130                 135                 140

Trp Val Lys Val His Ser Met Val Asp Ala Lys Gly Ile Tyr Tyr Thr
145                 150                 155                 160

Cys Gly Gln Phe Lys Thr Tyr Tyr Val Asn Phe Val Lys Glu Ala Glu
                165                 170                 175

Lys Tyr Gly Ser Thr Lys His Trp Glu Val Cys Tyr Gly Ser Thr Val
            180                 185                 190

Ile Cys Ser Pro Ala Ser Val Ser Ser
        195                 200

<210>  28
<211>  201
<212>  PRT
<213>  Human papillomavirus

<400>  28

Met Glu Ala Ile Ala Lys Arg Leu Asp Ala Cys Gln Asp Gln Leu Leu
1               5                   10                  15

Glu Leu Tyr Glu Glu Asn Ser Ile Asp Ile His Lys His Ile Met His
            20                  25                  30

Trp Lys Cys Ile Arg Leu Glu Ser Val Leu Leu His Lys Ala Lys Gln
        35                  40                  45

Met Gly Leu Ser His Ile Gly Leu Gln Val Val Pro Pro Leu Thr Val
    50                  55                  60

Ser Glu Thr Lys Gly His Asn Ala Ile Glu Met Gln Met His Leu Glu
65                  70                  75                  80

Ser Leu Ala Lys Thr Gln Tyr Gly Val Glu Pro Trp Thr Leu Gln Asp
                85                  90                  95

Thr Ser Tyr Glu Met Trp Leu Thr Pro Pro Lys Arg Cys Phe Lys Lys
            100                 105                 110

Gln Gly Asn Thr Val Glu Val Lys Phe Asp Gly Cys Glu Asp Asn Val
        115                 120                 125

Met Glu Tyr Val Val Trp Thr His Ile Tyr Leu Gln Asp Asn Asp Ser
    130                 135                 140

Trp Val Lys Val Thr Ser Ser Val Asp Ala Lys Gly Ile Tyr Tyr Thr
145                 150                 155                 160

Cys Gly Gln Phe Lys Thr Tyr Tyr Val Asn Phe Asn Lys Glu Ala Gln
                165                 170                 175

Lys Tyr Gly Ser Thr Asn His Trp Glu Val Cys Tyr Gly Ser Thr Val
            180                 185                 190

Ile Cys Ser Pro Ala Ser Val Ser Ser
        195                 200

<210>  29
<211>  202
<212>  PRT
<213>  Human papillomavirus

<400>  29

Met Glu Thr Leu Ala Lys Arg Leu Asp Ala Cys Gln Glu Gln Leu Leu
1               5                   10                  15

Glu Leu Tyr Glu Glu Asp Ser Lys His Leu Glu Lys His Val Gln His
            20                  25                  30

Trp Lys Cys Leu Arg Ile Glu Ala Ala Leu Leu Phe Lys Ala Arg Glu
        35                  40                  45

Met Gly Tyr Ala Gln Val Gly His Gln Ile Val Pro Ala Leu Glu Ile
    50                  55                  60

Ser Arg Ala Lys Ala His Val Ala Ile Glu Ile Gln Leu Ala Leu Glu
65                  70                  75                  80

Thr Leu Leu Gln Ser Thr Phe Gly Thr Glu Pro Trp Thr Leu Gln Glu
                85                  90                  95

Thr Ser Tyr Glu Met Trp His Ala Glu Pro Lys Lys Cys Leu Lys Lys
            100                 105                 110

Gln Gly Arg Thr Val Glu Val Val Phe Asp Gly Asn Pro Glu Asn Ala
        115                 120                 125

Met His Tyr Thr Ala Trp Thr Phe Ile Tyr Val Gln Thr Leu Asp Gly
    130                 135                 140

Thr Trp Cys Lys Val Tyr Gly His Val Cys Tyr Ala Gly Leu Tyr Tyr
145                 150                 155                 160

Ile Val Asp Asn Met Lys Gln Phe Tyr Cys Asn Phe Lys Asn Glu Ala
                165                 170                 175

Lys Lys Tyr Gly Val Thr Gly Gln Trp Glu Val His Asp Gly Thr Gln
            180                 185                 190

Val Ile Val Ser Pro Ala Ser Ile Ser Ser

195                 200


<210>  30
<211>  202
<212>  PRT
<213>  Human papillomavirus

<400>  30

Met Glu Arg Leu Ala Lys Arg Leu Asp Ala Cys Gln Glu Gln Leu Leu
1               5                   10                  15

Glu Leu Tyr Glu Glu Asn Ser Arg Asp Leu Gln Lys His Ile Glu His
            20                  25                  30

Trp Lys Cys Leu Arg Met Glu Ala Val Val Leu Tyr Lys Ala Arg Glu
        35                  40                  45

Met Gly Phe Ala Asn Ile Gly His Gln Ile Val Pro Thr Leu Glu Thr
    50                  55                  60

Cys Arg Ala Lys Ala His Met Ala Ile Glu Ile His Leu Ala Leu Glu
65                  70                  75                  80

Thr Leu Leu Gln Ser Ser Tyr Gly Lys Glu Pro Trp Thr Leu Gln Glu
                85                  90                  95

Thr Ser Asn Glu Leu Trp Leu Thr Asn Pro Lys Lys Cys Phe Lys Lys
            100                 105                 110

Gln Gly Arg Thr Val Glu Val Ile Phe Asp Gly Lys Gln Asp Asn Ala
        115                 120                 125

Met His Tyr Thr Ala Trp Thr Tyr Ile Tyr Ile Gln Thr Val Gln Gly
    130                 135                 140

Thr Trp Cys Lys Val Gln Gly His Val Cys His Ala Gly Leu Tyr Tyr
145                 150                 155                 160

Ile Val Glu Asn Met Lys Gln Phe Tyr Cys Asn Phe Lys Glu Glu Ala
                165                 170                 175

Lys Lys Tyr Gly Val Thr Asp Gln Trp Glu Val His Asp Gly Asn Gln
            180                 185                 190

Val Ile Val Ser Pro Ala Pro Ile Ser Ser
        195                 200


<210>  31
<211>  203
<212>  PRT
<213>  Human papillomavirus

<400>  31

Met Glu Thr Leu Ala Thr Arg Leu Asp Val Cys Gln Glu Arg Leu Leu
1               5                   10                  15

Asp Leu Tyr Glu Lys Asp Ser Asp Lys Leu Glu Asp Gln Ile Glu His
            20                  25                  30

Trp Lys Cys Ile Arg Leu Glu Cys Ala Leu Gln Tyr Lys Ala Arg Glu
        35                  40                  45

Leu Gly Tyr Lys Val Leu Gln His Gln Ala Leu Pro Ala Leu Ala Val
    50                  55                  60

Ser Lys Gly Lys Gly His Lys Ala Ile Glu Leu Gln Leu Ala Leu Gln
65                  70                  75                  80

Thr Leu Gln Gln Thr Val Tyr Ser Thr Glu Pro Trp Thr Leu Gln Asp
                85                  90                  95

Thr Cys Leu Glu Arg Trp Asp Ala Pro Pro Thr Gly Cys Phe Lys Lys
            100                 105                 110

Arg Gly Gln Thr Val Asp Val Ile Phe Asp Gly His Lys Glu Asn Thr
        115                 120                 125

Met Gln Tyr Val Met Trp Ala Cys Ile Tyr Phe Pro Asn Cys Asp Gly
    130                 135                 140

Glu Gly Trp Thr Lys Val Cys Ser Asn Ile Asp Ala Met Gly Ile Tyr
145                 150                 155                 160

Tyr Met Asp Ala Glu Tyr Lys Val Tyr Tyr Val Asp Phe Ala Lys Glu
                165                 170                 175

Ala Ser Lys Tyr Gly Glu His Gly Gln Trp Glu Val Arg Met Gly Ser
            180                 185                 190

Ser Ile Ile Phe Ser Pro Ala Ser Val Ser Ser
        195                 200


<210>  32
<211>  203
<212>  PRT
<213>  Human papillomavirus

<400>  32

Met Glu Thr Leu Ala Thr Arg Leu Asp Val Cys Gln Glu Arg Leu Leu
1               5                   10                  15

Asp Leu Tyr Glu Lys Asp Ser Asn Lys Leu Glu Asp Gln Ile Glu His
            20                  25                  30

Trp Lys Cys Ile Arg Leu Glu Cys Ala Leu Gln Tyr Lys Ala Arg Glu
        35                  40                  45

Met Gly Tyr Lys Val Leu Gln His Gln Ala Leu Pro Ala Leu Ala Val
    50                  55                  60

Ser Lys Gly Lys Gly His Lys Ala Ile Glu Leu Gln Leu Ala Leu Glu
65                  70                  75                  80

Thr Leu Gln Lys Thr Val Tyr Ser Thr Glu Pro Trp Thr Leu Gln Asp
                85                  90                  95

Thr Cys Leu Glu Arg Trp Asn Ala Pro Pro Thr Gly Cys Leu Lys Arg
            100                 105                 110

Arg Gly Gln Thr Val Asp Val Ile Phe Asp Gly His Gln Asp Asn Thr
        115                 120                 125

Met Gln Tyr Val Met Trp Gly Asp Ile Tyr Tyr Gln Asn Cys Asp Gly
    130                 135                 140

Glu Gly Trp Thr Lys Val Cys Ser Asn Ile Asp Ala Met Gly Ile Tyr
145                 150                 155                 160

Tyr Met Asp Ala Glu His Lys Val Tyr Tyr Val Asp Phe Lys Lys Glu
                165                 170                 175

Ala Ser Lys Tyr Gly Glu Tyr Gly Gln Trp Glu Val Arg Met Gly Ser
            180                 185                 190

Ser Ile Ile Phe Ser Pro Ala Ser Val Ser Ser
        195                 200

<210>  33
<211>  201
<212>  PRT
<213>  Human papillomavirus

<400>  33

Met Glu Thr Leu Cys Lys Arg Leu Ser Ala Cys Gln Asp Ala Ile Leu
1               5                   10                  15

Glu Leu Tyr Glu Arg Asp Ser Val His Leu Ser Asp His Ile Asp His
            20                  25                  30

Trp Lys His Val Arg His Glu Asn Val Leu Leu His Lys Ala Arg Glu
        35                  40                  45

Met Gly Leu Gln Thr Val Asn Asn Gln Ala Val Pro Ser Leu Ala Val
    50                  55                  60

Ser Arg Ser Lys Gly Tyr Asn Ala Ile Glu Met Gln Ile Ala Leu Glu
65                  70                  75                  80

Ser Leu Asn Glu Ser Leu Tyr Asn Thr Glu Glu Trp Thr Leu Gln His
                85                  90                  95

Thr Ser Trp Glu Leu Trp Val Thr Glu Pro Lys Gln Cys Phe Lys Lys
            100                 105                 110

Asp Gly Lys Thr Val Glu Val Arg Tyr Asp Cys Glu Lys Asp Asn Ser
        115                 120                 125

Met Gln Tyr Val Phe Trp Thr His Ile Tyr Cys Trp Tyr Glu Gly Gly
    130                 135                 140

Trp Ala Lys Val Gly Ser Lys Ile Asp Tyr Asn Gly Ile Tyr Tyr Glu
145                 150                 155                 160

Thr Asp Asp Glu Glu Lys Val Tyr Tyr Thr Arg Phe Asp Thr Asp Ala
                165                 170                 175

Lys Arg Tyr Gly Val Lys Gly Ile Trp Glu Val His Met Gly Gly Gln
            180                 185                 190

Val Ile Cys Cys Ala Pro Val Ser Ser
        195                 200


<210>  34
<211>  201
<212>  PRT
<213>  Human papillomavirus

<400>  34

Met Glu Thr Leu Cys Lys Arg Leu Ser Ala Cys Gln Asp Ala Ile Leu
1               5                   10                  15

Glu Leu Tyr Glu Arg Asp Ser Ile His Leu Ser Asp His Ile Asp His
            20                  25                  30

Trp Lys His Val Arg Leu Glu Asn Val Leu Leu His Lys Ala Arg Glu
        35                  40                  45

Met Gly Leu Gln Ser Val Asn Gln Gln Ala Val Pro Ser Leu Ala Val
    50                  55                  60

Ser Arg Ser Lys Gly His Asn Ala Ile Glu Leu Gln Leu Ala Leu Glu
65                  70                  75                  80

Ser Leu Asn Glu Ser Ser Tyr Asn Thr Glu Glu Trp Thr Leu Gln Gln
                85                  90                  95

Thr Ser Trp Glu Gln Trp Val Thr Asp Pro Lys Gln Cys Phe Lys Lys
            100                 105                 110

Gly Gly Lys Thr Val Glu Val Arg Tyr Asp Cys Asp Lys Asp Asn Thr
        115                 120                 125

Met Gln Tyr Val Val Trp Thr Phe Val Tyr Tyr Trp Leu Glu Gly Lys
    130                 135                 140

Trp Tyr Lys Val Ser Ser His Val Asp Tyr Asn Gly Ile Tyr Tyr Glu
145                 150                 155                 160

Thr Gln Asp Asn Glu Lys Val Tyr Tyr Thr Gln Phe Asp Arg Asp Ala
                165                 170                 175

Lys Arg Tyr Gly Val Lys Gly Ile Trp Asp Val Cys Met Gly Gly Lys
            180                 185                 190

Val Ile Cys Phe Ala Pro Val Phe Ser
        195                 200

<210>  35
<211>  202
<212>  PRT
<213>  Human papillomavirus

<400>  35

Met Glu Thr Leu Ser Ala Arg Phe Thr Val Leu Gln Glu Lys Leu Met
1               5                   10                  15

Asp Ile Tyr Glu Ser Gly Val Glu Asp Leu Asp Thr Gln Ile Gln His
            20                  25                  30

Trp Gln Leu Leu Arg Gln Glu Gln Ile Ile Tyr His Tyr Ala Arg Arg
        35                  40                  45

His Gly Val Thr Arg Leu Gly Tyr Gln Pro Val Pro Ser Leu Ala Ser
    50                  55                  60

Ser Glu Ala Lys Ala Lys Asp Ala Ile Ser Met Val Leu Leu Leu Glu
65                  70                  75                  80

Ser Leu Lys Lys Ser Lys Tyr Ala Asp Glu Gln Trp Thr Leu Ala Gln
                85                  90                  95

Thr Ser Leu Glu Ala Val Arg Ser Pro Pro Ala Asp Cys Phe Lys Lys
            100                 105                 110

Gly Pro Lys Asn Ile Glu Val Val Phe Asp Gly Asp Pro Glu Asn Leu
        115                 120                 125

Met Ser Tyr Thr Val Trp Thr Tyr Ile Tyr Tyr Leu Thr Asp Glu Asp
    130                 135                 140

Ile Trp Glu Lys Val Glu Gly His Val Asp Tyr Thr Gly Ala Tyr Tyr
145                 150                 155                 160

Tyr Glu Gly Lys Leu Lys Val Tyr Tyr Leu Lys Phe Glu Asn Asp Ala
                165                 170                 175

Lys Arg Tyr Gly Val Thr Gly Leu Trp Glu Val His Val Asn Lys Asp
            180                 185                 190

Thr Val Phe Thr Pro Val Thr Ser Ser Thr
        195                 200

<210>  36
<211>  202
<212>  PRT
<213>  Human papillomavirus

<400>  36

Met Glu Ala Leu Ser Glu Arg Phe Ser Ala Leu Gln Asp Lys Leu Met
1               5                   10                  15

Asp Leu Tyr Glu Ser Gly Leu Glu Asp Leu Glu Thr Gln Ile Gln His
            20                  25                  30

Trp Lys Leu Leu Arg Gln Glu Gln Ile Leu Leu Tyr Tyr Ala Arg Lys
        35                  40                  45

Arg Gly Ile Met Arg Leu Gly Tyr Gln Pro Val Pro Pro Leu Ala Thr
    50                  55                  60

Ser Glu Ile Lys Ala Lys Asp Ala Ile Ala Ile Gly Ile Leu Leu Glu
65                  70                  75                  80

Ser Leu Gln Lys Ser Lys Tyr Ala Asp Glu Pro Trp Thr Leu Val Glu
                85                  90                  95

Thr Ser Leu Glu Thr Ile Arg Ser Pro Pro Val Asp Cys Phe Lys Lys
            100                 105                 110

Gly Pro Lys Thr Val Glu Val Tyr Phe Asp Gly Asp Pro Glu Asn Val
        115                 120                 125

Met Pro Tyr Thr Val Trp Ser Tyr Ile Tyr Tyr Gln Thr Asp Glu Asp
    130                 135                 140

Thr Trp Glu Lys Val Glu Gly His Val Asp Tyr Thr Gly Ala Tyr Phe
145                 150                 155                 160

Tyr Glu Gly Gln Leu Lys Asn Tyr Tyr Ile Lys Phe Glu Ala Asp Ala
                165                 170                 175

Lys Arg Phe Gly Thr Thr Gly Met Trp Glu Val His Val Asn Lys Asp
            180                 185                 190

Thr Val Phe Thr Pro Val Thr Ser Ser Thr
        195                 200

<210>  37
<211>  202
<212>  PRT
<213>  Human papillomavirus

<400>  37

Met Glu Lys Leu Ser Glu Arg Phe Ser Ala Leu Gln Glu Lys Leu Met
1               5                   10                  15

Asp Leu Tyr Glu Ser Gly Val Glu Asp Leu Glu Thr Gln Ile Gln His
            20                  25                  30

Trp Lys Leu Leu Arg Gln Glu Gln Val Leu Phe Tyr Tyr Ala Arg Arg
        35                  40                  45

His Gly Ile Leu Arg Leu Gly Tyr Gln Pro Val Pro Thr Leu Ala Thr
    50                  55                  60

Ser Glu Ser Lys Ala Lys Asp Ala Ile Ala Met Gly Leu Leu Leu Glu
65                  70                  75                  80

Ser Leu Gln Lys Ser Gln Tyr Ala Glu Glu Pro Trp Thr Leu Val Glu
                85                  90                  95

Thr Ser Leu Glu Thr Val Lys Ser Pro Pro Ala Asp Cys Phe Lys Lys
            100                 105                 110

Gly Pro Lys Ser Val Glu Val Tyr Phe Asp Gly Asp Pro Glu Asn Val
        115                 120                 125

Met Ser Tyr Thr Val Trp Ser Tyr Ile Tyr Tyr Gln Thr Asp Asp Glu
    130                 135                 140

Ser Trp Glu Lys Val Glu Gly His Val Asp Tyr Thr Gly Ala Tyr Tyr
145                 150                 155                 160

Ile Glu Gly Thr Phe Lys Thr Tyr Tyr Ile Lys Phe Glu Thr Asp Ala
                165                 170                 175

Lys Arg Tyr Gly Thr Thr Gly His Trp Glu Val His Val Asn Lys Asp
            180                 185                 190

Thr Val Phe Thr Pro Val Thr Ser Ser Thr
        195                 200

<210>  38
<211>  202
<212>  PRT
<213>  Human papillomavirus

<400>  38

Met Asp Thr Leu Ser Asp Arg Phe Asn Ala Leu Gln Glu Asn Leu Met

1               5                   10                  15

Asp Ile Tyr Glu Ser Gly Arg Asp Asp Leu Glu Thr Gln Ile Met His
            20                  25                  30

Trp Gln Leu Leu Arg Gln Glu Gln Ile Leu Phe His Tyr Ala Arg Lys
        35                  40                  45

Asn Gly Val Met Arg Leu Gly Tyr Gln Pro Val Pro Pro Leu Ala Thr
    50                  55                  60

Ser Glu Ala Lys Ala Lys Asp Ala Ile Gly Met Val Ile Leu Leu Glu
65                  70                  75                  80

Ser Leu Gln Gln Ser Ala Tyr Gly Lys Glu Ser Trp Thr Leu Thr Gln
                85                  90                  95

Thr Ser Leu Glu Thr Val Arg Ser Pro Pro Ala Asn Cys Phe Lys Lys
            100                 105                 110

Gly Pro Gln Asn Ile Glu Val Met Phe Asp Asn Asp Pro Glu Asn Leu
        115                 120                 125

Met Val Tyr Thr Ala Trp Ser Phe Ile Tyr Tyr Gln Thr Val Asp Asp
    130                 135                 140

Thr Trp Asn Lys Val Glu Gly His Val Asp Tyr Tyr Gly Ala Tyr Tyr
145                 150                 155                 160

Phe Glu Gly Asp Leu Lys Val Tyr Tyr Ile Gln Phe Glu Gly Asp Ala
                165                 170                 175

Ala Arg Phe Ser Lys Thr Gly Arg Trp Glu Val His Val Asn Lys Asp
            180                 185                 190

Thr Ile Phe Ala Pro Val Thr Ser Ser Ser
        195                 200

<210>  39
<211>  202
<212>  PRT
<213>  Human papillomavirus

<400>  39

Met Asp Asn Leu Ser Glu Arg Phe Asn Val Leu Gln Glu Asn Leu Met
1               5                   10                  15

Asp Ile Tyr Glu Ser Gly Gln Glu Asp Ile Glu Thr Gln Ile Lys His
            20                  25                  30

Trp Gln Leu Leu Arg Gln Glu Gln Val Leu Phe Tyr Tyr Ala Arg Lys
        35                  40                  45

Asn Gly Val Met Arg Val Gly Tyr Gln Pro Val Pro Pro Leu Ala Thr
    50                  55                  60

Ser Glu Ala Lys Ala Lys Asp Ala Ile Gly Met Val Leu Leu Leu Gln
65                  70                  75                  80

Ser Leu Gln Lys Ser Pro Tyr Gly Lys Glu Pro Trp Thr Leu Thr Gln
                85                  90                  95

Thr Ser Leu Glu Thr Val Arg Ser Pro Pro Ala Asn Cys Phe Lys Lys
            100                 105                 110

Gly Pro Gln Asn Ile Glu Val Met Phe Asp Asn Asp Pro Glu Asn Leu
        115                 120                 125

Met Ser Tyr Thr Val Trp Ser Phe Ile Tyr Tyr Gln Asn Leu Asp Asp
    130                 135                 140

Thr Trp Asn Lys Val Glu Gly Arg Val Asp Tyr His Gly Ala Tyr Tyr
145                 150                 155                 160

Met Glu Gly Ser Leu Lys Val Tyr Tyr Ile Gln Phe Glu Val Asp Ala
                165                 170                 175

Ala Arg Phe Gly Lys Thr Gly Arg Trp Glu Val His Val Asn Glu Asp
            180                 185                 190

Thr Ile Phe Ala Pro Val Thr Ser Ser Ser
        195                 200

<210>  40
<211>  202
<212>  PRT
<213>  Human papillomavirus

<400>  40

Met Glu Thr Leu Ser Ala Arg Phe Asn Ala Leu Gln Glu Thr Leu Met
1               5                   10                  15

Asp Leu Tyr Glu Ser Gly Arg Glu Asp Leu Gln Ser Gln Ile Asp His
            20                  25                  30

Trp Gln Thr Leu Arg Gln Glu Gln Ile Leu Leu His Tyr Ala Arg Lys
        35                  40                  45

Asn Gly Val Met Arg Leu Gly Tyr Gln Pro Val Pro Pro Leu Ala Thr
    50                  55                  60

Ser Glu Gln Lys Ala Lys Asp Ala Ile Gly Met Val Leu Leu Leu Gln
65                  70                  75                  80

Ser Leu Gln Arg Ser Ala Tyr Gly Gln Glu Pro Trp Thr Leu Ala Gln

85                  90                  95

Thr Ser Leu Glu Ala Val Arg Ser Pro Pro Ala Tyr Ala Phe Lys Lys
            100                 105                 110

Gly Pro Gln Asn Ile Glu Val Val Tyr Asp Gly Asp Pro Asp Asn Val
        115                 120                 125

Met Ser Tyr Thr Ile Trp Asn Phe Ile Tyr Tyr Gln Thr Val Asn Asp
    130                 135                 140

Thr Trp Glu Lys Val Gln Gly His Val Asp Tyr Phe Gly Ala Tyr Tyr
145                 150                 155                 160

Phe Glu Gly Thr Val Lys Thr Tyr Tyr Ile Asn Phe Asp Lys Asp Ala
                165                 170                 175

Ala Arg Tyr Gly Arg Thr Gly Val Trp Glu Val His Val Asn Lys Asp
            180                 185                 190

Ile Val Phe Ala Pro Val Thr Ser Ser Ser
        195                 200

<210>  41
<211>  202
<212>  PRT
<213>  Human papillomavirus

<400>  41

Met Glu Ala Leu Asn Ala Arg Phe Asn Val Leu Gln Glu Met Leu Met
1               5                   10                  15

Asp Ile Tyr Glu Ser Gly Lys Glu Asp Leu Glu Thr Gln Ile Glu His
            20                  25                  30

Trp Lys Leu Leu Arg Gln Glu Gln Ala Leu Leu Phe Phe Ala Arg Lys
        35                  40                  45

His Ser Ile Met Arg Leu Gly Tyr Gln Pro Val Pro Pro Met Ala Val
    50                  55                  60

Ser Glu Thr Lys Ala Lys Gln Ala Ile Gly Met Met Leu Thr Leu Gln
65                  70                  75                  80

Ser Leu Gln Lys Ser Pro Phe Gly Lys Glu Lys Trp Thr Leu Val Asn
                85                  90                  95

Thr Ser Leu Glu Thr Tyr Asn Ala Pro Pro Ala Gln Cys Phe Lys Lys
            100                 105                 110

Gly Pro Tyr Asn Ile Glu Val Ile Phe Asp Gly Asp Pro Glu Asn Leu
        115                 120                 125

Met Val Tyr Thr Ala Trp Lys Glu Ile Tyr Phe Val Asp Ser Asp Asp
    130                 135                 140

Met Trp Gln Lys Val Gln Gly Glu Val Asp Tyr Ala Gly Ala Tyr Tyr
145                 150                 155                 160

Lys Asp Gly Thr Ile Lys Gln Tyr Tyr Val Thr Phe Ala Asp Asp Ala
                165                 170                 175

Val Arg Tyr Gly Thr Ser Gly Gln Tyr Glu Val Arg Ile Asn Asn Glu
            180                 185                 190

Thr Val Phe Ala Pro Val Thr Ser Ser Thr
        195                 200

<210>  42
<211>  202
<212>  PRT
<213>  Human papillomavirus

<400>  42

Met Glu Asn Leu Ser Glu Arg Phe Asn Ala Leu Gln Asp Gln Leu Met
1               5                   10                  15

Asn Ile Tyr Glu Ala Ala Glu Gln Thr Leu Gln Ala Gln Ile Lys His
            20                  25                  30

Trp Gln Thr Leu Arg Lys Glu Ala Val Leu Leu Tyr Tyr Ala Arg Glu
        35                  40                  45

Lys Gly Val Thr Arg Leu Gly Tyr Gln Pro Val Pro Val Lys Ala Val
    50                  55                  60

Ser Glu Thr Lys Ala Lys Glu Ala Ile Ala Met Val Leu Gln Leu Glu
65                  70                  75                  80

Ser Leu Gln Thr Ser Asp Phe Ala His Glu Pro Trp Thr Leu Val Asp
                85                  90                  95

Thr Ser Thr Glu Thr Phe Arg Ser Ala Pro Glu Gly His Phe Lys Lys
            100                 105                 110

Gly Pro Val Pro Val Glu Val Ile Tyr Asp Asn Asp Pro Asp Asn Ala
        115                 120                 125

Asn Leu Tyr Thr Met Trp Thr Tyr Val Tyr Tyr Met Asp Ala Asp Asp
    130                 135                 140

Lys Trp His Lys Ala Arg Ser Gly Val Asn His Ile Gly Ile Tyr Tyr
145                 150                 155                 160

Leu Gln Gly Thr Phe Lys Asn Tyr Tyr Val Leu Phe Ala Asp Asp Ala

165                 170                 175

Lys Arg Tyr Gly Thr Thr Gly Glu Trp Glu Val Lys Val Asn Lys Asp
            180                 185                 190

Thr Val Phe Ala Pro Val Thr Ser Ser Thr
        195                 200

<210>  43
<211>  202
<212>  PRT
<213>  Human papillomavirus

<400>  43

Met Glu Asn Leu Ser Glu Arg Phe Asn Ala Leu Gln Asp Gln Leu Met
1               5                   10                  15

Asn Ile Tyr Glu Ala Ala Glu Gln Thr Leu Gln Ala Gln Ile Lys His
            20                  25                  30

Trp Gln Thr Leu Arg Lys Glu Pro Val Leu Leu Tyr Tyr Ala Arg Glu
        35                  40                  45

Lys Gly Val Thr Arg Leu Gly Tyr Gln Pro Val Pro Val Lys Ala Val
    50                  55                  60

Ser Glu Thr Lys Ala Lys Glu Ala Ile Ala Met Val Leu Gln Leu Glu
65                  70                  75                  80

Ser Leu Gln Thr Ser Asp Phe Ala His Glu Pro Trp Thr Leu Val Asp
                85                  90                  95

Thr Ser Ile Glu Thr Phe Arg Ser Ala Pro Glu Gly His Phe Lys Lys
            100                 105                 110

Gly Pro Leu Pro Val Glu Val Ile Tyr Asp Asn Asp Pro Asp Asn Ala
        115                 120                 125

Asn Leu Tyr Thr Met Trp Thr Tyr Val Tyr Tyr Met Asp Ala Asp Asp
    130                 135                 140

Lys Trp His Lys Ala Arg Ser Gly Val Asn His Ile Gly Ile Tyr Tyr
145                 150                 155                 160

Leu Gln Gly Thr Phe Lys Asn Tyr Tyr Val Leu Phe Ala Asp Asp Ala
                165                 170                 175

Lys Arg Tyr Gly Thr Thr Gly Glu Trp Glu Val Lys Val Asn Lys Glu
            180                 185                 190

Thr Val Phe Ala Pro Val Thr Ser Ser Thr
        195                 200

<210>  44
<211>  201
<212>  PRT
<213>  Human papillomavirus

<400>  44

Met Glu Asn Leu Ser Glu Arg Phe Asn Ala Leu Gln Asp Leu Leu Met
1               5                   10                  15

Asn Ile Tyr Glu Ala Ala Glu Gln Thr Leu Glu Ala Gln Ile Lys His
            20                  25                  30

Trp Gln Thr Leu Arg Gln Glu Ala Val Leu Leu Tyr Phe Ala Arg Gln
        35                  40                  45

Arg Gly Val Thr Arg Leu Gly Tyr Gln Pro Val Pro Val Lys Ala Val
    50                  55                  60

Ser Glu Ala Lys Ala Lys Glu Ala Ile Ala Met Val Leu Gln Leu Gln
65                  70                  75                  80

Ser Leu Gln Thr Ser Glu Tyr Ala Ser Glu Thr Trp Thr Leu Val Asp
                85                  90                  95

Thr Ser Ile Glu Thr Phe Arg Ser Ala Pro Asp Gly His Phe Lys Lys
            100                 105                 110

Gly Pro Val Pro Val Glu Val Ile Tyr Asp Asn Asp Ala Asp Asn Ala
        115                 120                 125

Asn Leu Tyr Thr Met Trp Thr Tyr Val Tyr Tyr Met Glu Asp Asp Val
    130                 135                 140

Trp His Lys Ala Arg Ser Gly Val Asn Glu Thr Gly Ile Tyr Tyr Leu
145                 150                 155                 160

Gln Gly Thr Phe Lys Tyr Tyr Tyr Val Leu Phe Ala Asp Asp Ala Arg
                165                 170                 175

Lys Tyr Ser Gln Thr Gly Gln Trp Glu Val Lys Val Asn Lys Glu Thr
            180                 185                 190

Val Phe Ala Pro Val Thr Ser Ser Thr
        195                 200

<210>  45
<211>  202
<212>  PRT
<213>  Human papillomavirus

<400>  45

Met Glu Asn Leu Ser Glu Arg Phe Asn Ala Leu Gln Glu Gln Leu Met
1               5                   10                  15

Asn Ile Tyr Glu Ala Ala Glu Gln Thr Leu Lys Ala Gln Ile Leu His
            20                  25                  30

Trp Gln Thr Leu Arg Lys Glu Ala Val Thr Leu Tyr Phe Ala Arg Gln
        35                  40                  45

Lys Gly Ile Asn Arg Leu Gly Tyr Gln Pro Val Pro Ala Leu Ala Ile
    50                  55                  60

Ser Glu Ala Arg Ala Lys Glu Ala Ile Tyr Met Val Leu Gln Leu Glu
65                  70                  75                  80

Ser Leu Gln Lys Ser Ala Phe Ala Leu Glu Pro Trp Thr Leu Val Asp
                85                  90                  95

Thr Ser Thr Glu Thr Phe Lys Ser Ala Pro Glu Asn His Phe Lys Lys
            100                 105                 110

Gly Pro Val Pro Val Glu Val Ile Tyr Asp Lys Asp Glu Ala Asn Ala
        115                 120                 125

Asn Leu Tyr Thr Met Trp Thr Phe Val Tyr Tyr Met Asp Ser Asp Asp
    130                 135                 140

Val Trp His Lys Thr Thr Ser Gly Val Asn Gln Thr Gly Ile Tyr Tyr
145                 150                 155                 160

Leu Val Glu Asn Met Lys Gln Phe Tyr Cys Asn Phe Lys Glu Glu Ala
                165                 170                 175

Lys Lys Tyr Gly Val Thr Asp Gln Trp Glu Val His Asp Gly Asn Gln
            180                 185                 190

Val Ile Val Ser Pro Ala Pro Ile Ser Ser
        195                 200

<210>  46
<211>  202
<212>  PRT
<213>  Human papillomavirus

<400>  46

Met Glu Asn Leu Ser Glu Arg Phe Asn Val Leu Gln Asp Gln Leu Met
1               5                   10                  15

Asn Ile Tyr Glu Ala Ala Glu His Thr Leu Glu Thr Gln Ile Ala His
            20                  25                  30

Trp Thr Leu Leu Arg Arg Glu Ala Val Leu Leu Tyr Tyr Ala Arg Gln
        35                  40                  45

Lys Gly Ile Thr Arg Leu Gly Tyr Gln Pro Val Pro Thr Leu Ala Val
    50                  55                  60

Ser Glu Ala Lys Ala Lys Glu Ala Ile Gly Ile Met Leu Gln Leu Gln
65                  70                  75                  80

Ser Leu Gln Lys Ser Glu Tyr Ala Ser Glu Asn Trp Thr Leu Val Asp
                85                  90                  95

Thr Ser Ala Glu Thr Tyr Asn Asn Val Pro Glu His His Phe Lys Lys
            100                 105                 110

Gly Pro Val Pro Val Glu Val Ile Tyr Asp Lys Glu Pro Glu Asn Ala
        115                 120                 125

Asn Val Tyr Thr Met Trp Lys Tyr Val Tyr Tyr Met Asp Pro Glu Asp
    130                 135                 140

Val Trp His Lys Thr Thr Ser Gly Val Asn Gln Thr Gly Ile Tyr Tyr
145                 150                 155                 160

Leu His Gly Asp Phe Lys His Tyr Tyr Val Leu Phe Ala Asp Gly Ala
                165                 170                 175

Arg Met Tyr Ser Lys Thr Gly Gln Trp Glu Val Lys Val Asn Lys Glu
            180                 185                 190

Thr Val Phe Ala Pro Val Thr Ser Ser Thr
        195                 200


<210>  47
<211>  202
<212>  PRT
<213>  Human papillomavirus

<400>  47

Met Glu Asn Leu Ser Glu Arg Phe Asn Val Leu Gln Asp Gln Leu Met
1               5                   10                  15

Asn Ile Tyr Glu Ala Ala Glu Gln Thr Leu Glu Ala Gln Ile Ala His
            20                  25                  30

Trp Leu Leu Leu Arg Lys Glu Ala Val Leu Leu Tyr Phe Ala Arg Gln
        35                  40                  45

Lys Gly Ile Thr Arg Ile Gly Tyr Gln Pro Val Pro Pro Leu Ala Val
    50                  55                  60

Ser Glu Ala Lys Ala Lys Gln Ala Ile Gly Ile Met Leu Gln Leu Gln
65                  70                  75                  80

Ser Leu Gln Lys Ser Glu Phe Ala Asp Glu Pro Trp Thr Leu Val Asp
                85                  90                  95

Thr Ser Ile Glu Thr Tyr Lys Asn Ala Pro Glu Asn His Phe Lys Lys
            100                 105                 110

Gly Ala Thr Pro Val Glu Val Ile Tyr Asp Lys Gln Pro Asp Asn Ala
        115                 120                 125

Asn Val Tyr Thr Met Trp Lys His Ile Tyr Tyr Thr Asp Ala Asp Asp
    130                 135                 140

Lys Trp His Lys Thr Thr Ser Gly Val Asn Gln Thr Gly Ile Tyr Tyr
145                 150                 155                 160

Met Gln Gly Ser Phe Arg His Tyr Tyr Val Val Phe Ala Asp Asp Ala
                165                 170                 175

Arg Arg Tyr Ser Ala Thr Gly Glu Trp Glu Val Lys Ile Asn Lys Asp
            180                 185                 190

Thr Val Phe Ala Pro Val Thr Ser Ser Thr
        195                 200

<210>  48
<211>  202
<212>  PRT
<213>  Human papillomavirus

<400>  48

Met Glu Asn Leu Ser Asp Arg Phe Asn Val Leu Gln Asp Gln Leu Met
1               5                   10                  15

Asn Ile Tyr Glu Ser Ala Ala Asn Thr Ile Glu Ser Gln Ile Glu His
            20                  25                  30

Trp Gln Thr Leu Arg Lys Glu Ala Val Leu Leu Tyr Phe Ala Arg Gln
        35                  40                  45

Lys Gly Val Thr Arg Leu Gly Tyr Gln Tyr Val Pro Pro Leu Ala Val
    50                  55                  60

Ser Glu Ser Arg Ala Lys Gln Ala Ile Gly Met Met Leu Gln Leu Gln
65                  70                  75                  80

Ser Leu Gln Lys Ser Glu Tyr Ala Lys Glu Pro Trp Ser Leu Val Asp
                85                  90                  95

Thr Ser Ala Glu Thr Phe Arg Ser Pro Pro Glu Asn His Phe Lys Lys
            100                 105                 110

Gly Pro Val Ser Val Glu Val Ile Tyr Asp Asn Asp Lys Asp Asn Ala
        115                 120                 125

Asn Ala Tyr Thr Met Trp Arg Tyr Val Tyr Tyr Val Asp Asp Asp Asp
    130                 135                 140

Gln Trp His Lys Ser Pro Ser Gly Val Asn His Thr Gly Ile Tyr Phe
145                 150                 155                 160

Met Gln Gly Thr Phe Arg His Tyr Tyr Val Leu Phe Ala Asp Asp Ala
                165                 170                 175

Ser Arg Tyr Ser Arg Thr Gly His Trp Glu Val Asn Val Asn Lys Glu
            180                 185                 190

Thr Val Phe Ala Pro Val Thr Ser Ser Thr
        195                 200

<210>  49
<211>  202
<212>  PRT
<213>  Human papillomavirus

<400>  49

Met Glu Asn Leu Ser Asp Arg Phe Asn Ala Leu Gln Asp Gln Leu Met
1               5                   10                  15

Asn Ile Tyr Glu Thr Ala Ala Asn Thr Leu Glu Ser Gln Ile Glu His
            20                  25                  30

Trp Gln Thr Leu Arg Lys Glu Ala Val Leu Leu Tyr Phe Ala Arg Gln
        35                  40                  45

Asn Gly Val Thr Arg Leu Gly Tyr Gln Val Val Pro Thr Leu Ala Ile
    50                  55                  60

Ser Glu Ala Lys Ala Lys Gln Ala Ile Gly Met Val Leu Gln Leu Gln
65                  70                  75                  80

Ser Leu Gln Lys Ser Gln Phe Gly Ser Glu Pro Trp Ser Leu Val Asp
                85                  90                  95

Thr Ser Gly Glu Thr Phe Arg Ser Ala Pro Glu Asn His Phe Lys Lys
            100                 105                 110

Gly Pro Val Ser Val Glu Val Ile Tyr Asp Asn Asp Lys Asp Asn Ala
        115                 120                 125

Asn Ala Tyr Thr Met Trp Lys His Ile Tyr Tyr Gln Asp Asp Asp Glu
    130                 135                 140

Gln Trp His Lys Ser Ala Ser Gly Val Asn His Thr Gly Ile Tyr Tyr
145                 150                 155                 160

Met Gln Gly Thr Phe Arg Asn Tyr Tyr Val Leu Phe Ala Asp Asp Ala
                165                 170                 175

Thr Arg Tyr Ser Lys Thr Gly His Trp Glu Val Lys Val Asn Lys Glu
            180                 185                 190

Thr Val Phe Thr Pro Val Thr Ser Ser Thr
        195                 200

<210>  50
<211>  202
<212>  PRT
<213>  Human papillomavirus

<400>  50

Met Glu Asn Leu Ser Lys Arg Phe Asn Ala Leu Gln Asp Gln Leu Met
1               5                   10                  15

Asn Ile Tyr Glu Ser Ala Pro Asp Thr Leu Glu Ser Gln Ile Glu His
            20                  25                  30

Trp Gln Thr Leu Arg Lys Glu Ala Val Leu Leu Tyr Phe Ala Arg Gln
        35                  40                  45

His Gly Ile Ser Arg Val Gly Tyr Gln Pro Val Pro Val Leu Ala Val
    50                  55                  60

Ser Glu Ala Lys Ala Lys Gln Ala Ile Gly Met Val Leu Arg Leu Gln
65                  70                  75                  80

Ser Leu Gln Lys Ser Glu Tyr Gly Ser Glu Pro Trp Ser Leu Val Asp
                85                  90                  95

Ala Ser Ala Glu Thr Phe Arg Ser Pro Pro Glu Asn His Phe Lys Lys
            100                 105                 110

Gly Pro Ile Ser Val Glu Val Ile Tyr Asp Lys Asp Lys Asp Asn Ala
        115                 120                 125

Asn Ala Tyr Thr Met Trp Arg Phe Val Tyr Tyr Gln Asp Asp Asp Asp
    130                 135                 140

Lys Trp His Lys Ser Ala Ser Gly Val Asn Gln Thr Gly Ile Tyr Phe
145                 150                 155                 160

Met Gln Gly Thr Phe Arg His Tyr Tyr Val Leu Phe Ala Asp Asp Ala
                165                 170                 175

Ser Arg Tyr Ser Thr Thr Gly Gln Trp Glu Val Lys Val Asn Lys Glu
            180                 185                 190

Thr Val Phe Ala Pro Val Thr Ser Ser Thr
        195                 200

<210>  51
<211>  202
<212>  PRT
<213>  Human papillomavirus

<400>  51

Met Glu Asn Leu Ser Glu Arg Phe Asn Val Leu Gln Asp Gln Leu Met
1               5                   10                  15

Asn Ile Tyr Glu Thr Ala Ala Gln Thr Leu Glu Ala Gln Ile Glu His
            20                  25                  30

Trp Gln Ile Leu Arg Arg Glu Ala Val Leu Leu Tyr Phe Ala Arg Gln
        35                  40                  45

Lys Gly Val Thr Arg Leu Gly Tyr Gln Pro Val Pro Ala Leu Met Val
    50                  55                  60

Ser Glu Ala Lys Ala Lys Glu Ala Ile Gly Met Val Leu Gln Leu Gln
65                  70                  75                  80

Ser Leu Gln Lys Ser Glu Phe Gly Lys Glu Pro Trp Ser Leu Val Asp
                85                  90                  95

Thr Ser Thr Glu Thr Tyr Lys Ser Pro Pro Glu Asn His Phe Lys Lys
            100                 105                 110

Gly Pro Met Pro Ile Glu Val Ile Tyr Asp Lys Asp Ala Asp Asn Ala
        115                 120                 125

Asn Ala Tyr Thr Met Trp Arg Tyr Ile Tyr Tyr Val Asp Asp Asp Asp
    130                 135                 140

Lys Trp His Lys Ser Ala Ser Gly Val Asn His Thr Gly Ile Tyr Phe
145                 150                 155                 160

Met His Gly Ser Phe Arg His Tyr Tyr Val Leu Phe Ala Asp Asp Ala
                165                 170                 175

Arg Arg Tyr Ser Asn Thr Gly His Trp Glu Val Lys Val Asn Lys Asp
            180                 185                 190

Thr Val Phe Thr Pro Val Thr Ser Ser Thr
        195                 200


<210>  52
<211>  202
<212>  PRT
<213>  Human papillomavirus

<400>  52

Met Glu Asn Leu Ser Glu Arg Phe Asn Val Leu Gln Asp Gln Leu Met
1               5                   10                  15

Asn Ile Tyr Glu Ser Ala Ala Glu Thr Leu Glu Ser Gln Ile Glu His
            20                  25                  30

Trp Gln Ile Leu Arg Lys Glu Ala Val Leu Leu Tyr Phe Ala Arg Arg
        35                  40                  45

Lys Gly Val Thr Arg Ile Gly Tyr Gln Pro Val Pro Thr Leu Ala Val
    50                  55                  60

Ser Glu Ala Lys Ala Lys Glu Ala Ile Gly Met Val Leu Gln Leu Gln
65                  70                  75                  80

Ser Leu Gln Lys Ser Glu Tyr Gly Thr Glu Pro Trp Ser Leu Val Asp
                85                  90                  95

Thr Ser Ala Glu Thr Tyr Arg Ser Ala Pro Glu Asn Tyr Phe Lys Lys
            100                 105                 110

Gly Pro Met Pro Ile Glu Val Ile Tyr Asp Lys Asp Ala Asp Asn Ala
        115                 120                 125

Asn Leu Tyr Thr Met Trp Lys Phe Val Tyr Tyr Val Asp Glu Asp Asp
    130                 135                 140

Asn Trp His Lys Ser Glu Ser Gly Val Asn His Thr Gly Leu Tyr Phe
145                 150                 155                 160

Met Gln Gly Asn Phe Arg His Tyr Tyr Val Leu Phe Ala Asp Asp Ala
                165                 170                 175

Arg Lys Tyr Ser Ala Thr Gly His Trp Glu Val Lys Val Asn Lys Glu
            180                 185                 190

Thr Val Phe Thr Pro Val Thr Ser Ser Thr
        195                 200

<210>  53
<211>  202
<212>  PRT
<213>  Human papillomavirus

<400>  53

Met Glu Thr Leu Arg Glu Arg Phe Asp Val Leu Gln Asp Gln Leu Met
1               5                   10                  15

Asn Ile Tyr Glu Val Ala Ser Asp Thr Leu Glu Ala Gln Ile Glu His
            20                  25                  30

Trp Arg Leu Leu Arg Lys Glu Ala Val Leu Leu Tyr Phe Ala Arg Gln
        35                  40                  45

Asn Gly Val Leu Arg Leu Gly Tyr Gln Pro Val Pro Pro Leu Ala Thr

50                  55                  60

Ser Glu Ala Lys Ala Lys Glu Ala Ile Gly Met Met Leu Gln Leu Gln
65                  70                  75                  80

Ser Leu Gln Lys Ser Ala Tyr Ala Ser Glu Lys Trp Thr Leu Val Asp
                85                  90                  95

Thr Ser Ile Glu Thr Phe Lys Asn Thr Pro Glu Asn His Phe Lys Lys
            100                 105                 110

Gly Pro Val Asn Val Glu Val Ile Tyr Asp Gly Asp Pro Asp Asn Ala
        115                 120                 125

Asn Leu Tyr Thr Met Trp Lys Tyr Val Tyr Tyr Leu Asp Ser Glu Asp
    130                 135                 140

Arg Trp Gln Lys Thr Glu Ser Gly Ala Asn His Thr Gly Ile Tyr Tyr
145                 150                 155                 160

Thr Ile Lys Asp Phe Lys His Tyr Tyr Thr Leu Phe Ala Asp Asp Ala
                165                 170                 175

Lys Lys Tyr Gly Lys Ser Gly Gln Trp Glu Val Arg Ile Asn Lys Glu
            180                 185                 190

Thr Val Phe Ala Pro Val Thr Ser Ser Thr
        195                 200

<210>  54
<211>  202
<212>  PRT
<213>  Human papillomavirus

<400>  54

Met Glu Asn Leu Lys Lys Arg Phe Asp Val Leu Gln Asp Leu Leu Met
1               5                   10                  15

Asn Ile Tyr Glu Gln Gly Ser Asp Thr Leu Glu Ser Gln Ile Glu His
            20                  25                  30

Trp Gln Ala Leu Arg Arg Glu Ala Val Leu Leu Tyr Tyr Ala Arg Gln
        35                  40                  45

Asn Gly Val Leu Arg Leu Gly Tyr Leu Pro Val Pro Pro Leu Ala Thr
    50                  55                  60

Ser Glu Ala Lys Ala Lys Gln Ala Ile Ser Met Val Leu Gln Leu Gln
65                  70                  75                  80

Ser Leu Gln Gln Ser Pro Tyr Gly Thr Glu Lys Trp Thr Leu Val Asp
                85                  90                  95

Thr Ser Ile Glu Thr Phe Lys Asn Thr Pro Glu Asn His Phe Lys Lys
            100                 105                 110

Gly Pro Ile Asn Val Glu Val Ile Tyr Asp Gly Asp Pro Asp Asn Ala
        115                 120                 125

Asn Leu Tyr Thr Met Trp Lys Tyr Val Tyr Tyr Met Asp Asp Asn Asp
    130                 135                 140

Gln Trp Gln Lys Thr Glu Ser Gly Ala Asn His Thr Gly Ile Tyr Tyr
145                 150                 155                 160

Leu Ile Gly Glu Phe Lys His Tyr Tyr Val Leu Phe Ala Asp Asp Ala
                165                 170                 175

Asn Arg Tyr Ser Lys Ser Gly Gln Trp Glu Val Arg Ile Asn Lys Glu
            180                 185                 190

Thr Val Phe Ala Pro Val Thr Ser Ser Thr
        195                 200

<210>  55
<211>  203
<212>  PRT
<213>  Human papillomavirus

<400>  55

Met Glu Ser Leu Asn Asn Arg Leu Asp Trp Leu Gln Glu Gln Leu Leu
1               5                   10                  15

Thr Leu Tyr Glu Lys Asp Ser Lys Asp Ile Glu Asp Gln Ile Met Gln
            20                  25                  30

Trp Asn Leu Leu Arg Gln Glu Gln Val Leu Phe His Tyr Ala Arg Lys
        35                  40                  45

Lys Gly Ile Met Arg Leu Gly Leu Gln Val Val Pro Ser Leu Ala Ala
    50                  55                  60

Ser Gln Asp Lys Ala Lys Thr Ala Ile Glu Met Thr Leu Tyr Leu Ser
65                  70                  75                  80

Gly Leu Arg Asp Ser Gln Tyr Gly Ser Glu Gln Trp Ser Leu Gln Asp
                85                  90                  95

Thr Ser Arg Glu Ile Phe Leu Ala Pro Pro Asp His Thr Phe Lys Lys
            100                 105                 110

Gly Gly Gln Thr Ile Glu Val Ile Tyr Asp Glu Asp Pro Asn Asn Ser
        115                 120                 125

Thr Arg His Thr Val Trp Arg His Ile Tyr Tyr Gln Asn Gly Asp Asn

130                 135                 140

Arg Trp Arg Lys Ala Ala Ser Asp Val Asp Val His Gly Val Phe Tyr
145                 150                 155                 160

Leu Glu Tyr Asp Gly Val Lys Asn Tyr Tyr Val Asp Phe Gln Glu Glu
                165                 170                 175

Ala Asn Arg Tyr Ser Lys Thr Gly Arg Tyr Thr Val Gln Tyr Glu Gly
            180                 185                 190

Lys Arg Phe Thr Asn Val Met Ser Pro Val Asn
        195                 200

<210>  56
<211>  203
<212>  PRT
<213>  Human papillomavirus

<400>  56

Met Glu Asn Leu Ser Ser Arg Leu Asp Leu Leu Gln Glu Gln Leu Met
1               5                   10                  15

Asn Leu Tyr Glu Gln Asp Ser Lys Leu Ile Glu Asp Gln Ile Lys Gln
            20                  25                  30

Trp Asn Leu Ile Arg Gln Glu Gln Val Leu Phe His Phe Ala Arg Lys
        35                  40                  45

Asn Gly Val Met Arg Ile Gly Leu Gln Ala Val Pro Ser Leu Ala Ser
    50                  55                  60

Ser Gln Glu Lys Ala Lys Thr Ala Ile Glu Met Val Leu His Leu Glu
65                  70                  75                  80

Ser Leu Lys Asp Ser Pro Tyr Gly Thr Glu Asp Trp Ser Leu Gln Asp
                85                  90                  95

Thr Ser Arg Glu Leu Phe Leu Ala Pro Pro Ala Gly Thr Phe Lys Lys
            100                 105                 110

Ser Gly Ser Thr Leu Glu Val Thr Tyr Asp Asn Asn Pro Asp Asn Gln
        115                 120                 125

Thr Arg His Thr Ile Trp Asn His Val Tyr Tyr Gln Asn Gly Asp Asp
    130                 135                 140

Val Trp Arg Lys Val Ser Ser Gly Val Asp Ala Val Gly Val Tyr Tyr
145                 150                 155                 160

Leu Glu His Asp Gly Tyr Lys Asn Tyr Tyr Val Leu Phe Ala Glu Glu
                165                 170                 175

Ala Ser Lys Tyr Ser Thr Thr Gly Gln Tyr Ala Val Asn Tyr Arg Gly
            180                 185                 190

Lys Arg Phe Thr Asn Val Met Ser Ser Thr Ser
        195                 200

<210>  57
<211>  200
<212>  PRT
<213>  Human papillomavirus

<400>  57

Leu Thr Glu Arg Ser Asp Ala Leu Gln Glu Gln Ile Leu Asn Leu Tyr
1               5                   10                  15

Glu Gln Asp Ser Lys Asp Ile Gln Ala Gln Ile Gln Tyr Trp Asp Leu
            20                  25                  30

Asn Arg Lys Leu Tyr Val Thr Tyr Tyr Tyr Ala Arg Lys Glu Gly Tyr
        35                  40                  45

Ser His Leu Gly Leu Gln Pro Leu Pro Ala Leu Gln Val Ser Glu Tyr
    50                  55                  60

Lys Ala Lys Gln Ala Ile Glu Met Gly Leu Leu Leu Thr Ser Leu Ser
65                  70                  75                  80

Lys Ser Gln Tyr Ala Ser Glu Leu Trp Gly Leu Thr Asp Thr Ser Ala
                85                  90                  95

Glu Leu Leu Leu Thr Pro Pro Arg Asn Thr Phe Lys Lys Lys Gly Tyr
            100                 105                 110

Thr Val Asn Val Trp Phe Asp Asn Asn Glu Asn Asn Thr Phe Pro Tyr
        115                 120                 125

Thr Asn Trp Glu Tyr Ile Tyr Tyr Gln Asp Asp Ile Glu Gln Trp His
    130                 135                 140

Arg Thr Arg Gly Glu Val Asp Tyr Asn Gly Leu Tyr Phe Thr Glu Asn
145                 150                 155                 160

Asn Gly Asn Arg Ala Tyr Phe Leu Leu Phe Asp Ser Asp Ala Gln Thr
                165                 170                 175

Tyr Ser Gln Thr Gly Thr Trp Thr Val His Tyr Lys Asn Gln Ile Ile
            180                 185                 190

Ser Ala Pro Val Thr Ser Ser Ser
        195                 200

<210>  58

<211>  203
<212>  PRT
<213>  Human papillomavirus

<400>  58

Met Glu Ser Leu Val Ala Arg Phe Asp Ala Leu Gln Glu Glu Ile Leu
1               5                   10                  15

Thr His Ile Glu Ser Gly Asn Thr Thr Leu Glu Ser Gln Ile Lys Tyr
            20                  25                  30

Trp Glu Asn Val Arg Lys Glu Asn Ala Ile Met His Tyr Ala Arg Lys
        35                  40                  45

Gln Gly Leu Thr Lys Leu Gly Leu Gln Pro Leu Pro Ser Leu Leu Ala
    50                  55                  60

Ser Glu Tyr Asn Ala Lys Gln Ala Ile Gln Ile Gln Leu Thr Leu Leu
65                  70                  75                  80

Ser Leu Leu Lys Ser Pro Tyr Ala Ser Glu Pro Trp Thr Leu Pro Glu
                85                  90                  95

Val Ser Ala Glu Leu Ile Asn Thr Pro Pro Gln Asn Val Leu Lys Lys
            100                 105                 110

Gly Gly Tyr Asp Val Thr Val Trp Phe Asp Asp Asp Arg Asn Asn Thr
        115                 120                 125

Met Val Tyr Thr Asn Trp Thr Ala Leu Tyr Tyr Gln Asp Ala Asn Glu
    130                 135                 140

Ile Trp His Lys Val Lys Gly Glu Val Asp Tyr Asn Gly Leu Phe Phe
145                 150                 155                 160

Thr Asp His Thr Gly Glu Arg Ala Tyr Phe Thr Leu Phe Ser Thr Asp
                165                 170                 175

Ala Glu Arg Tyr Ser Gln Thr Gly Leu Trp Thr Val His Phe Lys Thr
            180                 185                 190

Gln Val Ile Ser Ser Ser Val Val Ser Ser Thr
        195                 200


<210>  59
<211>  203
<212>  PRT
<213>  Human papillomavirus

<400>  59

Met Glu Ser Leu Val Ala Arg Phe Asp Ala Leu Gln Glu Ala Ile Leu
1               5                   10                  15

Thr His Ile Glu Ser Gln Asp Asp Thr Leu Glu Ser Gln Ile Arg Tyr
            20                  25                  30

Trp Glu Asn Ile Arg Lys Glu Asn Ala Ile Met His Phe Ala Arg Lys
        35                  40                  45

Gln Gly Leu Thr Lys Leu Gly Leu Gln Pro Leu Pro Thr Leu Ala Val
    50                  55                  60

Thr Glu Tyr Asn Ala Lys Gln Ala Ile Gln Ile His Leu Thr Leu Gln
65                  70                  75                  80

Ser Leu Leu Lys Ser Pro Tyr Gly Ser Glu Arg Trp Thr Leu Pro Glu
                85                  90                  95

Val Ser Ala Glu Leu Ile Asn Thr Ala Pro Gln Asn Cys Leu Lys Lys
            100                 105                 110

Gly Gly Tyr Asp Val Ser Val Trp Phe Asp Asn Asp Arg Tyr Asn Ala
        115                 120                 125

Met Val Tyr Thr Asn Trp Asp Tyr Leu Tyr Tyr Gln Asp Val Asn Glu
    130                 135                 140

Ile Trp His Lys Val Lys Gly Glu Val Asp Tyr Asp Gly Leu Tyr Phe
145                 150                 155                 160

Thr Asp His Thr Gly Glu Arg Ala Tyr Phe Thr Leu Phe Ser Thr Asp
                165                 170                 175

Ala His Arg Phe Ser Arg Thr Gly Leu Trp Thr Val His Phe Lys Thr
            180                 185                 190

Gln Val Ile Ser Ser Ser Val Val Ser Ser Thr
        195                 200


<210>  60
<211>  203
<212>  PRT
<213>  Human papillomavirus

<400>  60

Met Glu Ser Leu Val Ala Arg Phe Asp Ala Leu Gln Glu Ala Ile Leu
1               5                   10                  15

Thr His Ile Glu Ser Gln Glu Ser Thr Leu Glu Ser Gln Ile Gln Tyr
            20                  25                  30

Trp Glu Asn Ile Arg Lys Glu Asn Ala Ile Met His Tyr Ala Arg Lys
        35                  40                  45

Gln Gly Leu Thr Lys Leu Gly Leu Gln Pro Leu Pro Thr Leu Ala Val
    50                  55                  60

Thr Glu Tyr Asn Ala Lys Gln Ala Ile Gln Ile His Leu Thr Leu Gln
65                  70                  75                  80

Ser Leu Leu Lys Ser Pro Phe Ala Ser Glu Arg Trp Thr Leu Thr Asp
                85                  90                  95

Val Ser Ala Glu Leu Ile Asn Thr Ser Pro Gln Asn Cys Leu Lys Lys
            100                 105                 110

Gly Gly Tyr Asp Val Ala Val Trp Phe Asp Asn Asp Arg Gln Asn Ala
        115                 120                 125

Met Leu Tyr Thr Asn Trp Asp Phe Leu Tyr Tyr Gln Asp Met Asn Glu
    130                 135                 140

Gln Trp His Lys Val Lys Gly Glu Val Asp Tyr Asp Gly Leu Tyr Phe
145                 150                 155                 160

Thr Asp His Thr Gly Glu Arg Ala Tyr Phe Thr Leu Phe Ser Ser Asp
                165                 170                 175

Ala Gln Arg Phe Ser Arg Thr Gly Leu Trp Thr Val His Phe Lys Thr
            180                 185                 190

Gln Val Ile Ser Ser Pro Ile Val Ser Ser Thr
        195                 200

<210>  61
<211>  202
<212>  PRT
<213>  Human papillomavirus

<400>  61

Glu Thr Leu Ser Ala Arg Phe Leu Ala Gln Gln Asp Ile Gln Leu Asn
1               5                   10                  15

Leu Ile Glu Lys Asp Ser Lys Asn Leu Lys Asp His Ile Asp Tyr Trp
            20                  25                  30

Glu Ser Met Arg Lys Glu Gln Val Leu Ala Phe Tyr Ala Lys Lys Glu
        35                  40                  45

Asn Met Ser Arg Leu Gly Leu Gln Pro Leu Pro Pro Ala Lys Val Ser
    50                  55                  60

Glu Gln Lys Ala Lys Asp Ala Ile Arg Ile Gln Leu Leu Leu Gln Ser
65                  70                  75                  80

Leu Tyr Lys Ser Asp Phe Gly Ser Glu Pro Trp Thr Leu Ser Glu Cys
                85                  90                  95

Ser Leu Glu Met Leu Asn Ala Pro Pro Arg Asn Cys Phe Lys Lys Gln
            100                 105                 110

Pro Phe Thr Val Thr Val Gln Phe Asp Asn Asp Pro Lys Asn Val Tyr
        115                 120                 125

Pro Tyr Ile Cys Tyr Glu Tyr Ile Tyr Tyr Gln Asp Asp Arg Asp Lys
    130                 135                 140

Trp His Lys Val Lys Gly Leu Val Asp His Asn Gly Leu Tyr Phe Lys
145                 150                 155                 160

Glu Val Thr Gly Asp Ser Val Tyr Phe Lys Leu Phe Gln Pro Asp Ala
                165                 170                 175

Thr Val Tyr Gly Lys Ser Gly Gln Trp Thr Val Ile Phe Lys Asn Lys
            180                 185                 190

Thr Ile His Ser Ser Val Thr Ser Ser Ser
        195                 200


<210>  62
<211>  202
<212>  PRT
<213>  Human papillomavirus

<400>  62

Glu Ser Leu Ser Thr Arg Phe Ala Ala Gln Gln Glu Ile Gln Leu Thr
1               5                   10                  15

Leu Ile Glu Lys Glu Ser Tyr Asp Leu Lys Asp His Leu Ala Tyr Trp
            20                  25                  30

Lys Ala Val Arg Leu Glu Asn Val Ile Ala Tyr Tyr Ala Arg Lys Glu
        35                  40                  45

His Ile Thr Lys Leu Gly Leu Gln Pro Leu Pro Thr Leu Ala Val Thr
    50                  55                  60

Glu Tyr Lys Ala Lys Glu Ala Ile Asn Ile Gln Leu Leu Ile Gln Ser
65                  70                  75                  80

Leu Leu Lys Ser Glu Phe Ala Leu Glu Arg Trp Thr Leu Ala Glu Thr
                85                  90                  95

Ser Ala Glu Thr Ile Asn Ser Ser Pro Arg Asn Cys Phe Lys Lys Val
            100                 105                 110

Pro Phe Ile Val Asn Val Trp Phe Asp Asn Asp Glu Arg Asn Ser Phe
        115                 120                 125

Pro Tyr Thr Cys Trp Asp Phe Ile Tyr Tyr Gln Asp Asp Gln Asn Lys
    130                 135                 140

Trp His Lys Thr Glu Gly Leu Val Asp His Asn Gly Cys Tyr Tyr Val
145                 150                 155                 160

Asp Leu Asn Gly Asp Phe Val Tyr Phe Thr Leu Phe Gln Pro Asp Ala
                165                 170                 175

Val Lys Tyr Gly Lys Thr Gly Leu Trp Thr Val Arg Phe Lys Asn Lys
            180                 185                 190

Thr Ile Ser Ala Ser Val Thr Ser Ser Ser
        195                 200

<210>  63
<211>  203
<212>  PRT
<213>  Human papillomavirus

<400>  63

Met Glu Arg Leu Leu Glu Arg Leu Asp Tyr Ile Gln Glu Gln Ile Leu
1               5                   10                  15

Thr Leu Tyr Glu Lys Asp Ser Val Asp Leu Glu Asp His Ile Arg Leu
            20                  25                  30

Trp Asn Leu Leu Arg Arg Glu Asn Ala Ile Trp Tyr Val Leu Arg Gln
        35                  40                  45

Glu Gly His Ala Arg Val Gly Gly Arg Ala Val Pro Ala Met Thr Val
    50                  55                  60

Ser Glu Ala Asn Ala Lys Phe Ala Ile Glu Met Gln Ile Lys Leu Glu
65                  70                  75                  80

Ser Leu Lys Ala Ser Pro Tyr Ala Ala Glu Gly Trp Ser Leu Gln Glu
                85                  90                  95

Thr Thr Lys Glu Arg Tyr Leu Ala Glu Pro Ser Arg Thr Phe Lys Lys
            100                 105                 110

Leu Gly Gln Pro Val Thr Leu Met Phe Asp Asn Asp Pro Glu Asn Leu
        115                 120                 125

Thr Glu Val Val Leu Trp Lys Trp Val Tyr Tyr Ile Thr Pro Thr Asp
    130                 135                 140

Glu Trp Tyr Lys Ala Arg Gly Gly Ile Asp Asp Thr Gly Ile Tyr Tyr
145                 150                 155                 160

Ile Asp His Glu Ser Val Lys Met Tyr Tyr Val Arg Phe Asp Met Glu
                165                 170                 175

Ala Glu Asn Phe Ser Glu Thr Gly Thr Val Thr Tyr Arg Leu Gly Ser
            180                 185                 190

Ala Leu Val Asn Val Pro Glu Pro Val Thr Val
        195                 200

<210>  64
<211>  12
<212>  PRT
<213>  artificial

<220>
<223>  consensus sequence

<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<400>  64

Xaa Trp Xaa Xaa Xaa Arg Xaa Xaa Xaa Xaa Xaa Xaa
1               5                   10

<210>  65
<211>  13
<212>  PRT
<213>  artificial

<220>
<223>  consensus sequence

<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<400>  65

Xaa Trp Xaa Xaa Xaa Arg Xaa Xaa Xaa Xaa Xaa Xaa Xaa
1               5                   10

<210>  66
<211>  14
<212>  PRT
<213>  artificial

<220>
<223>  consensus sequence

<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<220>
<221>  MISC_FEATURE
<222>  (14)..(14)
<223>  X= K, T, A, Y, F, or V

<400>  66

Xaa Trp Xaa Xaa Xaa Arg Xaa Xaa Xaa Xaa Xaa Xaa Xaa Xaa
1               5                   10


<210>  67
<211>  15
<212>  PRT
<213>  artificial

<220>
<223>  consensus sequence


<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<220>
<221>  MISC_FEATURE
<222>  (14)..(14)
<223>  X= K, T, A, Y, F, or V

<220>
<221>  MISC_FEATURE
<222>  (15)..(15)
<223>  X= A, I, L, M, F, W, V, or Y

<400>  67

Xaa Trp Xaa Xaa Xaa Arg Xaa Xaa Xaa Xaa Xaa Xaa Xaa Xaa Xaa
1               5                   10                  15


<210>  68
<211>  16
<212>  PRT
<213>  artificial

<220>
<223>  consensus sequence


<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)

<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<220>
<221>  MISC_FEATURE
<222>  (14)..(14)
<223>  X= K, T, A, Y, F, or V

<220>
<221>  MISC_FEATURE
<222>  (15)..(15)
<223>  X= A, I, L, M, F, W, V, or Y

<220>
<221>  MISC_FEATURE
<222>  (16)..(16)
<223>  X= K, R, or H

<400>  68

Xaa Trp Xaa Xaa Xaa Arg Xaa Xaa Xaa Xaa Xaa Xaa Xaa Xaa Xaa Xaa
1               5                   10                  15


<210>  69
<211>  17
<212>  PRT
<213>  artificial

<220>
<223>  consensus sequence


<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE

<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<220>
<221>  MISC_FEATURE
<222>  (14)..(14)
<223>  X= K, T, A, Y, F, or V

<220>
<221>  MISC_FEATURE
<222>  (15)..(15)
<223>  X= A, I, L, M, F, W, V, or Y

<220>
<221>  MISC_FEATURE
<222>  (16)..(16)
<223>  X= K, R, or H

<220>
<221>  MISC_FEATURE
<222>  (17)..(17)
<223>  X= K, R, Q or E

<400>  69

Xaa Trp Xaa Xaa Xaa Arg Xaa Xaa Xaa Xaa Xaa Xaa Xaa Xaa Xaa Xaa
1               5                   10                  15


Xaa



<210>  70
<211>  18
<212>  PRT
<213>  artificial sequence

<220>

<223>  consensus sequence

<220>
<221>  MISC_FEATURE
<222>  (1)..(1)
<223>  X= H, D, Q or L

<220>
<221>  MISC_FEATURE
<222>  (3)..(3)
<223>  X= S, T, K, R, N, D, C, G, Q, H or E

<220>
<221>  MISC_FEATURE
<222>  (4)..(4)
<223>  X= S, T, KN, C, H, L, Y, A, or I

<220>
<221>  MISC_FEATURE
<222>  (5)..(5)
<223>  X= T, R, N, A, I, L, M, F, W, V or Y

<220>
<221>  MISC_FEATURE
<222>  (7)..(7)
<223>  X= L, M, I, W, Y, H, Q, K, or R

<220>
<221>  MISC_FEATURE
<222>  (8)..(8)
<223>  X= L or E

<220>
<221>  MISC_FEATURE
<222>  (9)..(9)
<223>  X= C, N, A, S, Q or P

<220>
<221>  MISC_FEATURE
<222>  (10)..(10)
<223>  X= A, I, L, M, F, W, V or Y

<220>
<221>  MISC_FEATURE
<222>  (11)..(11)
<223>  X= A, I, L, M, F, W, V or Y

<220>
<221>  MISC_FEATURE
<222>  (12)..(12)
<223>  X= Q, A, I, L, M, F, W, V, Y

<220>
<221>  MISC_FEATURE
<222>  (13)..(13)
<223>  X= H, Y, F or W

<220>
<221>  MISC_FEATURE
<222>  (14)..(14)
<223>  X= K, T, A, Y, F, or V

<220>
<221>  MISC_FEATURE
<222>  (15)..(15)
<223>  X= A, I, L, M, F, W, V, or Y

<220>

<221>  MISC_FEATURE
<222>  (16)..(16)
<223>  X= K, R, or H

<220>
<221>  MISC_FEATURE
<222>  (17)..(17)
<223>  X= K, R, Q or E

<220>
<221>  MISC_FEATURE
<222>  (18)..(18)
<223>  X= K, R, Q, E, M, L, G, H, A, N

<400>  70

Xaa Trp Xaa Xaa Xaa Arg Xaa Xaa Xaa Xaa Xaa Xaa Xaa Xaa Xaa Xaa
1               5                   10                  15

Xaa Xaa

## Description

### Field of the invention

The present invention relates to cancer treatment and, more specifically, to a polypeptide inducing apoptosis for treating cancer.

### Background of the invention

Apoptosis is essential for the maintenance of tissue size and cell number homeostasis of multi-cellular organisms, and apoptotic abnormalities are thought to play an important role in the development of various neoplastic diseases as well as a number of neurodegenerative diseases.

Notably, many studies have established the implication of apoptosis defaults in tumor progression. For tumor treatment, it is thus necessary to develop new apoptosis inducers.

Viruses have evolved complex strategies to interact with the cellular apoptotic machinery, and exhibit both pro- and anti-apoptotic functions. Notably, viral anti-apoptotic activities enable viruses to escape immune surveillance, while pro-apoptotic activities serve specific functions such as release of viral particles at the end of the viral vegetative cycle. Oncogenic, or "high-risk", genital Human Papillomaviruses (HPV) display well-characterized anti-apoptotic activities notably mediated by the E6 oncogene, which interferes with both p53- and Fas-mediated apoptosis by targeting p53 and FADD for proteasomal degradation (SCHEFFNER et al., Cell, vol.75, p: 495-505, 1993; FILIPPOVA et al., J. Biol. Chem., vol.279, p: 25729-25744, 2004). HPV pro-apoptotic functions are provided by the E7 oncogene (JONES et al., Virology, vol.239, p: 97-107, 1997), as well as by the viral E2 transcription factor (BLACHON et al., J. Biol. Chem., vol.280, p:36088-36098, 2005; DEMERET et al., Oncogene, vol.22, p:168-175, 2003). However, the pro-apoptotic function of E2 is restricted to the oncogenic HPV types, and not to the low-risk types which are only associated with benign lesions, indicating a putative link between the pro-apoptotic function of E2 and the oncogenic potential of HPVs (BLACHON *et al.,* abovementioned, 2005).

### Summary of the invention

The present invention relates to a polypeptide comprising the sequence X₁WX₂X₃X₄RX₅X₆X₇ X₈ X₉ (SEQ ID NO:1), and derivatives thereof, wherein:
- X₁ is an amino acid selected in the group consisting in histidine (H), tyrosine (Y), aspartic acid (D), glutamine (Q), and leucine (L), preferably histidine (H) or tyrosine (Y);
- X₂ is a polar amino acid, charged or not, and selected in the group consisting in serine (S) threonine (T), lysine (K), arginine (R), asparagine (N), aspartic acid (D), cysteine (C), glycine (G), glutamine (Q), histidine (H), and glutamic acid (E);
- X₃ is a polar amino acid, charged or not, and selected in the group consisting in serine (S), threonine (T), asparagine (N), cysteine (C), histidine (H), leucine (L), tyrosine (Y), alanine (A), and isoleucine (I);
- X₄ is an amino acid selected in the group consisting in threonine (T), arginine (R), asparagine (N), alanine (A), isoleucine (I), leucine (L), methionine (M), phenylalanine (F), tryptophane (W), valine (V) and tyrosine;
- X₅ is an amino acid selected in the group consisting in leucine (L), methionine (M), isoleucine (I), tryptophane (W), tyrosine (Y), histidine (H), glutamine (Q), lysine (K), and arginine (R);
- X₆ is an amino acid selected in the group consisting in leucine (L), and glutamic acid (E), preferably glutamic acid (E)
- X₇ is an amino acid selected in the group consisting in cysteine (C), asparagine (N), alanine (A), tyrosine (Y), serine (S), glutamine (Q), and proline (P);
- X₈ is a hydrophobic amino acid selected in the group consisting in alanine (A), isoleucine (I), leucine (L), methionine (M), phenylalanine (F), tryptophane (W), valine (V) and tyrosine; and
- X₉ is a threonine or an hydrophobic amino acid selected in the group consisting in alanine (A), isoleucine (I), leucine (L), methionine (M), phenylalanine (F), tryptophane (W), valine (V) and tyrosine.

In another embodiment, the present invention relates to a nucleic acid encoding for a polypeptide as defined previously.

In still another embodiment, the present invention relates to a vector comprising a nucleic acid as defined previously.

In still another embodiment, the present invention relates to a composition comprising a polypeptide as defined previously, a nucleic acid encoding for such a polypeptide, or a vector comprising said nucleic acid, optionally associated with a pharmaceutically acceptable vehicle.

In still another embodiment, the present invention finally relates to a use of a composition as defined previously for treating cancer in a subject.

Finally, the present invention also relates to a method for treating cancer comprising administrating a therapeutically effective amount of a composition as defined previously to a subject.

### Brief description of the drawings

Figure 1 shows the inhibition of E2-induced apoptosis by caspase 8 inhibitors.
Figure 2 shows that the E2-caspase 8 filament formation precedes cell death.
Figure 3 shows that GFP-E2/capsase 8 filament formation occurs without caspase activation.
Figure 4 shows that E2 caspase 8 filament formation correlates with E2 pro-apoptotic activity and is mediated through direct binding of E2 to the DED-containing pro-domain of caspase 8.
Figure 5 shows that E2-caspase 8 filament formation is inhibited by MC159 and that E2 enhances Death-Receptor mediated apoptosis.
Figure 6 shows that the second helix of the amino-terminal domain of E2 recapitulates E2-induced filament formation and cell death.
Figure 7 shows the alignment of amino-terminal domain of E2 proteins from different types of HPV.

### Detailed description

The present invention is based on the discovery of that a small polypeptidic domain of E2 protein from HPV is able to induce apoptosis.

Thus, in a first aspect, the present invention provides a polypeptide comprising the sequence X₁WX₂X₃X₄RX₅X₆X₇ X₈ X₉ (SEQ ID NO:1), and derivatives thereof, wherein:
- X₁ is an amino acid (i.e., an α amino acid), preferably an amino acid selected in the group consisting in histidine (H), tyrosine (Y), aspartic acid (D), glutamine (Q), and leucine (L), preferably histidine (H) or tyrosine (Y).
- X₂ is an amino acid (i.e., an α amino acid), preferably a polar amino acid, charged or not, and selected in the group consisting in serine (S) threonine (T), lysine (K), arginine (R), asparagine (N), aspartic acid (D), cysteine (C), glycine (G), glutamine (Q), histidine (H), and glutamic acid (E).
   Preferably, X₂ is selected in the group consisting in threonine (T), lysine (K), arginine (R), asparagine (N), glutamine (Q), histidine (H), glutamic acid (E), most preferably lysine (K)or glutamine (Q).
- X₃ is an amino acid (i.e., an α amino acid), preferably a polar amino acid, charged or not, and selected in the group consisting in serine (S), threonine (T), asparagine (N), cysteine (C), histidine (H), leucine (L), tyrosine (Y), alanine (A), isoleucine (I) most preferably selected in the group consisting in histidine (H), leucine (L), cysteine (C), or threonine (T).
- X₄ is an amino acid (i.e., an α amino acid), preferably selected in the group consisting in threonine (T), arginine (R), asparagine (N), alanine (A), isoleucine (I), leucine (L), methionine (M), phenylalanine (F), tryptophane (W), valine (V) and tyrosine.
   Preferably, X₄ is an hydrophobic amino acid selected in the group consisting in alanine (A), isoleucine (I), leucine (L), methionine (M), phenylalanine (F), tryptophane (W), valine (V) and tyrosine, most preferably selected in the group consisting in isoleucine (I), valine (V), and leucine (L).
- X₅ is an amino acid (i.e., an α amino acid), preferably selected in the group consisting in leucine (L), methionine (M), isoleucine (I), tryptophane (W), tyrosine (Y), histidine (H), glutamine (Q), lysine (K), and arginine (R), most preferably selected in the group consisting in leucine (L), glutamine (Q), lysine (K), and arginine (R).
- X₆ is an amino acid (i.e., an α amino acid), preferably selected in the group consisting in leucine (L), and glutamic acid (E), most preferably glutamic acid (E).
- X₇ is an amino acid (i.e., an α amino acid), preferably selected in the group consisting in cysteine (C), asparagine (N), alanine (A), tyrosine (Y), serine (S), glutamine (Q), and proline (P), most preferably selected in the group consisting in cysteine (C), asparagine (N), alanine (A), serine (S), and glutamine (Q).
- X₈ is an amino acid (i.e., an α amino acid), preferably X₈ is an hydrophobic amino acid selected in the group consisting in alanine (A), isoleucine (I), leucine (L), methionine (M), phenylalanine (F), tryptophane (W), valine (V) and tyrosine, most preferably selected in the group consisting in isoleucine (I), valine (V), and alanine (A).
- X₉ is an amino acid (i.e., an α amino acid), preferably X₉ is a threonine or an hydrophobic amino acid selected in the group consisting in alanine (A), isoleucine (I), leucine (L), methionine (M), phenylalanine (F), tryptophane (W), valine (V) and tyrosine, most preferably X₉ is an hydrophobic amino, and is selected, as an example, in the group consisting in isoleucine (I), leucine (L), valine (V), and methionine (M).

The polypeptide of the invention is able to induce apoptosis. In view of the example, one of skill in the art can simply identify the claimed polypeptides which induce apoptosis.

In the specification, each amino acid is represented follows:
- Serine (S)
- Threonine (T)
- Asparagine (N)
- Glutamine (Q)
- Lysine (K)
- Arginine (R)
- Histidine (H)
- Glutamic acid (E)
- Aspartic acid (D)
- Cysteines (C)
- Glycine (G)
- Proline (P)
- Alanine (E)
- Isoleucine (I)
- Leucine (L)
- Methionine (M)
- Phenylalanine (F)
- Tryptophane (W)
- Valine (V)
- Tyrosine (Y)

Advantageously, the polypeptide of the invention comprises an amino acid sequence having less than 50 amino acids in length derived from the E2 protein of a HPV virus, preferably less than 25 amino acids in length, and more preferably less than 20 amino acids in length.

More advantageously, the polypeptide of the invention is less than 100 amino acids in length, preferably less than 50 amino acids in length, and more preferably less than 25 amino acids in length.

As used herein, a "derivative" or "an amino acid sequence derived from" refers to an amino acid sequence having at least 70 % of identity with the reference amino acid sequence, preferably at least 80% of identity, and most preferably at least 90% of identity.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004). To get the best local alignment, one can preferably used BLAST software, with the BLOSUM 62 matrix, or the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

Preferably, the polypeptide of the invention comprises an amino acid sequence which is selected in the group comprising YWKHMRLECAI (position 32 to 42 of SEQ ID NO:2), YWKHIRLECVL (position 32 to 42 of SEQ ID NO:3), YWKLIRLECAV (position 32 to 42 of SEQ ID NO:4), HWKLTRMECVL (position 32 to 42 of SEQ ID NO:5), HWKLIRMECAI (position 32 to 42 of SEQ ID NO:6), HWKLIRMECAL (position 32 to 42 of SEQ ID NO:7), HWRLRRIECAL (position 32 to 42 of SEQ ID NO:8), YWNCVRLENAI (position 32 to 42 of SEQ ID NO:9), YWKYVRLENAI (position 32 to 42 of SEQ ID NO:10), YWKCVRMENAI (position 32 to 42 of SEQ ID NO:11), YWKLVRMENVI (position 32 to 42 of SEQ ID NO: 12), YWQLIRWENAI (position 31 to 41 of SEQ ID NO:13), YWQLIRLENAI (position 31 to 41 of SEQ ID NO:14), YWKLVRYECAI (position 32 to 42 of SEQ ID NO:15), YWKLVRYECAI (position 32 to 42 of SEQ ID NO:16), YWTLVRYECAM (position 32 to 42 of SEQ ID NO:17), YWTLLRYEAAM (position 32 to 42 of SEQ ID NO:18), YWKAVRHEYVL (position 32 to 42 of SEQ ID NO:19), YWKAVRQENVI (position 32 to 42 of SEQ ID NO:20), YWKAVRHENVV (position 32 to 42 of SEQ ID NO:21), HWHYVRLENAML(position 32 to 42 of SEQ ID NO:22), HWKCIRYECVL (position 32 to 42 of SEQ ID NO:23), HWKCIRYECVL (position 32 to 42 of SEQ ID NO:24), HWKCLRYESVL (position 35 to 45 of SEQ ID NO:25), HWKCMRHESVL (position 32 to 2 of SEQ ID NO:26), HWKCMRHESVL (position 32 to 42 of SEQ ID NO:27), HWKCIRLESVL (position 32 to 42 of SEQ ID NO:28), HWKCLRIEAAL (position 32 to 42 of SEQ ID NO:29), HWKCLRMEAVV (position 32 to 42 of SEQ ID NO:30), HWKCIRLECAL (position 32 to 42 of SEQ ID NO:31), HWKCIRLECAL (position 32 to 42 of SEQ ID NO:32), HWKHVRHENVL (position 32 to 42 of SEQ ID NO:33), HWKHVRLENVL (position 32 to 42 of SEQ ID NO:34), HWQLLRQEQII (position 32 to 42 of SEQ ID NO:35), HWKLLRQEQIL (position 32 to 42 of SEQ ID NO:36), HWKLLRQEQVL (position 32 to 42 of SEQ ID NO:37), HWQLLRQEQIL (position 32 to 42 of SEQ ID NO:38), HWQLLRQEQVL (position 32 to 42 of SEQ ID NO:39), HWQTLRQEQIL (position 32 to 42 of SEQ ID NO:40), HWKLLRQEQAL (position 32 to 42 of SEQ ID NO:41), HWQTLRKEAVL (position 32 to 42 of SEQ ID NO:42), HWQTLRKEPVL (position 32 to 42 of SEQ ID NO:43), HWQTLRQEAVL (position 32 to 42 of SEQ ID NO:44), HWQTLRKEAVT (position 32 to 42 of SEQ ID NO:45), HWTLLRREAVL (position 32 to 42 of SEQ ID NO:46), HWLLLRKEAVL (position 32 to 42 of SEQ ID NO:47), HWQTLRKEAVL (position 32 to 42 of SEQ ID NO:48), HWQTLRKEAVL (position 32 to 42 of SEQ ID NO:49), HWQTLRKEAVL (position 32 to 42 of SEQ ID NO:50), HWQILRREAVL (position 32 to 42 of SEQ ID NO:51), HWQILRKEAVL (position 32 to 42 of SEQ ID NO:52), HWRLLRKEAVL (position 32 to 42 of SEQ ID NO:53), HWQALRREAVL (position 32 to 42 of SEQ ID NO:54), QWNLLRQEQVL (position 32 to 42 of SEQ ID NO:55), QWNLIRQEQVL (position 32 to 432 of SEQ ID NO:56), YWDLNRKLYVT (position 29 to 39 of SEQ ID NO:57), YWENVRKENAI (position 32 to 42 of SEQ ID NO:58), YWENIRKENAI (position 32 to 43 of SEQ ID NO:59), YWENIRKENAI (position 32 to 42 of SEQ ID NO:60), YWESMRKEQVL (position 31 to 41 of SEQ ID NO:61), YWKAVRLENVI (position 31 to 41 of SEQ ID NO:62), and LWNLLRRENAI (position 32 to 42 of SEQ ID NO:63).

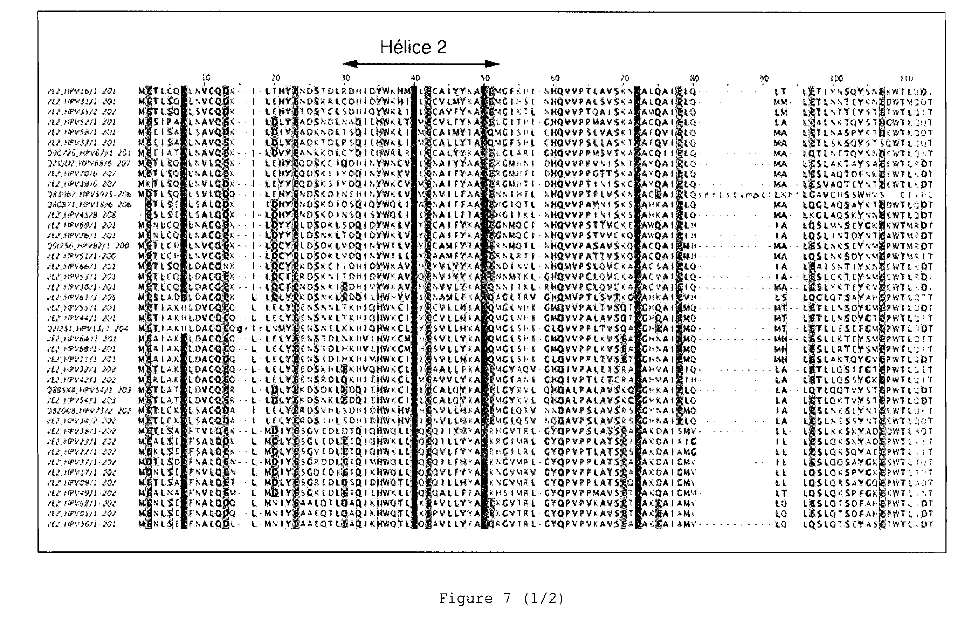

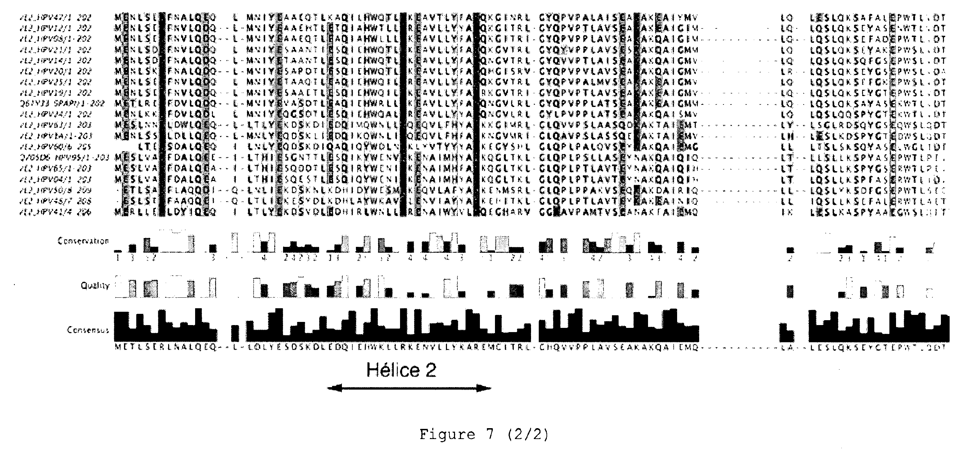

Said sequences correspond to amino acids 36 to 46 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAI (position 31 to 41 of SEQ ID NO:13), YWKHMRLECAI (position 32 to 42 of SEQ ID NO:2), HWKCIRLESVL (position 32 to 42 of SEQ ID NO:28), and HWKCMRHESVL (position 32 to 41 of SEQ ID NO:26).

According to a preferred embodiment, the polypeptide of the invention comprises the sequence X₁WX₂X₃X₄RX₅X₆X₇X₈X₉X₁₀ (SEQ ID NO:64), and derivatives thereof, wherein:
- X₁, X₂, X₃, X₄, X₅, X₆, X₇, X₈, and X₉ are as described previously.
- X₁₀ is an amino acid (i.e., an α amino acid), preferably X₁₀ is a glutamine (Q) or an hydrophobic amino acid selected in the group consisting in alanine (A), isoleucine (I), leucine (L), methionine (M), phenylalanine (F), tryptophane (W), valine (V) and tyrosine, most preferably X₁₀ is an hydrophobic amino, and is selected, as an example, in the group consisting in alanine (A), leucine (L), methionine (M), phenylalanine (F), tryptophane (W), valine (V) and tyrosine.

Preferably, the polypeptide of the invention comprises an amino acid sequence which is selected in the group comprising YWKHMRLECAIY (position 32 to 43 of SEQ ID NO:2), YWKHIRLECVLM (position 32 to 43 of SEQ ID NO:3), YWKLIRLECAVF (position 32 to 43 of SEQ ID NO:4), HWKLTRMECVLF (position 32 to 43 of SEQ ID NO:5), HWKLIRMECAIM (position 32 to 43 of SEQ ID NO:6), HWKLIRMECALL (position 32 to 43 of SEQ ID NO:7), HWRLRRIECALY (position 32 to 43 of SEQ ID NO:8), YWNCVRLENAIY (position 32 to 43 of SEQ ID NO:9), YWKYVRLENAIF (position 32 to 43 of SEQ ID NO:10), YWKCVRMENAIF (position 32 to 43 of SEQ ID NO:11), YWKLVRMENVIL (position 32 to 43 of SEQ ID NO:12), YWQLIRWENAIF (position 31 to 42 of SEQ ID NO:13), YWQLIRLENAIL (position 31 to 42 of SEQ ID NO:14), YWKLVRYECAIF (position 32 to 43 of SEQ ID NO:15), YWKLVRYECAIF (position 32 to 43 of SEQ ID NO:16), YWTLVRYECAMF (position 32 to 43 of SEQ ID NO:17), YWTLLRYEAAMF (position 32 to 43 of SEQ ID NO:18), YWKAVRHEYVLY (position 32 to 434 of SEQ ID NO:19), YWKAVRQENVIY (position 32 to 43 of SEQ ID NO:20), YWKAVRHENVVL (position 32 to 43 of SEQ ID NO:21), HWHYVRLENAML (position 32 to 43 of SEQ ID NO:22), HWKCIRYECVLL (position 32 to 43 of SEQ ID NO:23), HWKCIRYECVLL (position 32 to 43 of SEQ ID NO:24), HWKCLRYESVLL (position 35 to 46 of SEQ ID NO:25), HWKCMRHESVLL (position 32 to 43 of SEQ ID NO:26), HWKCMRHESVLL (position 32 to 43 of SEQ ID NO:27), HWKCIRLESVLL (position 32 to 43 of SEQ ID NO:28), HWKCLRIEAALL (position 32 to 43 of SEQ ID NO:29), HWKCLRMEAVVL (position 32 to 43 of SEQ ID NO:30), HWKCIRLECALQ (position 32 to 43 of SEQ ID NO:31), HWKCIRLECALQ (position 32 to 43 of SEQ ID NO:32), HWKHVRHENVLL (position 32 to 43 of SEQ ID NO:33), HWKHVRLENVLL (position 32 to 43 of SEQ ID NO:34), HWQLLRQEQIIY (position 32 to 43 of SEQ ID NO:35), HWKLLRQEQILL (position 32 to 43 of SEQ ID NO:36), HWKLLRQEQVLF (position 32 to 43 of SEQ ID NO:37), HWQLLRQEQILF (position 32 to 43 of SEQ ID NO:38), HWQLLRQEQVLF (position 32 to 43 of SEQ ID NO:39), HWQTLRQEQILL (position 32 to 43 of SEQ ID NO:40), HWKLLRQEQALL (position 32 to 43 of SEQ ID NO:41), HWQTLRKEAVLL (position 32 to 43 of SEQ ID NO:42), HWQTLRKEPVLL (position 32 to 43 of SEQ ID NO:43), HWQTLRQEAVLL (position 32 to 43 of SEQ ID NO:44), HWQTLRKEAVTL (position 32 to 43 of SEQ ID NO:45), HWTLLRREAVLL (position 32 to 43 of SEQ ID NO:46), HWLLLRKEAVLL (position 32 to 43 of SEQ ID NO:47), HWQTLRKEAVLL (position 32 to 44 of SEQ ID NO:48), HWQTLRKEAVLL (position 32 to 43 of SEQ ID NO:49), HWQTLRKEAVLL (position 32 to 43 of SEQ ID NO:50), HWQILRREAVLL (position 32 to 43 of SEQ ID NO:51), HWQILRKEAVLL (position 32 to 43 of SEQ ID NO:52), HWRLLRKEAVLL (position 32 to 43 of SEQ ID NO:53), HWQALRREAVLL (position 32 to 43 of SEQ ID NO:54), QWNLLRQEQVLF (position 32 to 43 of SEQ ID NO:55), QWNLIRQEQVLF (position 32 to 43 of SEQID NO:56), YWDLNRKLYVTY (position 29 to 40 of SEQ ID NO:57), YWENVRKENAIM (position 32 to 43 of SEQ ID NO:58), YWENIRKENAIM (position 32 to 43 of SEQ ID NO:59), YWENIRKENAIM (position 32 to 43 of SEQ ID NO:60), YWESMRKEQVLA (position 31 to 42 of SEQ ID NO:61), YWKAVRLENVIA (position 31 to 42 of SEQ ID NO:62), and LWNLLRRENAIW (position 32 to 43 of SEQ ID NO:63).

Said sequences correspond to amino acids 36 to 47 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIF (position 31 to 42 of SEQ ID NO:13), YWKHMRLECAIY (position 32 to 43 of SEQ ID NO:2), HWKCIRLESVLL (position 32 to 43 of SEQ ID NO:28), and HWKCMRHESVLL (position 32 to 42 of SEQ ID NO:26).

According to another preferred embodiment, the polypeptide of the invention comprises the sequence X₁WX₂X₃X₄RX₅X₆X₇X₈X₉X₁₀X₁₁ (SEQ ID NO:65), and derivatives thereof, wherein:
- X₁, X₂, X₃, X₄, X₅, X₆, X₇, X₈, X₉, and X₁₀ are as described previously.
- X₁₁ is an amino acid (i.e., an α amino acid), preferably X₁₁ is a histidine (H) or an aromatic amino acid selected in the group consisting in tyrosine (Y), phenylalanine (F), and tryptophane (W), most preferably X₁₁ is selected in the group consisting in histidine (H), tyrosine (Y), and phenylalanine (F).

Preferably, the polypeptide of the invention comprises an amino acid sequence which is selected in the group comprising YWKHMRLECAIYY (position 32 to 44 of SEQ ID NO:2), YWKHIRLECVLMY (position 32 to 44 of SEQ ID NO:3), YWKLIRLECAVFY (position 32 to 44 of SEQ ID NO:4), HWKLTRMECVLFY (position 32 to 44 of SEQ ID NO:5), HWKLIRMECAIMY (position 32 to 44 of SEQ ID NO:6), HWKLIRMECALLY (position 32 to 44 of SEQ ID NO:7), HWRLRRIECALYY (position 32 to 44 of SEQ ID NO:8), YWNCVRLENAIYY (position 32 to 44 of SEQ ID NO:9), YWKYVRLENAIFY (position 32 to 44 of SEQ ID NO:10), YWKCVRMENAIFY (position 32 to 44 of SEQ ID NO:11), YWKLVRMENVILF (position 32 to 44 of SEQ ID NO:12), YWQLIRWENAIFF (position 31 to 43 of SEQ ID NO:13), YWQLIRLENAILF (position 31 to 43 of SEQ ID NO:14), YWKLVRYECAIFY (position 32 to 44 of SEQ ID NO:15), YWKLVRYECAIFY (position 32 to 44 of SEQ ID NO:16), YWTLVRYECAMFY (position 32 to 44 of SEQ ID NO:17), YWTLLRYEAAMFY (position 32 to 44 of SEQ ID NO:18), YWKAVRHEYVLYY (position 32 to 44 of SEQ ID NO:19), YWKAVRQENVIYY (position 32 to 44 of SEQ ID NO:20), YWKAVRHENVVLY (position 32 to 44 of SEQ ID NO:21), HWHYVRLENAMLF (position 32 to 44 of SEQ ID NO:22), HWKCIRYECVLLH (position 32 to 44 of SEQ ID NO:23), HWKCIRYECVLLH (position 32 to 44 of SEQ ID NO:24), HWKCLRYESVLLH (position 35 to 47 of SEQ ID NO:25), HWKCMRHESVLLY (position 32 to 44 of SEQ ID NO:26), HWKCMRHESVLLY (position 32 to 44 of SEQ ID NO:27), HWKCIRLESVLLH (position 32 to 44 of SEQ ID NO:28), HWKCLRIEAALLF (position 32 to 44 of SEQ ID NO:29), HWKCLRMEAVVLY (position 32 to 44 of SEQ ID NO:30), HWKCIRLECALQY (position 32 to 44 of SEQ ID NO:31), HWKCIRLECALQY (position 32 to 44 of SEQ ID NO:32), HWKHVRHENVLLH (position 32 to 44 of SEQ ID NO:33), HWKHVRLENVLLH (position 32 to 44 of SEQ ID NO:34), HWQLLRQEQIIYH (position 32 to 44 of SEQ ID NO:35), HWKLLRQEQILLY (position 32 to 44 of SEQ ID NO:36), HWKLLRQEQVLFY (position 32 to 44 of SEQ ID NO:37), HWQLLRQEQILFH (position 32 to 44 of SEQ ID NO:38), HWQLLRQEQVLFY (position 32 to 44 of SEQ ID NO:39), HWQTLRQEQILLH (position 32 to 44 of SEQ ID NO:40), HWKLLRQEQALLF (position 32 to 44 of SEQ ID NO:41), HWQTLRKEAVLLY (position 32 to 44 of SEQ ID NO:42), HWQTLRKEPVLLY (position 32 to 44 of SEQ ID NO:43), HWQTLRQEAVLLY (position 32 to 44 of SEQ ID NO:44), HWQTLRKEAVTLY (position 32 to 44 of SEQ ID NO:45), HWTLLRREAVLLY (position 32 to 44 of SEQ ID NO:46), HWLLLRKEAVLLY (position 32 to 44 of SEQ ID NO:47), HWQTLRKEAVLLY (position 32 to 44 of SEQ ID NO:48), HWQTLRKEAVLLY (position 32 to 44 of SEQ ID NO:49), HWQTLRKEAVLLY (position 32 to 44 of SEQ ID NO:50), HWQILRREAVLLY (position 32 to 44 of SEQ ID NO:51), HWQILRKEAVLLY (position 32 to 44 of SEQ ID NO:52), HWRLLRKEAVLLY (position 32 to 44 of SEQ ID NO:53), HWQALRREAVLLY (position 32 to 44 of SEQ ID NO:54), QWNLLRQEQVLFH (position 32 to 44 of SEQ ID NO:55), QWNLIRQEQVLFH (position 32 to 44 of SEQ ID NO:56), YWDLNRKLYVTYY (position 29 to 41 of SEQ ID NO:57), YWENVRKENAIMH (position 32 to 44 of SEQ ID NO:58), YWENIRKENAIMH (position 32 to 44 of SEQ ID NO:59), YWENIRKENAIMH (position 32 to 44 of SEQ ID NO:60), YWESMRKEQVLAF (position 31 to 43 of SEQ ID NO:61), YWKAVRLENVIAY (position 31 to 43 of SEQ ID NO:62), and LWNLLRRENAIWY (position 32 to 44 of SEQ ID NO:63).

Said sequences correspond to amino acids 36 to 48 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFF (position 31 to 43 of SEQ ID NO:13), YWKHMRLECAIYY (position 32 to 44 of SEQ ID NO:2), HWKCIRLESVLLH (position 32 to 44 of SEQ ID NO:28), and HWKCMRHESVLLY (position 32 to 43 of SEQ ID NO:26).

According to still another preferred embodiment, the polypeptide of the invention comprises the sequence X₁WX₂X₃X₄RX₅X₆X₇X₈X₉X₁₀X₁₁X₁₂ (SEQ ID NO:66), and derivatives thereof, wherein:
- X₁, X₂, X_{3,} X₄, X₅, X₆, X₇, X₈, X₉, X₁₀, and X₁₁ are as described previously.
- X₁₂ is an amino acid (i.e., an α amino acid), preferably X₁₂ is selected in the group consisting in lysine (K), threonine (T), alanine (A), tyrosine (Y), phenylalanine (F), valine (V), and most preferably X₁₂ is selected in the group consisting in lysine (K), tyrosine (Y), and phenylalanine (F).

Preferably, the polypeptide of the invention comprises an amino acid sequence which is selected in the group comprising YWKHMRLECAIYYK (position 32 to 45 of SEQ ID NO:2), YWKHIRLECVLMYK (position 32 to 45 of SEQ ID NO:3), YWKLIRLECAVFYK (position 32 to 45 of SEQ ID NO:4), HWKLTRMECVLFYK (position 32 to 45 of SEQ ID NO:5), HWKLIRMECAIMYT (position 32 to 45 of SEQ ID NO:6), HWKLIRMECALLYT (position 32 to 45 of SEQ ID NO:7), HWRLRRIECALYYK (position 32 to 45 of SEQ ID NO:8), YWNCVRLENAIYYA (position 32 to 45 of SEQ ID NO:9), YWKYVRLENAIFYA (position 32 to 45 of SEQ ID NO:10), YWKCVRMENAIFYA (position 32 to 45 of SEQ ID NO:11), YWKLVRMENVILFA (position 32 to 45 of SEQ ID NO:12), YWQLIRWENAIFFA (position 31 to 44 of SEQ ID NO:13), YWQLIRLENAILFT (position 31 to 44 of SEQ ID NO:14), YWKLVRYECAIFYK (position 32 to 45 of SEQ ID NO:15), YWKLVRYECAIFYK (position 32 to 45 of SEQ ID NO:16), YWTLVRYECAMFYT (position 32 to 45 of SEQ ID NO:17), YWTLLRYEAAMFYA (position 32 to 45 of SEQ ID NO:18), YWKAVRHEYVLYYK (position 32 to 45 of SEQ ID NO:19), YWKAVRQENVIYYK (position 32 to 45 of SEQ ID NO:20), YWKAVRHENVVLYK (position 32 to 45 of SEQ ID NO:21), HWHYVRLENAMLFK (position 32 to 45 of SEQ ID NO:22), HWKCIRYECVLLHK (position 32 to 45 of SEQ ID NO:23), HWKCIRYECVLLHK (position 32 to 45 of SEQ ID NO:24), HWKCLRYESVLLHK (position 35 to 48 of SEQ ID NO:25), HWKCMRHESVLLYK (position 32 to 45 of SEQ ID NO:26), HWKCMRHESVLLYK (position 32 to 45 of SEQ ID NO:27), HWKCIRLESVLLHK (position 32 to 45 of SEQ ID NO:28), HWKCLRIEAALLFK (position 32 to 45 of SEQ ID NO:29), HWKCLRMEAVVLYK (position 32 to 45 of SEQ ID NO:30), HWKCIRLECALQYK (position 32 to 45 of SEQ ID NO:31), HWKCIRLECALQYK (position 32 to 45 of SEQ ID NO:32), HWKHVRHENVLLHK (position 32 to 45 of SEQ ID NO:33), HWKHVRLENVLLHK (position 32 to 45 of SEQ ID NO:34), HWQLLRQEQIIYHY (position 32 to 45 of SEQ ID NO:35), HWKLLRQEQILLYY (position 32 to 45 of SEQ ID NO:36), HWKLLRQEQVLFYY (position 32 to 45 of SEQ ID NO:37), HWQLLRQEQILFHY (position 32 to 45 of SEQ ID NO:38), HWQLLRQEQVLFYY (position 32 to 45 of SEQ ID NO:39), HWQTLRQEQILLHY (position 32 to 45 of SEQ ID NO:40), HWKLLRQEQALLFF (position 32 to 45 of SEQ ID NO:41), HWQTLRKEAVLLYY (position 32 to 45 of SEQ ID NO:42), HWQTLRKEPVLLYY (position 32 to 45 of SEQ ID NO:43), HWQTLRQEAVLLYF (position 32 to 45 of SEQ ID NO:44), HWQTLRKEAVTLYF (position 32 to 45 of SEQ ID NO:45), HWTLLRREAVLLYY (position 32 to 45 of SEQ ID NO:46), HWLLLRKEAVLLYF (position 32 to 45 of SEQ ID NO:47), HWQTLRKEAVLLYF (position 32 to 45 of SEQ ID NO:48), HWQTLRKEAVLLYF (position 32 to 45 of SEQ ID NO:49), HWQTLRKEAVLLYF (position 32 to 45 of SEQ ID NO:50), HWQILRREAVLLYF (position 32 to 45 of SEQ ID NO:51), HWQILRKEAVLLYF (position 32 to 45 of SEQ ID NO:52), HWRLLRKEAVLLYF (position 32 to 45 of SEQ ID NO:53), HWQALRREAVLLYY (position 32 to 45 of SEQ ID NO:54), QWNLLRQEQVLFHY (position 32 to 45 of SEQ ID NO:55), QWNLIRQEQVLFHF (position 32 to 45 of SEQ ID NO:56), YWDLNRKLYVTYYY (position 29 to 42 of SEQ ID NO:57), YWENVRKENAIMHY (position 32 to 45 of SEQ ID NO:58), YWENIRKENAIMHF (position 32 to 45 of SEQ ID NO:59), YWENIRKENAIMHY (position 32 to 45 of SEQ ID NO:60), YWESMRKEQVLAFY (position 31 to 44 of SEQ ID NO:61), YWKAVRLENVIAYY (position 31 to 44 of SEQ ID NO:62), and LWNLLRRENAIWYV (position 32 to 45 of SEQ ID NO:63).

Said sequences correspond to amino acids 36 to 49 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFFA (position 31 to 44 of SEQ ID NO:13), YWKHMRLECAIYYK (position 32 to 45 of SEQ ID NO:2), HWKCIRLESVLLHK (position 32 to 45 of SEQ ID NO:28), and HWKCMRHESVLLYK (position 32 to 44 of SEQ ID NO:26).

According to another preferred embodiment, the polypeptide of the invention comprises the sequence X₁WX₂X₃X₄RX₅X₆X₇X₈X₉X₁₀X₁₁X₁₂X₁₃ (SEQ ID NO:67), and derivatives thereof, wherein:
- X_{1,} X₂, X₃, X₄, X₅, X₆, X₇, X₈, X₉, X₁₀, X₁₁, and X₁₂ are as described previously.
- X₁₃ is an amino acid (i.e., an α amino acid), preferably X₁₃ is an hydrophobic amino acid selected in the group consisting in alanine (A), isoleucine (I), leucine (L), methionine (M), phenylalanine (F), tryptophane (W), valine (V) and tyrosine, most preferably X₁₃ is an alanine (A) or a leucine (L).
   Preferably, X₁₃ is an alanine (A).

Preferably, the polypeptide of the invention comprises an amino acid sequence which is selected in the group comprising YWKHMRLECAIYYKA (position 32 to 46 of SEQ ID NO:2), YWKHIRLECVLMYKA (position 32 to 46 of SEQ ID NO:3), YWKLIRLECAVFYKA (position 32 to 46 of SEQ ID NO:4), HWKLTRMECVLFYKA (position 32 to 46 of SEQ ID NO:5), HWKLIRMECAIMYTA (position 32 to 46 of SEQ ID NO:6), HWKLIRMECALLYTA (position 32 to 46 of SEQ ID NO:7), HWRLRRIECALYYKA (position 32 to 46 of SEQ ID NO:8), YWNCVRLENAIYYAA (position 32 to 46 of SEQ ID NO:9), YWKYVRLENAIFYAA (position 32 to 46 of SEQ ID NO:10), YWKCVRMENAIFYAA (position 32 to 46 of SEQ ID NO:11), YWKLVRMENVILFAA (position 32 to 46 of SEQ ID NO:12), YWQLIRWENAIFFAA (position 31 to 45 of SEQ ID NO:13), YWQLIRLENAILFTA (position 31 to 45 of SEQ ID NO:14), YWKLVRYECAIFYKA (position 32 to 46 of SEQ ID NO:15), YWKLVRYECAIFYKA (position 32 to 46 of SEQ ID NO:16), YWTLVRYECAMFYTA (position 32 to 46 of SEQ ID NO:17), YWTLLRYEAAMFYAA (position 32 to 46 of SEQ ID NO:18), YWKAVRHEYVLYYKA (position 32 to 46 of SEQ ID NO:19), YWKAVRQENVIYYKA (position 32 to 46 of SEQ ID NO:20), YWKAVRHENVVLYKA (position 32 to 46 of SEQ ID NO:21), HWHYVRLENAMLFKA (position 32 to 46 of SEQ ID NO:22), HWKCIRYECVLLHKA (position 32 to 46 of SEQ ID NO:23), HWKCIRYECVLLHKA (position 32 to 46 of SEQ ID NO:24), HWKCLRYESVLLHKA (position 35 to 49 of SEQ ID NO:25), HWKCMRHESVLLYKA (position 32 to 46 of SEQ ID NO:26), HWKCMRHESVLLYKA (position 32 to 46 of SEQ ID NO:27), HWKCIRLESVLLHKA (position 32 to 46 of SEQ ID NO:28), HWKCLRIEAALLFKA (position 32 to 46 of SEQ ID NO:29), HWKCLRMEAVVLYKA (position 32 to 46 of SEQ ID NO:30), HWKCIRLECALQYKA (position 32 to 46 of SEQ ID NO:31), HWKCIRLECALQYKA (position 32 to 46 of SEQ ID NO:32), HWKHVRHENVLLHKA (position 32 to 46 of SEQ ID NO:33), HWKHVRLENVLLHKA (position 32 to 46 of SEQ ID NO:34), HWQLLRQEQIIYHYA (position 32 to 46 of SEQ ID NO:35), HWKLLRQEQILLYYA (position 32 to 46 of SEQ ID NO:36), HWKLLRQEQVLFYYA (position 32 to 46 of SEQ ID NO:37), HWQLLRQEQILFHYA (position 32 to 46 of SEQ ID NO:38), HWQLLRQEQVLFYYA (position 32 to 46 of SEQ ID NO:39), HWQTLRQEQILLHYA (position 32 to 46 of SEQ ID NO:40), HWKLLRQEQALLFFA (position 32 to 46 of SEQ ID NO:41), HWQTLRKEAVLLYYA (position 32 to 46 of SEQ ID NO:42), HWQTLRKEPVLLYYA (position 32 to 46 of SEQ ID NO:43), HWQTLRQEAVLLYFA (position 32 to 46 of SEQ ID NO:44), HWQTLRKEAVTLYFA (position 32 to 46 of SEQ ID NO:45), HWTLLRREAVLLYYA (position 32 to 46 of SEQ ID NO:46), HWLLLRKEAVLLYFA (position 32 to 46 of SEQ ID NO:47), HWQTLRKEAVLLYFA (position 32 to 46 of SEQ ID NO:48), HWQTLRKEAVLLYFA (position 32 to 46 of SEQ ID NO:49), HWQTLRKEAVLLYFA (position 32 to 46 of SEQ ID NO:50), HWQILRREAVLLYFA (position 32 to 46 of SEQ ID NO:51), HWQILRKEAVLLYFA (position 32 to 46 of SEQ ID NO:52), HWRLLRKEAVLLYFA (position 32 to 46 of SEQ ID NO:53), HWQALRREAVLLYYA (position 32 to 46 of SEQ ID NO:54), QWNLLRQEQVLFHYA (position 32 to 46 of SEQ ID NO:55), QWNLIRQEQVLFHFA (position 32 to 46 of SEQ ID NO:56), YWDLNRKLYVTYYYA (position 29 to 43 of SEQ ID NO:57), YWENVRKENAIMHYA (position 32 to 46 of SEQ ID NO:58), YWENIRKENAIMHFA (position 32 to 46 of SEQ ID NO:59), YWENIRKENAIMHYA (position 32 to 46 of SEQ ID NO:60), YWESMRKEQVLAFYA (position 31 to 45 of SEQ ID NO:61), YWKAVRLENVIAYYA (position 31 to 45 of SEQ ID NO:62), and LWNLLRRENAIWYVL (position 32 to 46 of SEQ ID NO:63).

Said sequences correspond to amino acids 36 to 50 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFFAA (position 31 to 45 of SEQ ID NO:13), YWKHMRLECAIYYKA (position 32 to 46 of SEQ ID NO:2), HWKCIRLESVLLHKA (position 32 to 46 of SEQ ID NO:28), and HWKCMRHESVLLYKA (position 32 to 45 of SEQ ID NO:26).

According to another preferred embodiment, the polypeptide of the invention comprises the sequence X₁WX₂X₃X₄RX₅X₆X₇X₈X₉X₁₀X₁₁X₁₂X₁₃X₁₄ (SEQ ID NO:68), and derivatives thereof, wherein:
- X₁, X₂, X₃, X₄, X₅, X₆, X₇, X₈, X₉, X₁₀, X₁₁, X₁₂, and X₁₃ are as described previously.
- X₁₄ is an amino acid (i.e., an α amino acid), preferably X₁₄ is a positively charged amino acid selected in the group consisting in lysine (K), arginine (R), and histidine (H), and most preferably, X₁₄ is a lysine (K) or an arginine (R).

Preferably, the polypeptide of the invention comprises an amino acid sequence which is selected in the group comprising YWKHMRLECAIYYKAR (position 32 to 47 of SEQ ID NO:2), YWKHIRLECVLMYKAR (position 32 to 7 of SEQ ID NO:3), YWKLIRLECAVFYKAR (position 32 to 47 of SEQ ID NO:4), HWKLTRMECVLFYKAK (position 32 to 47 of SEQ ID NO:5), HWKLIRMECAIMYTAR (position 32 to 47 of SEQ ID NO:6), HWKLIRMECALLYTAK (position 32 to 47 of SEQ ID NO:7), HWRLRRIECALYYKAK (position 32 to 47 of SEQ ID NO:8), YWNCVRLENAIYYAAR (position 32 to 47 of SEQ ID NO:9), YWKYVRLENAIFYAAR (position 32 to 47 of SEQ ID NO:10), YWKCVRMENAIFYAAR (position 32 to 47 of SEQ ID NO:11), YWKLVRMENVILFAAR (position 32 to 47 of SEQ ID NO:12), YWQLIRWENAIFFAAR (position 31 to 46 of SEQ ID NO:13), YWQLIRLENAILFTAR (position 31 to 46 of SEQ ID NO:14), YWKLVRYECAIFYKAR (position 32 to 47 of SEQ ID NO:15), YWKLVRYECAIFYKAR (position 32 to 47 of SEQ ID NO:16), YWTLVRYECAMFYTAR (position 32 to 47 of SEQ ID NO:17), YWTLLRYEAAMFYAAR (position 32 to 47 of SEQ ID NO:18), YWKAVRHEYVLYYKAR (position 32 to 47 of SEQ ID NO:19), YWKAVRQENVIYYKAR (position 32 to 47 of SEQ ID NO:20), YWKAVRHENVVLYKAR (position 32 to 47 of SEQ ID NO:21), HWHYVRLENAMLFKAR (position 32 to 47 of SEQ ID NO:22), HWKCIRYECVLLHKAK (position 32 to 47 of SEQ ID NO:23), HWKCIRYECVLLHKAK (position 32 to 47 of SEQ ID NO:24), HWKCLRYESVLLHKAR (position 35 to 50 of SEQ ID NO:25), HWKCMRHESVLLYKAK (position 32 to 47 of SEQ ID NO:26), HWKCMRHESVLLYKAK (position 32 to 47 of SEQ ID NO:27), HWKCIRLESVLLHKAK (position 32 to 47 of SEQ ID NO:28), HWKCLRIEAALLFKAR (position 32 to 47 of SEQ ID NO:29), HWKCLRMEAVVLYKAR (position 32 to 47 of SEQ ID NO:30), HWKCIRLECALQYKAR (position 32 to 47 of SEQ ID NO:31), HWKCIRLECALQYKAR (position 32 to 47 of SEQ ID NO:32), HWKHVRHENVLLHKAR (position 32 to 47 of SEQ ID NO:33), HWKHVRLENVLLHKAR (position 32 to 47 of SEQ ID NO:34), HWQLLRQEQIIYHYAR (position 32 to 47 of SEQ ID NO:35), HWKLLRQEQILLYYAR (position 32 to 47 of SEQ ID NO:36), HWKLLRQEQVLFYYAR (position 32 to 47 of SEQ ID NO:37), HWQLLRQEQILFHYAR (position 32 to 47 of SEQ ID NO:38), HWQLLRQEQVLFYYAR (position 32 to 47 of SEQ ID NO:39), HWQTLRQEQILLHYAR (position 32 to 47 of SEQ ID NO:40), HWKLLRQEQALLFFAR (position 32 to 47 of SEQ ID NO:41), HWQTLRKEAVLLYYAR (position 32 to 47 of SEQ ID NO:42), HWQTLRKEPVLLYYAR (position 32 to 47 of SEQ ID NO:43), HWQTLRQEAVLLYFAR (position 32 to 47 of SEQ ID NO:44), HWQTLRKEAVTLYFAR (position 32 to 47 of SEQ ID NO:45), HWTLLRREAVLLYYAR (position 32 to 47 of SEQ ID NO:46), HWLLLRKEAVLLYFAR (position 32 to 47 of SEQ ID NO:47), HWQTLRKEAVLLYFAR (position 32 to 47 of SEQ ID NO:48), HWQTLRKEAVLLYFAR (position 32 to 47 of SEQ ID NO:49), HWQTLRKEAVLLYFAR (position 32 to 47 of SEQ ID NO:50), HWQILRREAVLLYFAR (position 32 to 47 of SEQ ID NO:51), HWQILRKEAVLLYFAR (position 32 to 47 of SEQ ID NO:52), HWRLLRKEAVLLYFAR (position 32 to 47 of SEQ ID NO:53), HWQALRREAVLLYYAR (position 32 to 47 of SEQ ID NO:54), QWNLLRQEQVLFHYAR (position 32 to 47 of SEQ ID NO:55), QWNLIRQEQVLFHFAR (position 32 to 47 of SEQ ID NO:56), YWDLNRKLYVTYYYAR (position 29 to 44 of SEQ ID NO:57), YWENVRKENAIMHYAR (position 32 to 47 of SEQ ID NO:58), YWENIRKENAIMHFAR (position 32 to 47 of SEQ ID NO:59), YWENIRKENAIMHYAR (position 32 to 47 of SEQ ID NO:60), YWESMRKEQVLAFYAK (position 31 to 46 of SEQ ID NO:61), YWKAVRLENVIAYYAR (position 31 to 46 of SEQ ID NO:62), and LWNLLRRENAIWYVLR (position 32 to 47 of SEQ ID NO:63).

Said sequences correspond to amino acids 36 to 51 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFFAAR (position 31 to 46 of SEQ ID NO:13), YWKHMRLECAIYYKAR (position 32 to 47 of SEQ ID NO:2), HWKCIRLESVLLHKAK (position 32 to 47 of SEQ ID NO:28), and HWKCMRHESVLLYKAK (position 32 to 47 of SEQ ID NO:26).

According to another preferred embodiment, the polypeptide of the invention comprises the sequence X₁WX₂X₃X₄RX₅X₆X₇X₈X₉X₁₀X₁₁X₁₂X₁₃X₁₄X₁₅ (SEQ ID NO:69), and derivatives thereof, wherein:
- X₁, X₂, X₃, X₄, X₅, X₆, X₇, X₈, X₉, X₁₀, X₁₁, X_{12,} X₁₃, and X₁₄ are as described previously.
- X₁₅ is an amino acid (i.e., an α amino acid), preferably X₁₅ is a polar amino acid selected in the group consisting in lysine (K), arginine (R), glutamine (Q), and glutamic acid (E), and most preferably, X₁₅ is a glutamine (Q), or glutamic acid (E).

Preferably, the polypeptide of the invention comprises an amino acid sequence which is selected in the group comprising YWKHMRLECAIYYKARE (position 32 to 48 of SEQ ID NO:2), YWKHIRLECVLMYKARE (position 32 to 48 of SEQ ID NO:3), YWKLIRLECAVFYKARE (position 32 to 48 of SEQ ID NO:4), HWKLTRMECVLFYKAKE (position 32 to 48 of SEQ ID NO:5), HWKLIRMECAIMYTARQ (position 32 to 48 of SEQ ID NO:6), HWKLIRMECALLYTAKQ (position 32 to 48 of SEQ ID NO:7), HWRLRRIECALYYKAKE (position 32 to 48 of SEQ ID NO:8), YWNCVRLENAIYYAARE (position 32 to 48 of SEQ ID NO:9), YWKYVRLENAIFYAARE (position 32 to 48 of SEQ ID NO:10), YWKCVRMENAIFYAARE (position 32 to 48 of SEQ ID NO:11), YWKLVRMENVILFAARE (position 32 to 48 of SEQ ID NO:12), YWQLIRWENAIFFAARE (position 31 to 47 of SEQ ID NO:13), YWQLIRLENAILFTARE (position 31 to 47 of SEQ ID NO:14), YWKLVRYECAIFYKARE (position 32 to 48 of SEQ ID NO:15), YWKLVRYECAIFYKARE (position 32 to 48 of SEQ ID NO:16), YWTLVRYECAMFYTARE (position 32 to 48 of SEQ ID NO:17), YWTLLRYEAAMFYAARE (position 32 to 48 of SEQ ID NO:18), YWKAVRHEYVLYYKARE (position 32 to 48 of SEQ ID NO:19), YWKAVRQENVIYYKARE (position 32 to 48 of SEQ ID NO:20), YWKAVRHENVVLYKARQ (position 32 to 48 of SEQ ID NO:21), HWHYVRLENAMLFKARQ (position 32 to 48 of SEQ ID NO:22), HWKCIRYECVLLHKAKQ (position 32 to 48 of SEQ ID NO:23), HWKCIRYECVLLHKAKQ (position 32 to 48 of SEQ ID NO:24), HWKCLRYESVLLHKARQ (position 35 to 51 of SEQ ID NO:25), HWKCMRHESVLLYKAKQ (position 32 to 48 of SEQ ID NO:26), HWKCMRHESVLLYKAKQ (position 32 to 48 of SEQ ID NO:27), HWKCIRLESVLLHKAKQ (position 32 to 48 of SEQ ID NO:28), HWKCLRIEAALLFKARE (position 32 to 48 of SEQ ID NO:29), HWKCLRMEAVVLYKARE (position 32 to 48 of SEQ ID NO:30), HWKCIRLECALQYKARE (position 32 to 48 of SEQ ID NO:31), HWKCIRLECALQYKARE (position 32 to 48 of SEQ ID NO:32), HWKHVRHENVLLHKARE (position 32 to 48 of SEQ ID NO:33), HWKHVRLENVLLHKARE (position 32 to 48 of SEQ ID NO:34), HWQLLRQEQIIYHYARR (position 32 to 48 of SEQ ID NO:35), HWKLLRQEQILLYYARK (position 32 to 48 of SEQ ID NO:36), HWKLLRQEQVLFYYARR (position 32 to 48 of SEQ ID NO:37), HWQLLRQEQILFHYARK (position 32 to 48 of SEQ ID NO:38), HWQLLRQEQVLFYYARK (position 32 to 48 of SEQ ID NO:39), HWQTLRQEQILLHYARK (position 32 to 48 of SEQ ID NO:40), HWKLLRQEQALLFFARK (position 32 to 48 of SEQ ID NO:41), HWQTLRKEAVLLYYARE (position 32 to 48 of SEQ ID NO:42), HWQTLRKEPVLLYYARE (position 32 to 48 of SEQ ID NO:43), HWQTLRQEAVLLYFARQ (position 32 to 48 of SEQ ID NO:44), HWQTLRKEAVTLYFARQ (position 32 to 48 of SEQ ID NO:45), HWTLLRREAVLLYYARQ (position 32 to 48 of SEQ ID NO:46), HWLLLRKEAVLLYFARQ (position 32 to 48 of SEQ ID NO:47), HWQTLRKEAVLLYFARQ (position 32 to 48 of SEQ ID NO:48), HWQTLRKEAVLLYFARQ (position 32 to 48 of SEQ ID NO:49), HWQTLRKEAVLLYFARQ (position 32 to 48 of SEQ ID NO:50), HWQILRREAVLLYFARQ (position 32 to 48 of SEQ ID NO:51), HWQILRKEAVLLYFARR (position 32 to 48 of SEQ ID NO:52), HWRLLRKEAVLLYFARQ (position 32 to 48 of SEQ ID NO:53), HWQALRREAVLLYYARQ (position 32 to 48 of SEQ ID NO:54), QWNLLRQEQVLFHYARK (position 32 to 48 of SEQ ID NO:55), QWNLIRQEQVLFHFARK (position 32 to 48 of SEQ ID NO:56), YWDLNRKLYVTYYYARK (position 29 to 45 of SEQ ID NO:57), YWENVRKENAIMHYARK (position 32 to 48 of SEQ ID NO:58), YWENIRKENAIMHFARK (position 32 to 48 of SEQ ID NO:59), YWENIRKENAIMHYARK (position 32 to 48 of SEQ ID NO:60), YWESMRKEQVLAFYAKK (position 31 to 47 of SEQ ID NO:61), YWKAVRLENVIAYYARK (position 31 to 47 of SEQ ID NO:62), and LWNLLRRENAIWYVLRQ (position 32 to 48 of SEQ ID NO:63).

Said sequences correspond to amino acids 36 to 52 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFFAARE (position 31 to 47 of SEQ ID NO:13), YWKHMRLECAIYYKARE (position 32 to 48 of SEQ ID NO:2), HWKCIRLESVLLHKAKQ (position 32 to 48 of SEQ ID NO:28), and HWKCMRHESVLLYKAKQ (position 32 to 48 of SEQ ID NO:26).

According to another preferred embodiment, the polypeptide of the invention comprises the sequence X₁WX₂X₃X₄RX₅X₆X₇X₈X₉X₁₀X₁₁X₁₂X₁₃X₁₄X₁₅X₁₆ (SEQ ID NO:70), and derivatives thereof, wherein:
- X₁, X₂, X₃, X₄, X₅, X₆, X₇, X₈, X₉, X₁₀, X₁₁, X₁₂, X_{13,} X₁₄, and X₁₅ are as described previously.
- X₁₆ is an amino acid (i.e., an α amino acid), preferably X₁₅ is selected in the group consisting in lysine (K), arginine (R), glutamine (Q), glutamic acid (E), methionine (M), leucine (L), glycine (G), , histidine (H), alanine (A), and asparagines (N).

Preferably, the polypeptide of the invention comprises an amino acid sequence which is selected in the group comprising YWKHMRLECAIYYKAREM (position 32 to 49 of SEQ ID NO:2), YWKHIRLECVLMYKAREM (position 32 to 49 of SEQ ID NO:3), YWKLIRLECAVFYKAREM (position 32 to 49 of SEQ ID NO:4), HWKLTRMECVLFYKAKEL (position 32 to 49 of SEQ ID NO:5), HWKLIRMECAIMYTARQM (position 32 to 49 of SEQ ID NO:6), HWKLIRMECALLYTAKQM (position 32 to 49 of SEQ ID NO:7), HWRLRRIECALYYKAKEL (position 32 to 49 of SEQ ID NO:8), YWNCVRLENAIYYAARER (position 32 to 49 of SEQ ID NO:9), YWKYVRLENAIFYAARER (position 32 to 49 of SEQ ID NO:10), YWKCVRMENAIFYAARER (position 32 to 49 of SEQ ID NO:11), YWKLVRMENVILFAAREN (position 32 to 49 of SEQ ID NO:12), YWQLIRWENAIFFAAREH (position 31 to 48 of SEQ ID NO:13), YWQLIRLENAILFTAREH (position 31 to 48 of SEQ ID NO:14), YWKLVRYECAIFYKAREG (position 32 to 49 of SEQ ID NO:15), YWKLVRYECAIFYKAREG (position 32 to 49 of SEQ ID NO:16), YWTLVRYECAMFYTARER (position 32 to 49 of SEQ ID NO:17), YWTLLRYEAAMFYAARER (position 32 to 49 of SEQ ID NO:18), YWKAVRHEYVLYYKAREN (position 32 to 49 of SEQ ID NO:19), YWKAVRQENVIYYKAREN (position 32 to 49 of SEQ ID NO:20), YWKAVRHENVVLYKARQN (position 32 to 49 of SEQ ID NO:21), HWHYVRLENAMLFKARQA (position 32 to 49 of SEQ ID NO:22), HWKCIRYECVLLHKAKQM (position 32 to 49 of SEQ ID NO:23), HWKCIRYECVLLHKAKQM (position 32 to 49 of SEQ ID NO:24), HWKCLRYESVLLHKARQM (position 35 to 52 of SEQ ID NO:25), HWKCMRHESVLLYKAKQM (position 32 to 49 of SEQ ID NO:26), HWKCMRHESVLLYKAKQM (position 32 to 49 of SEQ ID NO:27), HWKCIRLESVLLHKAKQM (position 32 to 49 of SEQ ID NO:28), HWKCLRIEAALLFKAREM (position 32 to 49 of SEQ ID NO:29), HWKCLRMEAVVLYKAREM (position 32 to 49 of SEQ ID NO:30), HWKCIRLECALQYKAREL (position 32 to 49 of SEQ ID NO:31), HWKCIRLECALQYKAREM (position 32 to 49 of SEQ ID NO:32), HWKHVRHENVLLHKAREM (position 32 to 49 of SEQ ID NO:33), HWKHVRLENVLLHKAREM (position 32 to 49 of SEQ ID NO:34), HWQLLRQEQIIYHYARRH (position 32 to 49 of SEQ ID NO:35), HWKLLRQEQILLYYARKR (position 32 to 49 of SEQ ID NO:36), HWKLLRQEQVLFYYARRH (position 32 to 49 of SEQ ID NO:37), HWQLLRQEQILFHYARKN (position 32 to 49 of SEQ ID NO:38), HWQLLRQEQVLFYYARKN (position 32 to 49 of SEQ ID NO:39), HWQTLRQEQILLHYARKN (position 32 to 49 of SEQ ID NO:40), HWKLLRQEQALLFFARKH (position 32 to 49 of SEQ ID NO:41), HWQTLRKEAVLLYYAREK (position 32 to 49 of SEQ ID NO:42), HWQTLRKEPVLLYYAREK (position 32 to 49 of SEQ ID NO:43), HWQTLRQEAVLLYFARQR (position 32 to 49 of SEQ ID NO:44), HWQTLRKEAVTLYFARQK (position 32 to 49 of SEQ ID NO:45), HWTLLRREAVLLYYARQK (position 32 to 49 of SEQ ID NO:46), HWLLLRKEAVLLYFARQK (position 32 to 49 of SEQ ID NO:47), HWQTLRKEAVLLYFARQK (position 32 to 49 of SEQ ID NO:48), HWQTLRKEAVLLYFARQN (position 32 to 49 of SEQ ID NO:49), HWQTLRKEAVLLYFARQH (position 32 to 49 of SEQ ID NO:50), HWQILRREAVLLYFARQK (position 32 to 49 of SEQ ID NO:51), HWQILRKEAVLLYFARRK (position 32 to 49 of SEQ ID NO:52), HWRLLRKEAVLLYFARQN (position 32 to 49 of SEQ ID NO:53), HWQALRREAVLLYYARQN (position 32 to 49 of SEQ ID NO:54), QWNLLRQEQVLFHYARKK (position 32 to 49 of SEQ ID NO:55), QWNLIRQEQVLFHFARKN (position 32 to 49 of SEQ ID NO:56), YWDLNRKLYVTYYYARKE (position 29 to 46 of SEQ ID NO:57), YWENVRKENAIMHYARKQ (position 32 to 49 of SEQ ID NO:58), YWENIRKENAIMHFARKQ (position 32 to 49 of SEQ ID NO:59), YWENIRKENAIMHYARKQ (position 32 to 49 of SEQ ID NO:60), YWESMRKEQVLAFYAKKE (position 31 to 48 of SEQ ID NO:61), YWKAVRLENVIAYYARKE (position 31 to 48 of SEQ ID NO:62), and LWNLLRRENAIWYVLRQE (position 32 to 49 of SEQ ID NO:63).

Said sequences correspond to amino acids 36 to 53 of the E2 protein from HPV-18 and amino acid sequences at equivalent position in E2 proteins from the different HPV viruses disclosed in figure 7.

More preferably, the polypeptide of the invention comprises an amino acid sequence which is selected in the group comprising YWQLIRWENAIFFAAREH (position 31 to 48 of SEQ ID NO:13), YWKHMRLECAIYYKAREM (position 32 to 49 of SEQ ID NO:2), HWKCIRLESVLLHKAKQM (position 32 to 49 of SEQ ID NO :28), and HWKCMRHESVLLYKAKQM (position 32 to 49 of SEQ ID NO:26).

According to still another preferred embodiment, the polypeptide of the invention also comprises an amino acid sequence coding for a cell penetrating peptide.

The term "cell penetrating peptide" (CPP) is defined as a carrier peptide that is capable of crossing biological membrane or a physiological barrier. Cell penetrating peptides are also called cell-permeable peptides, protein-transduction domains (PTD) or membrane- translocation sequences (MTS). CPPs have the ability to translocate in vitro and/or in vivo the mammalian cell membranes and enter into cells, and directs the apoptosis domain of interest to the cytoplasm.

Several proteins and their peptide derivatives have been found to possess cell internalization properties including but not limited to the Human Immunodef[iota]cency Virus type 1 (HIV-I) protein Tat (RUBEN et al., J. Virol., vol.63, p:1-8, 1989), the herpes virus tegument protein VP22 (ELLIOTT and O'HARE, Cell, vol.88, p:223-233, 1997)), the homeotic protein of Drosophila melanogaster Antennapedia (the CPP is called Penetratin) (DEROSSI et al., J. Biol. Chem., vol.271, p:18188-18193, 1996), the protegrin 1 (PG-I) anti-microbial peptide SynB (KOKRYAKOV et al, FEBS Lett., vol.327, p:231-236, 1993) and the basic fibroblast growth factor (JANS, Faseb J., vol.8, p:841-847, 1994). A number of other proteins and their peptide derivatives have been found to possess similar cell internalization properties such as the peptides disclosed in patent applications WO 01/64738 and EP1512696. As general references on CPPs it can be cited: CELL PENETRATING PEPTIDES: PROCESSES AND APPLICATIONS, edited by UIO LANGEL (2002); or Advanced Drug Delivery Reviews (vol.57, p:489-660, 2005); or DIETZ and BAHR (Moll. Cell. Neurosci., vol.27, p:85-131, 2004).

In a second aspect the present invention relates to a nucleic acid encoding for a polypeptide as described above.

Said nucleic acid corresponds to RNA or DNA, preferably to DNA.

Preferably, said nucleic acid corresponds to the insert of the expression plasmid contained in the bacteria CNCM-3743 or CNCM-3744.

According to a preferred embodiment, the nucleic acid encoding the polypeptide of the invention is operatively linked to a gene expression sequence, which directs the expression of nucleic acid within a prokarotic or an eukaryotic cell, preferably an eukaryotic cell. The "gene expression sequence" is any regulatory nucleotide sequence, such as a promoter sequence or promoter-enhancer combination, which facilitates the efficient transcription and translation of the peptidic antagonist nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, beta.-actin promoter, muscle creatine kinase promoter, human elongation factor promoter and other constitutive promoters. Exemplary viral promoters which function constitutively in eukaryotic cells include, for example, promoters from the simian virus (e.g., SV40), papilloma virus, adenovirus, human immunodeficiency virus (HIV), cytomegalovirus (CMV), Rous sarcoma virus (RSV), hepatitis B virus (HBV), the long terminal repeats (LTR) of Moloney leukemia virus and other retroviruses, and the thymidine kinase promoter of herpes simplex virus. Others constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Others inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired.

As used herein, the nucleic acid sequence encoding the polypeptide of the invention and the gene expression sequence are said to be "operably linked" when they are covalently linked in such a way as to place the expression or transcription and/or translation of the polypeptide of the invention coding sequence under the influence or control of the gene expression sequence. Two DNA sequences are said to be operably linked if induction of a promoter in the 5' gene expression sequence results in the transcription of the polypeptide of the invention and if the nature of the linkage between the two DNA sequences does not (1) result in the introduction of a frame-shift mutation, (2) interfere with the ability of the promoter region to direct the transcription of the polypeptide of the invention, or (3) interfere with the ability of the corresponding RNA transcript to be translated into a protein. Thus, a gene expression sequence would be operably linked to a nucleic acid sequence coding for the polypeptide of the invention if the gene expression sequence were capable of effecting transcription of that antigen nucleic acid sequence such that the resulting transcript is translated into the desired polypeptide.

The nucleic acid coding for the polypeptide of the invention may be delivered *in vivo* alone or in association with a vector. In its broadest sense, a "vector" is any vehicle capable of facilitating the transfer of the nucleic acid coding for the polypeptide of the invention to the cells. Preferably, the vector transports the nucleic acid to cells with reduced degradation relative to the extent of degradation that would result in the absence of the vector. In general, the vectors useful in the invention include, but are not limited to, plasmids, phagmids, viruses, other vehicles derived from viral or bacterial sources that have been manipulated by the insertion or incorporation of the peptidic antagonist nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in KRIEGLER (A Laboratory Manual," W.H. Freeman C.O., New York, 1990) and in MURRY ("Methods in Molecular Biology," vol.7, Humana Press, Inc., Cliffton, N.J., 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g., SANBROOK et al., "Molecular Cloning: A Laboratory Manual," Second Edition, Cold Spring Harbor Laboratory Press, 1989. In the last few years, plasmid vectors have been used as DNA vaccines for delivering antigen-encoding genes to cells *in vivo.* They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

The nucleic acid vector can include selectable markers that are active both in bacteria and in mammalian cells.

According to a first specific embodiment, the nucleic acid vector of the present invention corresponds to "naked DNA" like plasmids, cosmids or phagemids. Such naked DNA can be associated with non-lipid cationic polymers (WU and WU, J. Biol. Chem., vol.263, p: 14621-4, 1988) or liposomes (BRIGHMAN et al., Am. J. Med. Sci., vol.298, p: 278-81, 1989) to form complexes enhancing cellular uptake.

Preferably, said nucleic acid vector is a plasmid vector, and corresponds to one of the vectors contained in bacteria CNCM-3743 or CNCM-3744.

According to a second specific embodiment, the nucleic acid vector is a viral vector adapted for *in vivo* gene therapy protocols. Examples of appropriate viral vectors includes retroviral vectors as described in EP 0871459, EP 0386882 and EP 1222300 and adenovirus vectors as described in US 2004/ 265273 and US 6,638,502. In this case, the internalization of virus occurs through the specific interaction of the viral envelope with a cell surface receptor, followed by receptor-mediated endocytosis of the virus/receptor complex.

In a third aspect, the present invention relates to a host cell genetically engineered with the polynucleotide or with the vector described previously

As used herein, the term "host cell genetically engineered" relates to host cells which have been transduced, transformed or transfected with the polynucleotide or with the vector described previously.

As representative examples of appropriate host cells, one can cites bacterial cells, such as E. coli, Streptomyces, Salmonella typhimurium, fungal cells such as yeast, insect cells such as Sf9, animal cells such as CHO or COS, plant cells, etc. The selection of an appropriate host is deemed to be within the scope of those skilled in the art from the teachings herein.

Preferably, said host cell is a bacterial cell, most preferably a bacterial cell corresponding to the bacteria CNCM-3743 or CNCM-3744.

The introduction of the polynucleotide or of the vector described previously into the host cell can be effected by method well known from one of skill in the art such as calcium phosphate transfection, DEAE-Dextran mediated transfection, or electroporation.

In a forth aspect the present invention, the present invention relates to an antibody directed against a polypeptide as described previously.

Polyclonal antibodies can be prepared by immunizing a suitable animal, such as mouse, rabbit or goat, with a polypeptide as described previously. The antibody titer in the immunized animal can be monitored over time by standard techniques, such as with an enzyme linked immunosorbent assay (ELISA) using immobilized polypeptide. At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed. , John Wiley & Sons, New York, 1994). Hybridoma cells producing the desired monoclonal antibody are detected by screening the hybridoma culture supernatants for antibodies that bind the polypeptide of interest, e.g., using a standard ELISA assay.

In a fifth embodiment, the present invention relates to a composition comprising a polypeptide as described above, a nucleic acid encoding thereof, or a nucleic acid vector comprising said nucleic acid, eventually associated with a pharmaceutically acceptable vehicle.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

The polypeptides of the invention, nucleic acids coding therefore or nucleic acid vectors may be solubilized in a buffer or water or incorporated in emulsions and microemulsions. Suitable buffers include, but are not limited to, phosphate buffered saline Ca⁺⁺/Mg⁺⁺ free (PBS), phosphate buffered saline (PBS), normal saline (150 mM NaCl in water), Tris buffer and surfactants.

There are numerous causes of peptide instability or degradation, including hydrolysis and denaturation. Hydrophobic interaction may cause clumping of molecules together (i.e. aggregation). This result may entail diminution of the induction of a Treg response. Stabilizers may be added to lessen or prevent such problems.

Stabilizers include cyclodextrine and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide, and are water-miscible or water-soluble. Suitable polyols may be polyhydroxy alcohols, monosaccharides and disaccharides including mannitol, glycrol, ethylene glycol, propylene glycol, trimethyl glycol, vinyl pyrrolidone, glucose, fructose, arabinose, mannose, maltose, sucrose, and polymers thereof Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

In a sixth aspect, the present invention relates to the use of a polypeptide as describe above, a nucleic acid sequence coding therefore, a vector comprising such a nucleic acid sequence, or a composition as described previously for treating cancer in a subject.

In fact, the apoptosis activity of the polypeptide of the invention enables to induce the death of the cells wherein they are internalized.

As used herein, the term "subject" denotes a Mammal, such as a rodent, a feline, a canine and a primate. The subject is an animal such as cow, pig, horse, chicken, cat, dog and most preferably a human.

In a seventh aspect, the present invention relates to a method for treating cancer comprising administrating to a subject a therapeutically effective amount of a polypeptide as describe above, a nucleic acid sequence coding therefore, a vector comprising such a nucleic acid sequence, or a composition as described previously.

According to the present invention, an "effective amount" of a composition is one which is sufficient to achieve a desired biological effect, in this case inducing apoptosis in tumor cells. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the preferred dosage can be tailored to the individual subject, as is understood and determinable by one of skill in the art, without undue experimentation.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### Examples

### 1) Cell death induced by E2 is associated to caspase 8 activation

The role of caspase 8 as the initiator caspase of E2-induced apoptosis was investigated using specific inhibitors of the apical caspase 8; i.e. the MC159 vFLIP protein (viral FLICE Inhibitory protein) from *Molluscum Contagiosum* and the Cowpox virus serpin crmA.

293 cells were transfected by standard calcium phosphate co-precipitation technique into 6 well-plates with typically 1 to 2 µg plasmid coding for GFP or for GFP-E2 fusion protein as described in BELLANGER et al. (J. Virol., vol.75(16), p:7244-7251, 2001), wherein said E2 protein corresponds to HPV18 E2 protein, with or without 1 µg of plasmid coding for HA-MC 159 provided by Richard SIEGEL (National Institute of Allergy and Infectious Diseases, National Institutes of Health, Bethesda) or coding for crmA (provided by Visha DIXIT). 48 hours after transfection, cells were trypsinized, washed, resuspended in PBS-10% serum containing 25 µg/ml propidium iodide (PI)(SIGMA), and analyzed by flow cytometry for GFP fluorescence and PI content. Finally, the GFP-positive dead cells were estimated as the PI positive cells /GFP positive cells.

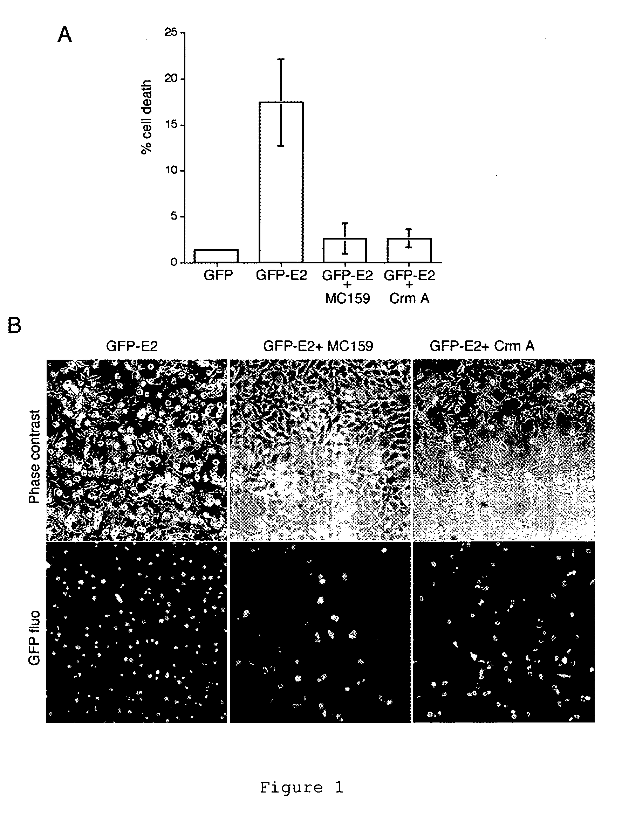

The figure 1 A) shows the dead cells percentage for 293 cells transfected with plasmids expressing GFP (GFP), GFP-E2 fusion protein (GFP-E2), with or without the caspase-8 inhibitors MC 159 (MC159) or crmA (crm A).

The figure 1 B) shows the phase contrast and GFP fluorescence of 293 cells 48 hours after transfection with plasmids expressing GFP-E2 fusion protein (GFP-E2), with or withour the caspase-8 inhibitors MC 159 (MC159) or crmA (crm A).

The results confirm the pro-apoptotic activity of the protein E2 (fusion protein GFP-E2) of oncogenic HPV, notably HPV16 and 18, which has been shown to be linked to the presence of E2 in the cytoplasm and active nucleo-cytoplasmic shuttling (BLACHON et al., J. Biol. Chem., vol.280, p: 36088-36098, 2005). The results also show that the caspase-8 inhibitors MC 159 (MC159) or crmA (crm A) efficiently abrogated E2-induced cell death when co-expressed with GFP-E2 (Figure 1), firmly establishing that caspase 8 activation is indeed the initiating event of the apoptotic pathway triggered by the HPV 18 E2 protein.

### 2) Cell death follows E2 assembly into filaments which recruit and activate caspase 8

Saos cells were infected at a multiplicity of infection of 500 particles/cell with recombinant adenoviruses expressing either the fusion protein HPV18 GFP-E2 (Ad GFP-E2) or a fusion protein GFP-E2ΔNT with a non apoptotic form of HPV18 E2 (Ad GFP-E2ΔNT) as described in DEMERET *et al.* (abovementioned, 2003). A video microscopy was performed on recombinant adenovirus-infected Saos-2 cells from 7 to 25 hours post infection with an AXIOVERT ZEISS microscope, and selected images were processed by the METAMORPH software.

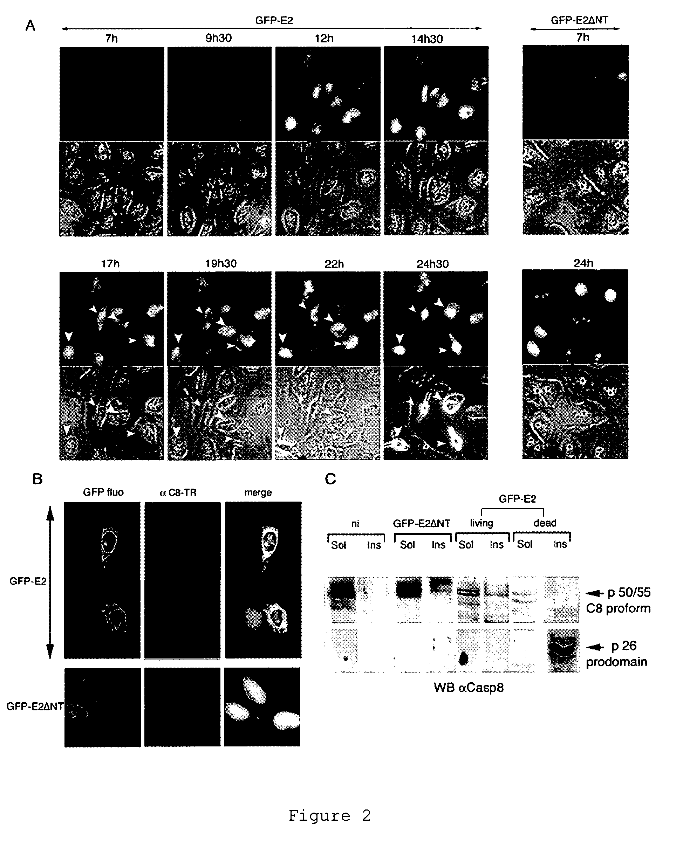

The figure 2 A) shows the fluorescent and phase contrast images of the same field, selected at different time points for Saos cells infected with either Ad GFP-E2 or Ad GFP-E2ΔNT adenovirus. White arrows show dying cells.

The results show that after a lag time of about 12 hours of nuclear accumulation, GFP-E2 progressively redistributed to the cytoplasm where it formed perinuclear filamentous structures (Figure 2A). Then, apoptosis occurred specifically in the cells containing these filaments at variable times after their formation. Under similar conditions, the cells expressing GFP fused with a non-apoptotic mutant form of HPV18 E2 (GFP-E2ΔNT) did not show cell death, despite high levels of nuclear accumulation (Figure 2A). Finally, the results established that E2-induced cell death is preceded by assembly of GFP-E2 into cytoplamic filaments.

Saos cells were infected with Ad GFP-E2 or Ad GFP-E2ΔNT as described previously, and then cultured on cover slides. Cells grown on cover slides were rinsed in PBS, fixed in 2% paraformaldehyde, permeabilized with 0.1 % Triton, saturated in PBS-10% SVF, and incubated for 1 hr with rabbit anti-caspase 8 (PHARMINGEN) according to manufacturer's protocol. Finally, the slides are incubated with anti-rabbit antibody Texas-Red (TR) conjugated (AMERSHAM) and then nuclei were stained with DAPI (SIGMA), at 0.15 µg/ml. Pictures were taken with a ZEISS AXIOPHOT microscope coupled to a CCD camera 20 hours post infection for GFP and Texas-red fluorescence.

Simultaneously, some Ad GFP-E2 or Ad GFP-E2ΔNT infected cells were extracted 30 hours post-infection in 1% TRITON P300 buffer (20 mM NaH₂PO₄, 300 mM NaCl, 5 mM EDTA, 5 mM DTT) supplemented with protease inhibitors (ROCHE DIAGNOSTICS) for 30 min at 4°C followed by centrifugation for 30 min at 13,000 rpm. Dead (floating) and living (attached) GFP-E2 expressing cells were processed separately. Pellets containing TRITON-insoluble material were denatured in 5X LAEMMLI sample buffer and boiled for 20 min. Equivalent fractions of soluble and insoluble material in 1X sample buffer were separated on SDS-PAGE and processed for immunoblotting with rabbit anti-caspase 8 antibody (PHARMINGEN) according to manufacturer's instructions.

The figure 2 B) shows the GFP fluorescence (GFP fluo), Texas-Red fluorescence (αC8-TR) and both fluorescence (Merge) for Saos-2 cells infected with Ad GFP-E2 (GFP-E2) or Ad GFP-E2ΔNT (GFP-E2ΔNT).

The figure 2 C) shows the immunoblots performed with anti-caspase 8 antibody on equivalent soluble (Sol) and insoluble (Ins) fractions of Saos-2 cells non infected (ni), infected with Ad GFP-E2ΔNT (GFP-E2ΔNT) or of dead or living Saos-2 cells infected with Ad GFP-E2 (GFP-E2). The blacks arrows indicate the positions of the p50/55 caspase precursor (proC8) and of the 26 Kd DED (Death Effector Domain) containing pro-domain.

The immunofluorescence analysis indicated that the GFP-E2 filaments contained endogenous caspase 8 that co-localized with E2 (Figure 2B). Formation of GFP-E2/caspase 8 cytoplasmic filaments correlated with translocation of both proteins to a detergent-insoluble fraction, containing processed caspase 8 (Figure 2C). Thus, E2-expressing dead cells contained only cleaved caspase 8, with the 26 Kd pro-domain present in a detergent-insoluble fraction (Figure 2C), suggesting that caspase 8 self catalytic processing had occurred within filaments. In contrast, in all living cells expressing GFP-E2ΔNT or GFP-E2, only the inactive pro-form of caspase 8 was detected in soluble fractions (Figure 2C). This suggests that self-catalytic processing of caspase 8 had occurred within filaments, reflecting its enzymatic activation. Indeed, the analysis of caspase 8 activity has established that such enzymatic activity was readily detectable in extracts of GFP-E2 expressing cells but not in non infected cells or in cells expressing the non apoptotic form of E2, providing a direct evidence of caspase 8 activation (data not shown).

In order to determinate if the filament formation results from caspase activation, Saos cells were infected with Ad GFP-E2 recombinant adenovirus as described previously. Then the infected cells were treated with 40 µM Z-VAD-fmk just after infection, which abrogates cell death (data not shown and DEMERET *et al.,* abovementioned, 2003); and processed for anti-caspase 8 immunofluorescence 24 hours post infection.

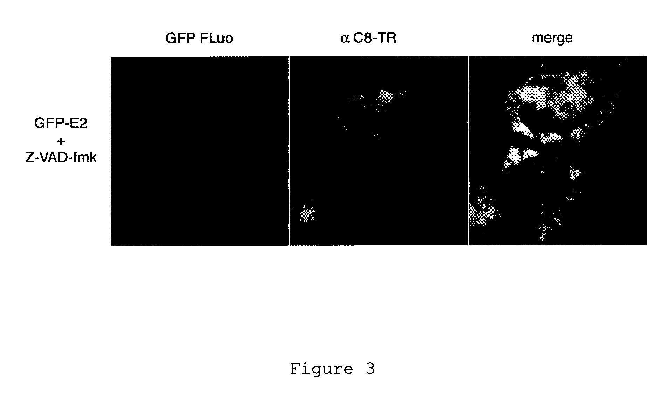

The figure 3 shows the GFP fluorescence (GFP fluo), texas-Red fluorescence (αC8-TR) and both fluorescence (Merge) for Saos-2 cells infected with Ad GFP-E and tretad with Z-VAD-fmk.

The results establish that E2/caspase 8 filaments can assemble in the presence of the caspase inhibitor Z-VAD-fmk, indicating that filament formation is independent of caspase activity and is an upstream event of E2-induced apoptosis (Figure 3).

Overall, these experiments show that the HPV18 E2 protein induces apoptosis through assembly into filaments that efficiently recruit and activate endogenous caspase 8.

### 3) The pro-apoptotic E2 protein induces caspase 8 oligomerization within filaments through direct interaction

Oligomerization of apical caspase 8 has been shown to be strictly dependent upon its homotypic binding to oligomerization-competent DFD (Death Fold Domain) containing adaptors, such as FADD which recruits caspase 8 to the Death Inducing Signaling Complex (DISC) through DED-mediated interactions. Actually, only few other caspase 8 adaptors have been described, among which the HIP-1/Hippi proteins which activate caspase 8 through pseudo-DEDs when expanded-poly Q Hungtintin is expressed in the cells (GERVAIS et al., Nat. Cell Biol., vol.4, p: 95-105, 2002). Nevertheless, analysis of the E2 protein crystal structure does not reveal any obvious DFD (ANTSON et al., Nature, vol.403, p: 805-809, 2000), and therefore E2 appears not to act as a typical DFD adaptor for caspase 8 oligomerization.

In order to better understand the interaction between E2 protein and caspase 8, 293 cells were transfected as previously described with typically 1 to 1.5 µg plasmid coding for GFP, GFP-E2 fusion protein or GFP-E2ΔNT, with or without 0.5 µg of pcDNA plasmid coding for caspase 8, and then cultured on cover slides. 24 hours after transfection, cells grown on cover slides were rinsed in PBS, fixed in 2% paraformaldehyde, permeabilized with 0.1 % Triton, saturated in PBS-10% SVF, and incubated for 1 hr with rabbit anti-caspase 8 (PHARMINGEN) according to manufacturer's protocol. Finally, the slides are incubated with anti-rabbit antibody Texas-Red (TR) conjugated (AMERSHAM) and then nuclei were stained with DAPI (SIGMA), at 0.15 µg/ml. Pictures were taken with a ZEISS AXIOPHOT microscope coupled to a CCD camera 24 hours post transfection for GFP and Texas-red fluorescence.

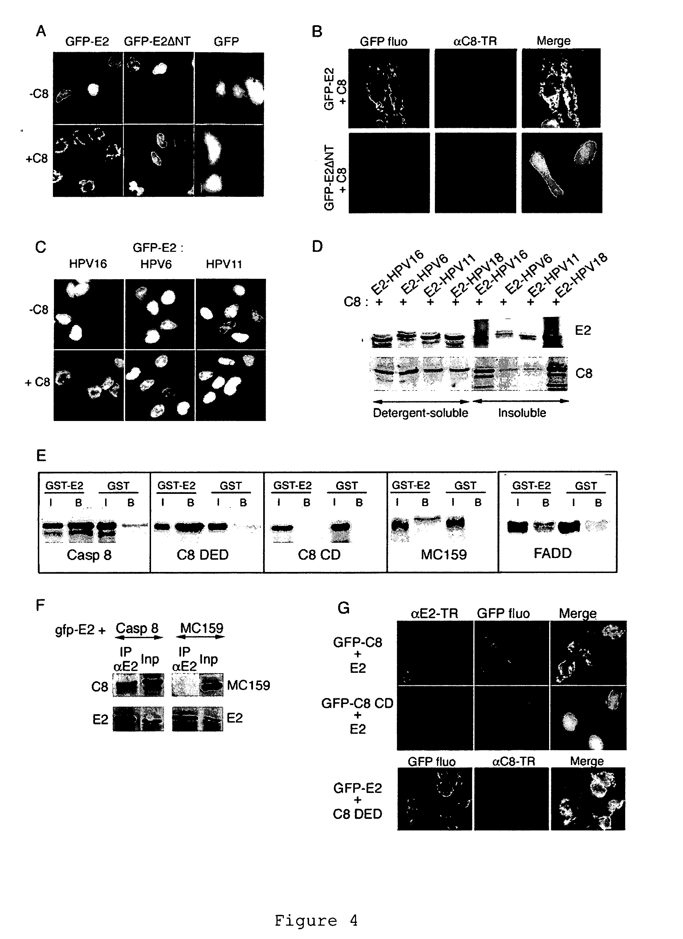

The figure 4 A) shows the GFP fluorescence of 293 living cells 24 hours after transfection with expression plasmids for GFP fusion proteins as indicated, in the presence or absence of a plasmid expressing caspase 8 (C8).

The figure 4 B) shows the GFP (GFP fluo), Texas Red (αC8-TR) and combined (Merge) fluorescence of 293 cells co-transfected with caspase 8 (C8) and GFP-E2 or GFP-E2ΔNT as indicated.

The results established that co-expression of caspase 8 with GFP-E2 in 293 cells induced a massive redistribution of the GFP-E2 into cytoplasmic filaments, under conditions where GFP-E2 alone was predominantly nuclear, indicating that ectopic expression E2 and caspase 8 is sufficient to induce filament formation (Figure 4A). Immunofluorescence analysis with anti-caspase 8 antibodies showed that the GFP-E2 filaments contained transfected caspase 8, with full co-localization of both proteins in the same structures (Figure 4B). Consequently, these results indicate that increased levels of caspase 8 improved GFP-E2/caspase filament assembly, and suggest that caspase 8 plays an active role in filament formation. In contrast, caspase 8 expression did not affect the distribution of GFP-E2ΔNT or GFP alone (Figure 4A).

Since many HPV do not induce apoptosis, the interaction of caspase-8 from E2 proteins of HPV species inducing or not apoptosis has been investigated. For this purpose, transfection experiments on 293 cells with or without caspase-8 expression have been done as described previously with different GFP-E2 fusion proteins, wherein E2 polypeptides correspond either to HPV species inducing (i.e., HPV16 and HPV18) or not (i.e., HPV6 and HPV11) apoptosis.

One part of the transfected cells were analysed 24 hours after transfection for GFP fluorescence as described previously.

Simultaneously, the other part of transfected cells were analysed by immunoblot. These cells were extracted 24 hours post transfection in 1% TRITON P300 buffer (20 mM NaH₂PO₄, 300 mM NaCl, 5 mM EDTA, 5 mM DTT) supplemented with protease inhibitors (ROCHE DIAGNOSTICS) for 30 min at 4°C followed by centrifugation for 30 min at 13,000 rpm. Pellets containing TRITON-insoluble material were denatured in 5X LAEMMLI sample buffer and boiled for 20 min. Equivalent fractions of soluble and insoluble material in 1X sample buffer were separated on SDS-PAGE and processed for immunoblotting with rabbit anti-caspase 8 (PHARMINGEN) and rabbit anti-GFP (TORREY PINES) according to manufacturers' instructions.

The figure 4 C) shows the GFP fluorescence of 293 living cells with expression plasmids for GFP fusion proteins as indicated, in the presence or absence of a plasmid expressing caspase 8.

The figure 4 D) shows the immunoblots with anti-GFP or anti-caspase 8 antibodies realized on equivalent fractions of detergent-soluble and insoluble extracts of cells co-expressing caspase 8 and the different GFP-E2 fusion proteins as indicated.

The results established that the non-apoptotic E2 proteins from the low-risk HPV types 6 and 11 (BLACHON *et al.,* abovementioned, 2005) did not form filaments when co-expressed with caspase 8, whereas the pro-apoptotic E2 protein from oncogenic HPV16 did induce filament formation (Figure 4C). Moreover, the assembly of GFP-E2 from HPV18 or HPV16 and caspase 8 into filaments correlated with translocation of both proteins into a detergent-insoluble fraction and caspase self-processing (Figure 4D). In the presence of the non-apoptotic E2 proteins from HPV 6 and 11, or with HPV18 E2ΔNT, ectopically expressed caspase 8 remained as the full-length pro-form predominantly present in the soluble fractions (Figure 4D). These results point to a clear correlation between filament forming properties of HVP18 and HPV 16 E2 proteins and their pro-apoptotic activities. Conversely, the assembly of caspase 8 into filaments was specific for the presence of pro-apoptotic E2 proteins, as the expression of caspase 8 with the non-apoptotic forms of E2 or with GFP alone led to a diffuse, distribution (Figure 4B). Filaments therefore represent a particular oligomerization state of caspase 8 that it is unable to implement itself.

Altogether, the results indicate that the pro-apoptotic E2 proteins and caspase 8 are necessary and sufficient to form filaments, suggesting that the oligomerization of caspase 8 is not driven by a typical DFD adaptor, which would be required at stochiometric levels with caspase 8. Indeed, the FADD adaptor was not detected as associated with insolubilized E2/caspase 8 filaments in either 293 or Saos cells (data not shown), indicating that E2-induced caspase 8 oligomerization was independent of FADD. In contrast, E2 and caspase 8 were both present at equally high levels in the insolubilized filaments, indicating that E2 plays a structural role in their formation despite its lack of an obvious DFD. Therefore, the caspase 8-containing filaments forming with pro-apoptotic E2 proteins may represent a novel type of intracellular death complexes independent of classical DFD adaptors.

In order to better determinate the nature of the interaction between E2 protein and caspase-8, GST-pull-down experiments have been performed. For these experiments, equivalent amounts of purified GST-E2 or GST fixed on Glutation conjugated beads were incubated for 1 hr with ³⁵S in vitro translated proteins (i.e., Caspase-8 (Casp 8), caspase 8 pro-domain (C8 DED), caspase 8 catalytic domain (C8 CD), vFLIP MC159 (MC159) or FADD) produced in TNT, in binding buffer (10 mM HEPES [pH 7.7], 200 mM NaCl, 1 mM MgCl₂, 2 mM DTT, 50 mM sucrose, 0.1% TRITON X-100). Beads were washed four times with binding buffer containing 1% TRITON X-100, bound proteins were separated by SDS-PAGE, and revealed by autoradiography.

The figure 4 E) shows the results of GST pull-down experiments of *in vitro* translated ³⁵S-labelled proteins with GST alone or GST-E2 fusion proteins as indicated. Bound (B) and 1/10^{th} input (I) ³⁵S-labelled proteins were loaded on SDS-PAGE and revealed by autoradiography.

The results established that HPV18 E2 interacted with caspase 8 through the caspase pro-domain that contains tandem DEDs, whereas no interaction was detected with the caspase catalytic domain (Figure 4E). In similar experiments, the E2 protein only marginally bound to the DED-containing vFLIP MC159 or FADD proteins (Figure 4E), indicating that E2 selectively binds to the DEDs domains of caspase 8.

In order to confirm said interaction, co-immunoprecipitation experiments have been performed. 293 cells were transfected as previously by plasmids expressing either GFP-E2 and caspase 8 or GFP-E2 and HA-MC159. Transfected cells were extracted in 1% TRITON P300 buffer (20 mM NaH₂PO₄, 300 mM NaCl, 5 mM EDTA, 5 mM DTT) supplemented with protease inhibitors (ROCHEDIAGNOSTICS) for 30 min at 4°C followed by centrifugation for 30 min at 13,000 rpm. Equal amounts of soluble proteins were then immunoprecipitated with anti-E2 polyclonal rabbit antibodies for 16 hours at 4°C, the immunoprecipitates were washed 3 times in 0.1% Triton P300 buffer, and processed for Western blotting with anti-GFP (TORREY PINES BIOLABS), anti-HA (SIGMA) or anti-caspase 8 antibodies.

The figure 4 F) shows the immunoblots realized with caspase 8, HA and GFP antibodies on anti-E2 immunoprecipitates (IPαE2) and 1/10 whole cell extracts (Inp) from 293 cells expressing GFP-E2 and caspase 8 or GFP-E2 and HA-MC159.

The results confirm that HPV18 E2 interact with caspase 8 but not with MC159 (Figure 4F).

Finally, and in order to confirm the interaction of E2 with the caspase catalytic domain of caspase 8, 293 cells were transfected as previously by plasmids expressing either GFP-caspase 8 (GFP-C8) and E2, GFP- catalytic domain of caspase 8 (GFP-C8 CD; aa 180 to 480 of caspase 8)), or GFP-E2 and caspase 8 pro-domain (C8 DED; aa 1 to 181 of caspase 8). The transfected cells were processed for immunofluorescence as described previously with anti-E2 or anti-caspase 8 antibodies, followed by secondary Texas Red (TR)-coupled antibodies.

The figure 4 G) upper panel shows the immunofluorescence pictures of 293 cells transfected with E2 and GFP fusion proteins with either full-length caspase 8 (GFP-C8) or its catalytic Caspase Domain alone (GFP-C8CD) and processed for immunofluorescence with anti E2-antibodies.

The figure 4 G) lower panel shows the immunofluorescence pictures of 293 cells transfected with GFP-E2 and the DED-containing pro-domain of caspase 8 (C8-DED) and processed for immunofluorescence using caspase 8 antibody.

Finally, the results show that HPV18 E2 engages direct, non-homotypic interactions with the DED of caspase 8 that are likely to be involved in filament formation. Indeed, the caspase pro-domain of caspase 8, as well as each of the DED domain, could mediate assembly into filaments with E2 (Figure 4G and data not shown).

### 4) E2/caspase 8 filaments are antagonized by the MC159 vFLIP protein but can include FADD

The present study has established that the assembly of GFP-E2/caspase 8 filaments occurs through direct contacts between E2 and the DED-containing domain of caspase 8, i.e. the same homotypic interaction domain involved in recruitment by FADD. However, interactions between E2 and caspase 8 are not homotypic (as discussed above) and an investigation on how they could be affected by typical DED-mediated interactions. For these experiments, the viral anti-apoptotic FLICE Inhibitory Protein (FLIP) MC159 and the FADD adaptor, both of which are known to engage homotypic interactions with the DED of caspase 8, have been used. In contrast to FADD, which drives caspase oligomerization, MC159 negatively interferes with these interactions thereby repressing caspase activation (GARVEY et al., J. Virol., vol.76, p:697-706, 2002; SIEGEL *et al.,* abovementioned, 1998).

293 cells were co-transfected as described previously with GFP-E2, caspase 8, HA-MC159 and FADD expression plasmids. 24 hrs post-transfection, the transfected cells were then processed for immunofluorescence as described previously with anti-caspase 8 (PHARMINGEN), anti-FADD (PHARMINGEN), or anti HA antibodies (SIGMA).

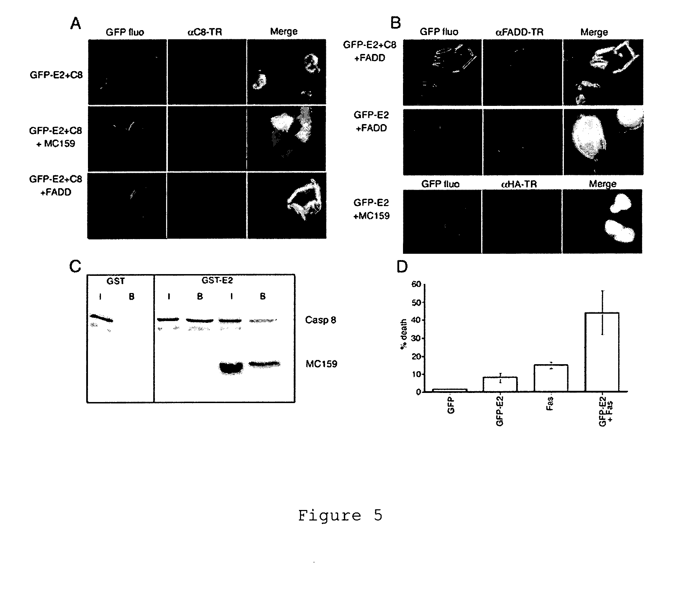

The figure 5 A) shows the pictures for GFP fluorescence (GFP fluo), Texas-Red fluorescence corresponding to caspase 8 cellular location (αC8-TR) or both (Merge) of 293 cells co-transfected with GFP-E2, caspase 8, HA-MC159 and FADD expression plasmids.

The figure 5 B) shows the pictures for GFP fluorescence (GFP fluo), Texas-Red fluorescence corresponding to FADD cellular location (αC8-TR) or corresponding to MC159 location (αHA-TR) or both (Merge) of 293 cells co-transfected with GFP-E2, caspase 8, HA-MC159 and FADD expression plasmids.

The results shows that that in the presence of MC159, GFP-E2/caspase 8 filaments disappeared, with caspase 8 expression reverting to a diffuse distribution and the E2 protein shuttling back to the nucleus (Figure 5A). MC159 itself consistently showed a diffuse cytoplasmic distribution as expected (SIEGEL *et al.,* abovementioned, 1998 and Figure 3-B). In contrast to MC159, co-expressed FADD is efficiently incorporated within filaments that contain both GFP-E2 and caspase 8 (Figure 5A and 5B). All filaments stained positive for GFP-E2 and either caspase 8 or FADD, indicating that they simultaneously contained the three proteins (Figure 5A and 5B). FADD spontaneously self-oligomerized to form filaments when it was ectopically expressed as previously reported (P PEREZ and WHITE, J. Cell Biol., vol.141, p:1255-66, 1998; SIEGEL et al., J. Cell Biol., vol.141, p:1243-53, 1998). In the absence of co-expressed caspase 8 however, FADD filaments only marginally recruited GFP-E2, which remained mainly nuclear (Figure 3B), as predicted by the GST pull-down experiments that show no specific interaction between E2 and FADD (Figure 4E).

In order to confirm the inhibition of E2/caspase-8 DED interaction by MC159, GST pull-down experiments have been performed as previously with incubation of GST or GST-E2 with ³⁵S TNT-produced caspase 8 and MC159 mixed in binding buffer 1 hour before addition of GST or GST-E2, or with ³⁵S TNT-produced caspase 8. Bound (B) and 1/10^{th} input (I) ³⁵S-labelled proteins were loaded on SDS-PAGE and revealed by autoradiography.

The figure 5 C) shows the GST pull-down experiments realized after incubation of GST-E2 or GST as indicated with ³⁵S TNT-produced caspase 8 with or without ³⁵S TNT-produced MC159. The lanes B and I correspond to bound and 1/10^{th} input ³⁵S-labelled proteins respectively.

The results effectively confirm that the binding of caspase 8 to E2 was reduced by the presence of MC159 (Figure 5C), indicating that the DED-mediated binding of MC159 to caspase 8 competes with caspase 8-E2 interactions and inhibits filament formation.

In conclusion, the E2/caspase 8/FADD filaments are made by caspase 8 interacting simultaneously with FADD and E2, leading to the assembly of multimerized protein complexes that contain the three proteins. The DED homotypic interactions between oligomerized FADD and caspase 8 therefore do not compete with E2-caspase 8 interaction, implying that the DEDs of caspase 8 could simultaneously engage with both partners. So, pro-apoptotic E2 and the FADD adaptor may be able to collaborate to induce caspase 8 oligomerization, through distinct and independent mechanisms.

To corroborate functionally such collaboration, , 293 cells were transfected as described previously with expression plasmids coding for GFP, GFP-E2, and the Death Receptor Fas. For Fas-induced cell death, the agonistic APO 1-3 anti-Fas antibody (2 µg/ml; ALEXIS BIOCHEMICALS) was added for 16 hours. Dead cells were quantified by propidium iodide uptake 48 hours after transfection as described previously.

The figure 5 D) shows the cell death percentage after induction of Fas-induced cell death with APO 1-3 antibody on 293 cells transfected with expression plasmids for GFP, GFP-E2, Fas, or GFP-E2 and Fas.

The results established that HPV 18 E2 enhanced Fas-induced apoptosis, probably by cooperating with FADD in the induction of caspase 8 oligomerization (Figure 5D). In this regard, the E2 protein counteracts the effects of the E6 oncoprotein of Human Papillomaviruses, which inhibits Fas-mediated apoptosis by accelerating the degradation of FADD (FILIPPOVA *et al.,* abovementioned, 2004). Inhibition of caspase 8 catalytic activity by crmA prevented caspase self-activation following filament assembly, while the MC159 vFLIP reduced E2-induced cell death by abrogating the assembly of GFP-E2 filaments (Figure 1). The HPV18 E2 protein therefore represents a new type of viral pro-apoptotic factor, acting conversely to the viral FLIP anti-apoptotic MC159 protein. HPV18 E2 uses a novel mechanism to activate caspase 8, by direct induction of caspase 8 oligomerization within filaments through non-DFD interactions with caspase 8 DEDs. This process might ensure efficient cell destruction even though they contain altered Death Receptor apoptotic pathways, as it is often the case in infected cells.

### 5) A short α-helix of the E2 amino-terminal domain mediates filament formation and cell death

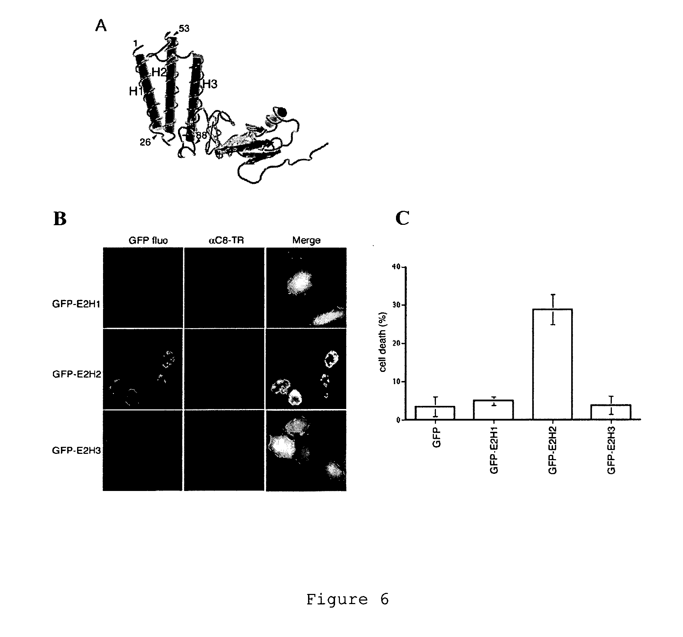

The figure 6 A) shows a schematic representation of the crystal structure of the HPV16 E2 amino-terminal domain, which is composed of three α-helix and a β-sheet bundle separated by a rigid fulcrum (ANTSON *et al.,* abovementioned, 2000).

DEMERET *et al.* (abovementioned, 2003) has established that the N-terminal domain of pro-apoptotic E2 protein from HPV18 is sufficient to induce apoptosis. In this N-terminal domain, the second helix H2 of pro-apoptotic E2 protein from HPV18 corresponds to a signal peptide enabling the delocalization of a nuclear protein to the cytoplasm, said cellular localization being critical for the apoptosis induction (BLACHON, abovementioned, 2005).

To obtain further insight into the type of interaction between E2 and caspase 8, GFP fusion proteins comprising different domains of E2 have been made and analyzed their ability to interact with caspase 8.

For these experiments, helix H1 (aa 1-26) or H3 (aa 54-88) from the E2 amino-terminal domain from HPV 18 were cloned by a conventional PCR-based technique in frame with GFP. The helix H2 (aa 27-53) corresponding to the cytoplasmic localization domain of E2 protein from HPV18 was also cloned in frame with GFP in the same way.

293 cells were transfected as previously described with plasmids expressing caspase 8 and the GFP fusion proteins comprising the α-helix 1 and 3 of E2 (E2H1spanning aa 1 to 26, E2H3 spanning aa 54 to 88). As a control, 293 cells were also transfected with plasmids expressing caspase 8 and the GFP fusion protein comprising the α-helix 2 of E2 (E2H2 spanning aa 27 to 53), since no modification of apoptosis has been observed in BLACHON *et al.* (abovementioned, 2005) after the cell expression of a fusion protein comprising the same domain fused to GFP and a NLS signal peptide.

24 hrs post-transfection, one part of the transfected cells were then processed for immunofluorescence as described previously with anti-caspase 8 antibody (PHARMINGEN). Simultaneously, the dead cells were quantified in the other part of transfected cells by propidium iodide uptake 48 hours after transfection as described previously.

The figure 6 B) shows the pictures for GFP fluorescence (GFP fluo), Texas-Red fluorescence corresponding to caspase 8 cellular location (αC8-TR) or both (Merge) of 293 cells co-transfected with GFP-E2H1, GFP-E2H3 and the control (GFP-E2H2) expression plasmids.

The figure 6 C) shows the viability of HeLa cells expressing each GFP-E2 α-helix as indicated, determined 48 hours post-transfection by propidium iodide uptake.

The results established that the polypeptides corresponding to either the first or the third α-helixs of the amino terminal domain of E2 were not able to induce caspase 8 filaments. Nevertheless, the results show surprisingly that the second α-helix (amino acids 27 to 53) of E2, which is able to localize the E2 protein in the cytoplasm, is also able to induce caspase 8 filaments.

In accordance with filaments formation, the second α-helix of E2 alone was able to induce apoptosis similarly to the full-length protein (Figure 4D), confirming that the E2 pro-apoptotic activity is based on induction of caspase 8 oligomerization. Such a result was unexpected since no apoptosis has been observed in BLACHON *et al.* (abovementioned, 2005) after the cell expression of a fusion protein comprising the same domain fused to GFP and a NLS signal peptide.

Consequently, these results definitively exclude the involvement of a pseudo "Death Fold Domain" within E2 in the interaction with caspase 8. Based on these observations, we propose that the E2 protein of HPV18 induces caspase 8 oligomerization through direct interactions involving only the second α-helix of E2. Assembly of caspase 8 and E2 within filaments may provide the caspase with adequate oligomerization state to be efficiently self-activated.

### 6) The short α-helix of the E2 amino-terminal domain mediating cell death also pre-exists in E2 protein from non-apoptotic HPV6 and HPV11

In order to investigate the existence of apoptotic activity associated with helix H2 from other HPV type, helix H2 (aa 27-53 from HPV 18, and aa 22-49 from HPV 6, HPV 11 and HPV 16)) from the E2 amino-terminal domain from pro-apoptotic HPV 16 and HPV 18, and from non-apoptotic HPV 6 and HPV 11 were cloned by a conventional PCR-based technique in frame with GFP.

The apoptotic activity of the fusion proteins were assayed as described previously.

The results have shown surprisingly that the second α-helix (amino acids 22 to 49) of E2 protein from the non-apoptotic HPV such as the apoptotic HPV were able to induce caspase 8 filaments and apoptosis.

Consequently, it seems that the second α-helix (amino acids 22 to 49) of E2 protein has conserved its function of apoptosis induction in the different HPV types. The figure 7 shows the alignment of amino-terminal domain of E2 proteins from different types of HPV.

### 7) The short α-helix of the E2 amino-terminal domain mediating cell death also pre-exists in E2 protein from non-apoptotic HPV6 and HPV11

In order to better identify the fragment of the second helix H2 implicated in apoptosis induction, different fragments from E2 protein from HPV18 (aa 27-53, 30-53, 30-44, 34-46, 36-53, 36-44, 36-46, 36-48, and 36-50) were cloned by a conventional PCR-based technique in frame with GFP.

The apoptotic activity of the fusion proteins were assayed as described previously.

The results are presented in the following table:

| GFP-E2 fusion protein | Apoptotic activity |
|---|---|
| GFP-E2 (27-53) Contained in bacteria CNCM-3743 | + |
| GFP-E2 (30-53) Contained in bacteria CNCM-3744 | + |
| GFP-E2 (30-44) | - |
| GFP-E2 (34-46) | - |
| GFP-E2 (36-53) | + |
| GFP-E2 (36-50) | |
| GFP-E2 (36-48) | |

## Claims

1. A polypeptide comprising the sequence X₁WX₂X₃X₄RX₅X₆X₇ X₈ X₉, and derivatives thereof, wherein:
• X₁ is an amino acid selected in the group consisting in histidine (H), tyrosine (Y), aspartic acid (D), glutamine (Q), and leucine (L), preferably histidine (H) or tyrosine (Y);
• X₂ is a polar amino acid, charged or not, and selected in the group consisting in serine (S) threonine (T), lysine (K), arginine (R), asparagine (N), aspartic acid (D), cysteine (C), glycine (G), glutamine (Q), histidine (H), and glutamic acid (E);
• X₃ is a polar amino acid, charged or not, and selected in the group consisting in serine (S), threonine (T), asparagine (N), cysteine (C), histidine (H), leucine (L), tyrosine (Y), alanine (A), and isoleucine (I);
• X₄ is an amino acid selected in the group consisting in threonine (T), arginine (R), asparagine (N), alanine (A), isoleucine (I), leucine (L), methionine (M), phenylalanine (F), tryptophane (W), valine (V) and tyrosine;
• X₅ is an amino acid selected in the group consisting in leucine (L), methionine (M), isoleucine (I), tryptophane (W), tyrosine (Y), histidine (H), glutamine (Q), lysine (K), and arginine (R);
• X₆ is an amino acid selected in the group consisting in leucine (L), and glutamic acid (E);
• X₇ is an amino acid selected in the group consisting in cysteine (C), asparagine (N), alanine (A), tyrosine (Y), serine (S), glutamine (Q), and proline (P);
• X₈ is a hydrophobic amino acid selected in the group consisting in alanine (A), isoleucine (I), leucine (L), methionine (M), phenylalanine (F), tryptophane (W), valine (V) and tyrosine; and
• X₉ is a threonine or an hydrophobic amino acid selected in the group consisting in alanine (A), isoleucine (I), leucine (L), methionine (M), phenylalanine (F), tryptophane (W), valine (V) and tyrosine.

2. The polypeptide according to claim 1, wherein said polypeptide is less than 100 amino acids in length.

3. The polypeptide according to any one of claims 1 or 2, wherein said polypeptide comprises an amino acid sequence which is selected in the group comprising YWKHMRLECAI (position 32 to 42 of SEQ ID NO:2), YWKHIRLECVL (position 32 to 42 of SEQ ID NO:3), YWKLIRLECAV (position 32 to 42 of SEQ ID NO:4), HWKLTRMECVL (position 32 to 42 of SEQ ID NO:5), HWKLIRMECAI (position 32 to 42 of SEQ ID NO:6), HWKLIRMECAL (position 32 to 42 of SEQ ID NO:7), HWRLRRIECAL (position 32 to 42 of SEQ ID NO:8), YWNCVRLENAI (position 32 to 42 of SEQ ID NO:9), YWKYVRLENAI (position 32 to 42 of SEQ ID NO:10), YWKCVRMENAI (position 32 to 42 of SEQ ID NO:11), YWKLVRMENVI (position 32 to 42 of SEQ ID NO:12), YWQLIRWENAI (position 31 to 41 of SEQ ID NO:13), YWQLIRLENAI (position 31 to 41 of SEQ ID NO:14), YWKLVRYECAI (position 32 to 42 of SEQ ID NO:15), YWKLVRYECAI (position 32 to 42 of SEQ ID NO:16), YWTLVRYECAM (position 32 to 42 of SEQ ID NO:17), YWTLLRYEAAM (position 32 to 42 of SEQ ID NO:18), YWKAVRHEYVL (position 32 to 42 of SEQ ID NO:19), YWKAVRQENVI (position 32 to 42 of SEQ ID NO:20), YWKAVRHENVV (position 32 to 42 of SEQ ID NO:21), HWHYVRLENAML(position 32 to 42 of SEQ ID NO:22), HWKCIRYECVL (position 32 to 42 of SEQ ID NO:23), HWKCIRYECVL (position 32 to 42 of SEQ ID NO:24), HWKCLRYESVL (position 35 to 45 of SEQ ID NO:25), HWKCMRHESVL (position 32 to 2 of SEQ ID NO:26), HWKCMRHESVL (position 32 to 42 of SEQ ID NO:27), HWKCIRLESVL (position 32 to 42 of SEQ ID NO:28), HWKCLRIEAAL (position 32 to 42 of SEQ ID NO:29), HWKCLRMEAVV (position 32 to 42 of SEQ ID NO:30), HWKCIRLECAL (position 32 to 42 of SEQ ID NO:31), HWKCIRLECAL (position 32 to 42 of SEQ ID NO:32), HWKHVRHENVL (position 32 to 42 of SEQ ID NO:33), HWKHVRLENVL (position 32 to 42 of SEQ ID NO:34), HWQLLRQEQII (position 32 to 42 of SEQ ID NO:35), HWKLLRQEQIL (position 32 to 42 of SEQ ID NO:36), HWKLLRQEQVL (position 32 to 42 of SEQ ID NO:37), HWQLLRQEQIL (position 32 to 42 of SEQ ID NO:38), HWQLLRQEQVL (position 32 to 42 of SEQ ID NO:39), HWQTLRQEQIL (position 32 to 42 of SEQ ID NO:40), HWKLLRQEQAL (position 32 to 42 of SEQ ID NO:41), HWQTLRKEAVL (position 32 to 42 of SEQ ID NO:42), HWQTLRKEPVL (position 32 to 42 of SEQ ID NO:43), HWQTLRQEAVL (position 32 to 42 of SEQ ID NO:44), HWQTLRKEAVT (position 32 to 42 of SEQ ID NO:45), HWTLLRREAVL (position 32 to 42 of SEQ ID NO:46), HWLLLRKEAVL (position 32 to 42 of SEQ ID NO:47), HWQTLRKEAVL (position 32 to 42 of SEQ ID NO:48), HWQTLRKEAVL (position 32 to 42 of SEQ ID NO:49), HWQTLRKEAVL (position 32 to 42 of SEQ ID NO:50), HWQILRREAVL (position 32 to 42 of SEQ ID NO:51), HWQILRKEAVL (position 32 to 42 of SEQ ID NO:52), HWRLLRKEAVL (position 32 to 42 of SEQ ID NO:53), HWQALRREAVL (position 32 to 42 of SEQ ID NO:54), QWNLLRQEQVL (position 32 to 42 of SEQ ID NO:55), QWNLIRQEQVL (position 32 to 432 of SEQ ID NO:56), YWDLNRKLYVT (position 29 to 39 of SEQ ID NO:57), YWENVRKENAI (position 32 to 42 of SEQ ID NO:58), YWENIRKENAI (position 32 to 43 of SEQ ID NO:59), YWENIRKENAI (position 32 to 42 of SEQ ID NO:60), YWESMRKEQVL (position 31 to 41 of SEQ ID NO:61), YWKAVRLENVI (position 31 to 41 of SEQ ID NO:62), and LWNLLRRENAI (position 32 to 42 of SEQ ID NO:63).

4. The polypeptide according to any one of claims 1 to 3, wherein said polypeptide comprises an amino acid sequence which is selected in the group comprising YWQLIRWENAI (position 31 to 41 of SEQ ID NO:13), YWKHMRLECAI (position 32 to 42 of SEQ ID NO:2), HWKCIRLESVL (position 32 to 42 of SEQ ID NO:28), and HWKCMRHESVL (position 32 to 41 of SEQ ID NO:26).

5. The polypeptide according to any one of claims 1 to 4, wherein said polypeptide comprises the sequence X₁WX₂X₃X₄RX₅X₆X₇X₈X₉X₁₀X₁₁X₁₂X₁₃X₁₄X₁₅X₁₆ (SEQ ID NO:70), and derivatives thereof, wherein:
• X₁, X₂, X₃, X₄, X₅, X₆, X₇, X₈, and X₉ are as in claim 1;
• X₁₀ is a glutamine (Q) or an hydrophobic amino acid selected in the group consisting in alanine (A), isoleucine (I), leucine (L), methionine (M), phenylalanine (F), tryptophane (W), valine (V) and tyrosine;
• X₁₁ is a histidine (H) or an aromatic amino acid selected in the group consisting in tyrosine (Y), phenylalanine (F), and tryptophane (W);
• X₁₂ is selected in the group consisting in lysine (K), threonine (T), alanine (A), tyrosine (Y), phenylalanine (F), valine (V);
• X₁₃ is an hydrophobic amino acid selected in the group consisting in alanine (A), isoleucine (I), leucine (L), methionine (M), phenylalanine (F), tryptophane (W), valine (V) and tyrosine;
• X₁₄ is a positively charged amino acid selected in the group consisting in lysine (K), arginine (R), and histidine (H);
• X₁₅ is a polar amino acid selected in the group consisting in lysine (K), arginine (R), glutamine (Q), and glutamic acid (E); and
• X₁₆ is an amino acid (i.e., an α amino acid), preferably X₁₅ is selected in the group consisting in lysine (K), arginine (R), glutamine (Q), glutamic acid (E), methionine (M), leucine (L), glycine (G), , histidine (H), alanine (A), and asparagines (N).

6. The polypeptide according to claim 5, wherein said polypeptide comprises an amino acid sequence which is selected in the group comprising YWKHMRLECAIYYKAREM (position 32 to 49 of SEQ ID NO:2), YWKHIRLECVLMYKAREM (position 32 to 49 of SEQ ID NO:3), YWKLIRLECAVFYKAREM (position 32 to 49 of SEQ ID NO:4), HWKLTRMECVLFYKAKEL (position 32 to 49 of SEQ ID NO:5), HWKLIRMECAIMYTARQM (position 32 to 49 of SEQ ID NO:6), HWKLIRMECALLYTAKQM (position 32 to 49 of SEQ ID NO:7), HWRLRRIECALYYKAKEL (position 32 to 49 of SEQ ID NO:8), YWNCVRLENAIYYAARER (position 32 to 49 of SEQ ID NO:9), YWKYVRLENAIFYAARER (position 32 to 49 of SEQ ID NO:10), YWKCVRMENAIFYAARER (position 32 to 49 of SEQ ID NO:11), YWKLVRMENVILFAAREN (position 32 to 49 of SEQ ID NO:12), YWQLIRWENAIFFAAREH (position 31 to 48 of SEQ ID NO:13), YWQLIRLENAILFTAREH (position 31 to 48 of SEQ ID NO:14), YWKLVRYECAIFYKAREG (position 32 to 49 of SEQ ID NO:15), YWKLVRYECAIFYKAREG (position 32 to 49 of SEQ ID NO:16), YWTLVRYECAMFYTARER (position 32 to 49 of SEQ ID NO:17), YWTLLRYEAAMFYAARER (position 32 to 49 of SEQ ID NO:18), YWKAVRHEYVLYYKAREN (position 32 to 49 of SEQ ID NO:19), YWKAVRQENVIYYKAREN (position 32 to 49 of SEQ ID NO:20), YWKAVRHENVVLYKARQN (position 32 to 49 of SEQ ID NO:21), HWHYVRLENAMLFKARQA (position 32 to 49 of SEQ ID NO:22), HWKCIRYECVLLHKAKQM (position 32 to 49 of SEQ ID NO:23), HWKCIRYECVLLHKAKQM (position 32 to 49 of SEQ ID NO:24), HWKCLRYESVLLHKARQM (position 35 to 52 of SEQ ID NO:25), HWKCMRHESVLLYKAKQM (position 32 to 49 of SEQ ID NO:26), HWKCMRHESVLLYKAKQM (position 32 to 49 of SEQ ID NO:27), HWKCIRLESVLLHKAKQM (position 32 to 49 of SEQ ID NO:28), HWKCLRIEAALLFKAREM (position 32 to 49 of SEQ ID NO:29), HWKCLRMEAVVLYKAREM (position 32 to 49 of SEQ ID NO:30), HWKCIRLECALQYKAREL (position 32 to 49 of SEQ ID NO:31), HWKCIRLECALQYKAREM (position 32 to 49 of SEQ ID NO:32), HWKHVRHENVLLHKAREM (position 32 to 49 of SEQ ID NO:33), HWKHVRLENVLLHKAREM (position 32 to 49 of SEQ ID NO:34), HWQLLRQEQIIYHYARRH (position 32 to 49 of SEQ ID NO:35), HWKLLRQEQILLYYARKR (position 32 to 49 of SEQ ID NO:36), HWKLLRQEQVLFYYARRH (position 32 to 49 of SEQ ID NO:37), HWQLLRQEQILFHYARKN (position 32 to 49 of SEQ ID NO:38), HWQLLRQEQVLFYYARKN (position 32 to 49 of SEQ ID NO:39), HWQTLRQEQILLHYARKN (position 32 to 49 of SEQ ID NO:40), HWKLLRQEQALLFFARKH (position 32 to 49 of SEQ ID NO:41), HWQTLRKEAVLLYYAREK (position 32 to 49 of SEQ ID NO:42), HWQTLRKEPVLLYYAREK (position 32 to 49 of SEQ ID NO:43), HWQTLRQEAVLLYFARQR (position 32 to 49 of SEQ ID NO:44), HWQTLRKEAVTLYFARQK (position 32 to 49 of SEQ ID NO:45), HWTLLRREAVLLYYARQK (position 32 to 49 of SEQ ID NO:46), HWLLLRKEAVLLYFARQK (position 32 to 49 of SEQ ID NO:47), HWQTLRKEAVLLYFARQK (position 32 to 49 of SEQ ID NO:48), HWQTLRKEAVLLYFARQN (position 32 to 49 of SEQ ID NO:49), HWQTLRKEAVLLYFARQH (position 32 to 49 of SEQ ID NO:50), HWQILRREAVLLYFARQK (position 32 to 49 of SEQ ID NO:51), HWQILRKEAVLLYFARRK (position 32 to 49 of SEQ ID NO:52), HWRLLRKEAVLLYFARQN (position 32 to 49 of SEQ ID NO:53), HWQALRREAVLLYYARQN (position 32 to 49 of SEQ ID NO:54), QWNLLRQEQVLFHYARKK (position 32 to 49 of SEQ ID NO:55), QWNLIRQEQVLFHFARKN (position 32 to 49 of SEQ ID NO:56), YWDLNRKLYVTYYYARKE (position 29 to 46 of SEQ ID NO:57), YWENVRKENAIMHYARKQ (position 32 to 49 of SEQ ID NO:58), YWENIRKENAIMHFARKQ (position 32 to 49 of SEQ ID NO:59), YWENIRKENAIMHYARKQ (position 32 to 49 of SEQ ID NO:60), YWESMRKEQVLAFYAKKE (position 31 to 48 of SEQ ID NO:61), YWKAVRLENVIAYYARKE (position 31 to 48 of SEQ ID NO:62), and LWNLLRRENAIWYVLRQE (position 32 to 49 of SEQ ID NO:63).

7. The polypeptide according to claim 5, wherein said polypeptide comprises an amino acid sequence which is selected in the group comprising YWQLIRWENAIFFAAREH (position 31 to 48 of SEQ ID NO: 13), YWKHMRLECAIYYKAREM (position 32 to 49 of SEQ ID NO:2), HWKCIRLESVLLHKAKQM (position 32 to 49 of SEQ ID NO:28), and HWKCMRHESVLLYKAKQM (position 32 to 49 of SEQ ID NO:26).

8. The polypeptide according to any one of claims 1 to 7, wherein said polypeptide further comprises an amino acid sequence coding for a cell penetrating peptide.

9. A nucleic acid encoding for a polypeptide as defined in any one claims 1 to 8.

10. The nucleic acid according to claim 9, wherein said nucleic acid corresponds to the insert of the expression plasmid contained in the bacteria CNCM-3743 or CNCM-3744.

11. A vector comprising a nucleic acid as defined in claim 9.

12. The vector according to claim 11, wherein said vector corresponds to the vector contained in the bacteria CNCM-3743 or CNCM-3744.

13. A host cell genetically engineered with the nucleic acid as defined in any one of claims 9 or 10, with the vector as defined in any one of claims 11 or 12.

14. The host cell according to claim 13, wherein said host cell corresponds to the bacteria CNCM-3743 or CNCM-3744.

15. An antibody directed against a polypeptide as defined in any one claims 1 to 8.

16. A composition comprising a polypeptide as defined in any one claims 1 to 8, a nucleic acid as defined in any one of claims 9 or 10, or a vector as defined in any one of claims 11 or 12, optionally associated with a pharmaceutically acceptable vehicle.

17. A use of a polypeptide as defined in any one claims 1 to 8, a nucleic acid as defined in any one of claims 9 or 10, a vector as defined in any one of claims 11 or 12, or a composition as defined in claim 16 for treating cancer in a subject.
